# EUROPEAN PATENT SPECIFICATION

(11) **EP 2 983 728 B1**
(45) Date of publication and mention of the grant of the patent: **06.06.2018**
(21) Application number: 14783395.8
(22) Date of filing: 10.04.2014
(51) Int. Cl.: A61L 27/40, C12N 5/02, C12N 5/074, C12N 5/0735, C12N 5/0775

(54) **POLYCAPROLACTONE MICROCARRIERS FOR STEM CELL CULTURE AND FABRICATION THEREOF**
POLYCAPROLACTONMIKROTRÄGER FÜR STAMMZELLENKULTUR UND HERSTELLUNG DAVON
MICROSUPPORTS DE POLYCAPROLACTONE POUR LA CULTURE DE CELLULES SOUCHES ET LEURS FABRICATIONS

(30) Priority: 10.04.2013 SG 201302683
(43) Date of publication of application: 17.02.2016
(73) Proprietor: Agency for Science, Technology and Research, Singapore 138632 (SG)
(72) Inventor: BIRCH, William, Singapore 117602 (SG); REUVENY, Shaul, Singapore 138668 (SG); OH, Steve, Singapore 138668 (SG); LI, Jian, Singapore 117602 (SG)
(74) Representative: Clegg, Richard Ian
(86) International application number: PCT/SG2014/000158
(87) International publication number: WO 2014/168585

(56) References cited:
- WO-A1-2013/169374
- WO-A2-2006/091921
- US-A1- 2011 165 646
- E LUONG-VAN ET AL.: "The in vivo assessment of a novel scaffold containing heparan sulfate for tissue engineering with human mesenchymal stem cells", JOURNAL OF MOLECULAR HISTOLOGY, vol. 38, 2007, pages 459-468, XP2724121, SPRINGER-VERLAG, DORDRECHT ISSN: 1567-2379
- S-C WU ET AL.: "Artificial extracellular matrix proteins contain heparin-binding and RGD-containing domains to improve osteoblast-lie cell attachment and growth", JOURNAL OF BIOMEDICAL MATERIALS RESEARCH, vol. 79A, no. 3, 1 December 2006 (2006-12-01), pages 557-565, XP002763345, WILEY, NEW YORK, NY ISSN: 0021-9304
- J LI ET AL.: "Fabrication of uniform-sized poly-epsilon-caprolactone microspheres and their applications in human embryonic stem cell culture", BIOMEDICAL MICRODEVICES, vol. 17, no. 6, 10 December 2015 (2015-12-10), pages 1-12, XP35562171, KLUWER, DORDRECHT, ISSN: 1387-2176
- CHEN ET AL.: 'Synthesis, characterization and osteoconductivity properties of bone fillers based on alendronate-loaded poly(e-caprolactone)/ hydroxyapatite microspheres' JOURNAL OF MATERIALS SCIENCE : MATERIALS IN MEDICINE vol. 22, no. 3, 2011, pages 547 - 555, XP019895555
- KIM ET AL.: 'Bioactive Porous Beads as an Injectable Urethral Bulking Agent: In Vivo Animal Study for the Treatment of Urinary Incontinence' TISSUE ENGINEERING, PART A vol. 17, no. 11-12, 2011, pages 1527 - 1535, XP055285139
- HONG ET AL.: 'Tissue Engineering Polymeric Microcarriers with Macroporous Morphology and Bone-Bioactive Surface' MACROMOLECULAR BIOSCIENCE vol. 9, no. 7, 2009, pages 639 - 645, XP055285140
- ZHANG ET AL.: 'Preparation of open porous polycaprolactone microspheres and their applications as effective cell carriers in hydrogel system' MATERIALS SCIENCE AND ENGINEERING: C vol. 32, no. 8, 2012, pages 2589 - 2595, XP055285142
- SCHENKE-LAYLAND ET AL.: 'Recapitulation of the embryonic cardiovascular progenitor cell niche' BIOMATERIALS vol. 32, no. 11, 2011, pages 2748 - 2756, XP028145161

## Description

### Field of the Invention

The present invention relates to the fields of cell biology, molecular biology and biotechnology. More particularly, the invention relates to culturing stem cells on particulate carriers.

### Background to the Invention

Stem cells, unlike differentiated cells have the capacity to divide and either self-renew or differentiate into phenotypically and functionally different daughter cells (Keller, Genes Dev. 2005;19:1129-1155; Wobus and Boheler, Physiol Rev. 2005;85:635-678; Wiles, Methods in Enzymology. 1993;225:900-918; Choi et al, Methods Mol Med. 2005;105:359-368).

While stem cell culture on planar surfaces is relatively well established, limitations on the available surface area for culturing cells present significant technological and engineering challenges. Given the nominal requirement of 1 billion (1 x 10⁹) cells for a therapeutic dose, scalable technologies are expected to target the routine batch fabrication of 1 trillion (1 x 10¹²) cells, corresponding to 1000 doses.

Three-dimensional bioreactors offer a highly scalable technology that incorporates the necessary degree of control over the cell culture environment. Under agitation, their volumes can range from milliliters, to tens of liters, or more.

Microspheres may be used as microcarriers, offering a support that is capable of supporting the expansion, and potentially the subsequent differentiation, of stem cells in a 3-dimensional environment. They have been proven successful for the culture of human embryonic stem cells, starting from single cell seeding, in a defined and scalable environment under agitation.

The present invention describes the fabrication and application of microcarriers with uniform size, made from a biodegradable and bioresorbable polymer that has been FDA-approved for implants since the 1970s.

US8137972B2 describes the fabrication of scaffolds for tissue engineering with a density lighter than or the same as water (∼1 g/cm³ at about 4°C). Many bioreactors are designed for use with slightly negatively buoyant microcarriers. Accordingly, such microcarriers are therefore not suitable for use in such bioreactors.

US20100322908A1 describes biodegradable microspheres, with a large size distribution, ranging over an order of magnitude (i.e. from 1 to 10 times a given size). WO2009014441A2 describes the fabrication of polycaprolactone (PCL) microspheres by fluidics; however, the size control of their method is unrefined and does not allow for low coefficient of variation (CV) values, as described in the present disclosure. Cultured cells seeded at a uniform density reach confluence at different times on such microcarriers.

J Mater Sci: Mater Med (2008) 19:1703-1711; DOI 10.1007/s10856-007-3253-9, Biomaterials, Volume 22, Number 20, 15 October 2001 , pp. 2785-2794(10), and Journal of Controlled Release 161 (2012) 927-932; doi:10.1016/j.jconrel.2012.05.003 describe fabrication of PCL microspheres for encapsulating and releasing bioactive molecules. US20080020049A1 describes microspheres with a high porosity, interconnected, and their positioning within a scaffold. These documents do not provide for size uniformity of the microspheres.

Similarly, US20110245456A1 does not similarly provide for achieving a uniform size distribution with low CV, of the fabricated biodegradable microspheres. The spray method described does not allow for a high degree of size uniformity of the fabricated microspheres.

US20080095822A1 describes embedding microspheres within a hydrogel, which may be cross-linked. It discloses uniform dispersion of microspheres within the hydrogel, although it does not provide for or describe fabrication of microspheres with uniform size.

WO 2006091921 A3 discloses microcarrier beads comprising a core comprising at least 99% of interconnected pores, and an outer protective layer. Luong-Van et al. 2007, J Mol Histol 38: 459-468 discloses an electrospun fiber scaffold of polycaprolactone incorporating heparan sulphate.

### Summary of the Invention

The present invention provides a polycaprolactone (PCL) microcarrier, or plurality thereof, as defined in the claims. Also provided by the present are methods for culturing stem cells, and methods for differentiating stem cells, as defined in the claims.

The present disclosure relates to microcarriers and a plurality of microcarriers comprising a bioresorbable, biodegradable polymer. The use of a biodegradable, bioresorbable, polymer that is FDA approved for implants allows cells cultured on the microcarriers to be implanted in-vivo, as an integral part of the tissue engineering process. This may be used in the context of bone, cartilage, or even neural regeneration.

In some embodiments disclosed herein, the bioresorbable, biodegradable polymer is polycaprolactone (PCL).

In some embodiments the microcarriers comprise a second biodegradable polymer, blended into PCL. In some embodiments the second biodegradable polymer is included as a biphasic emulsion, within the PCL. In some embodiments, the second biodegradable polymer is more rapidly biodegradable than PCL or can be dissolved in a solvent different from PCL (for example, aqueous or ethanol medium) as a porogen, capable of forming a porous structure within the PCL. In some embodiments the pores are isolated pores or interconnected pores. In some embodiments the pores are macropores.

In some embodiments, the microcarriers have a hydrophilic surface. In some embodiments, the microcarriers are wettable in water. In some embodiments the microcarriers have less than 0.01% surfactant by weight.

In some embodiments polyelectrolyte is included within the PCL. This may be blended into the PCL or in the form of a diblock copolymer, with one segment compatible with PCL and a second segment being the polyelectrolyte. One example of this is polystyrene-polyacrylic acid (PS-PAAc) diblock copolymer, which has been successfully used to confer negative charge to polystyrene microspheres, formed from dichloromethane (DCM).

Another advantage offered by PCL is its ability to release molecules, including bioactive molecules. The release of these molecules in direct contact with cultured cells can efficiently direct their evolution, be it to maintain pluripotency or to induce differentiation into a specific lineage. The range of molecular weight spans from small (a few hundred daltons) to proteins or polysaccharides, with molecular weights in the tens of kilodaltons.

Accordingly, in some embodiments, the microcarriers comprise a bioactive material, such as hydroxyapatite for bone formation, or other bioactive molecules, within the PCL. This can be achieved by direct blending of PCL with these materials or by encapsulation to incorporate them within the PCL.

In some embodiments the microcarriers comprise a compatible polymer with density less than 1 g/cm³, e.g. at 25°C). Advantageously, this can be used to adjust the density of microcarriers that can be used within an agitated bioreactor.

Also disclosed herein are microcarriers having a coating. In some embodiments the coating comprises poly-L-lysine, an adhesion promoting protein or bovine serum albumin. In some embodiments, the microcarriers are substantially or completely coated with a coating comprising one or more adhesion promoting molecules. Adhesion promoting molecules include polypeptides, glycopolypeptides, polysaccharides and cationic polypeptides capable of binding a mammalian surface cell polypeptide, glycopolypeptide, cationic polyelectrolyte or polysaccharide. In some embodiments, the cationic polyelectrolyte is polybrene. In some embodiments, the coating comprises one or more matrix components. Matrix components include components of biological matrices. In some embodiments, the matrix component is an extracellular matrix component.

Also disclosed herein is a PCL microcarrier or a plurality of PCL microcarriers coated with an adhesion promoting molecule. In some embodiments, the coating is a multilayer coating, and the multilayer coating comprises (i) a first layer, comprising an adhesion promoting molecule, and; (ii) another layer, comprising an adhesion promoting molecule. In some embodiments, the adhesion promoting molecule of the first layer is different from the adhesion promoting molecule of the second layer. In some embodiments, the multilayer coating comprises more than two layers. In some embodiments, the multilayer coating comprises 3, 4, 5, 6, 7, 8, 9 or 10 layers.

In some embodiments the adhesion promoting molecule is a matrix component. In some embodiments, the matrix component is selected from polylysine (or poly-L-lysine), laminin, gelatin, collagen, keratin, fibronectin, vitronectin, elastin, heparan sulphate, dextran, dextran sulphate, chondroitin sulphate or a mixture of laminin, collagen I, heparan sulfate proteoglycans, and entactin 1. In some embodiments, the matrix component is an extracellular matrix component. In some embodiments the coating comprises one or more extracellular matrix proteins crosslinked with a dialdehyde, for example glutaraldehyde

In some embodiments, the microcarriers are coated with combinations of adhesion promoting molecules, including combinations of matrix components. In some embodiments the microcarriers are coated with combinations of fibronectin, and/or poly-L-lysine, and/or vitronectin. In some embodiments the combinations of attachment proteins are provided in layers, on the same microcarrier. In some embodiments the microcarrier may be a PCL microcarrier coated with layers of fibronectin, then poly-L-lysine, then fibronectin. In some embodiments the microcarrier may be a PCL microcarrier coated with fibronectin and/or poly-L-lysine, and/or vitronectin, or fibronectin, then poly-L-lysine, then fibronectin.

Advantageously, combinations of attachment substrates may give better adhesion of cells to and/or proliferation of cells on the microcarriers. For example, use of fibronectin, and/or poly-L-lysine, and/or vitronectin, may allow mesenchymal stem cell proliferation in static or agitated culture, where such attachment substrates allow cell adhesion and proliferation during agitation. In some embodiments, the coating may be a fibronectin-PLL-fibronectin (FN-PLL-FN) coating, or a fibronectin-PLL-vitronectin (FN-PLL-VN) coating.

In some embodiments, the PCL microcarrier or plurality of PCL microcarriers is further coated with cells, and/or has cells attached thereto.

The cells may be of any kind, e.g. somatic cells. In some preferred embodiments the cells are stem cells, which may be pluripotent (e.g. embryonic stem cells of induced pluripotent stem cells) or multipotent (e.g. adult stem cells). The cells may be mesenchymal stem cells (MSCs), embryonic stem cells (ESCs) or induced pluripotent stem cells (iPS). Cells may be non-human, e.g. rabbit, guinea pig, rat, mouse or other rodent (including cells from any animal in the order Rodentia), cat, dog, pig, sheep, goat, cattle, horse, non-human primate or other non-human vertebrate organism; and/or non-human mammalian cells; and/or human cells.

While human embryonic stem cells typically grow in aggregates, mesenchymal stem cells are generally cultured in a single, or quasi-single layer. The latter presents a significant challenge, as the cells' evolution is altered after reaching confluence (i.e. when the cells cover the entire surface and begin to laterally exert pressure on each other). For this application, uniform microcarrier size enables the cultured cells to reach confluence at approximately the same time. Were the surface area of the microcarriers to differ greatly, cultured cells would reach confluence at different times, resulting in an inhomogeneous cell population. Hence the advantage of a low CV of their diameter (coefficient of variation, representing the ratio of the standard deviation to the mean diameter), less than 10% and preferably less than 5%.

Accordingly, the present disclosure provides a PCL microcarrier or plurality of PCL microcarriers having a coefficient of variation (CV) of their diameter of less than 10%. In some embodiments, the CV of their diameter is one of less than 9%, less than 8%, less than 7% or less than 6%. In some embodiments, the CV of the diameter is less than 5%. In some embodiments, the CV of their diameter is one of less than 4%, less than 3%, less than 2% or less than 1%. Advantageously, cultured cells, seeded on such microcarriers at uniform density reach confluence at approximately the same time.

Also disclosed herein is a PCL microcarrier or a plurality thereof having a mean diameter of about 50 to about 400 microns. In some embodiments the mean diameter is about 75 to about 300 microns, about 100 to 250 microns, about 150 to 200 microns.

Microcarrier density is preferably suitable to allow suspension of the microcarriers in suspension cell cultures, e.g. in agitated bioreactors.

The present invention provides a PCL microcarrier, or plurality thereof, having a density of 1.03 to 1.09 g/cm³.

Also disclosed herein is a PCL microcarrier or a plurality thereof having a density of 1.01 to 1.09 g/cm³. This may be one of:
1.01 to 1.09 g/cm³, 1.01 to 1.085 g/cm³, 1.01 to 1.08 g/cm³, 1.01 to 1.07 g/cm³, 1.01 to 1.06 g/cm³, 1.01 to 1.05 g/cm³, 1.01 to 1.04 g/cm³, 1.01 to 1.03 g/cm³, 1.01 to 1.02 g/cm³;
1.02 to 1.09 g/cm³, 1.02 to 1.085 g/cm³, 1.02 to 1.08 g/cm³, 1.02 to 1.07 g/cm³, 1.02 to 1.06 g/cm³, 1.02 to 1.05 g/cm³, 1.02 to 1.04 g/cm³, 1.02 to 1.03 g/cm³;
1.03 to 1.09 g/cm³, 1.03 to 1.085 g/cm³, 1.03 to 1.08 g/cm³, 1.03 to 1.07 g/cm³, 1.03 to 1.06 g/cm³, 1.03 to 1.05 g/cm³, 1.03 to 1.04 g/cm³;
1.04 to 1.09 g/cm³, 1.04 to 1.085 g/cm³, 1.04 to 1.08 g/cm³, 1.04 to 1.07 g/cm³, 1.04 to 1.06 g/cm³, 1.04 to 1.05 g/cm3;
1.05 to 1.09 g/cm³, 1.05 to 1.085 g/cm³, 1.05 to 1.08 g/cm³, 1.05 to 1.07 g/cm³, 1.05 to 1.06 g/cm³.

A preferred density is in the range about 1.02 to about 1.05 g/cm³. Suitable densities include one of about 1.02, 1.025, 1.03, 1.035, 1.04, 1.045, 1.05 g/cm³.

In some embodiments disclosed herein the density is about 1.03 to 1.1 g/ml.

In some embodiments the microcarriers are substantially spherical in shape.

PCL microcarriers may be provided in dried form. They may be substantially purified and isolated from contaminants. They may be provided in sterile or non-sterile form.

Also disclosed herein is sealed packaging containing at least 0.1 gram of PCL microcarriers according to the present disclosure. The PCL microcarriers may be provided in dried form.

Packaging may comprise a container, e.g. bag, box, vial, bottle, or jar. Packaging may be made from a plastics material and may be waterproof so as to keep the contents dry. The microcarriers may be non-sterile or sterile. Packaging may contain one of at least 0.2 g, 0.3 g, 0.4 g. 0.5 g, 0.6 g, 0.7 g, 0.8 g, 0.9 g, 1.0 g, 2.0 g, 3.0 g, 4.0 g, 5.0g, 10 g, 20g, 30g, 40 g, 50 g, 100 g, 200 g, 300 g, 400 g, 500 g, 600 g, 700 g, 800 g, 900 g, 1 kg or more of PCL microcarriers. The weight is preferably dry weight.

Also disclosed herein is a process, e.g. method of manufacture, for making microspheres comprising (i) injecting a first liquid through an outlet in an injector into a second liquid, said outlet being immersed in the second liquid which flows past said outlet during said injecting, wherein the first liquid comprises a polymer and wherein the first and second liquids are sufficiently immiscible that droplets of the first liquid form in the second liquid; and (ii) forming the microspheres from the droplets. In some embodiments the microspheres are biodegradable and/or bioresorbable. In some embodiments the polymer is caprolactone or a copolymer thereof.

Also disclosed herein is a process for producing a PCL microcarrier, comprising (i) injecting a first liquid through an outlet in an injector into a second liquid, the outlet being immersed in the second liquid which flows past the outlet during the injecting, wherein the first liquid comprises caprolactone or a copolymer thereof and wherein the first and second liquids are sufficiently immiscible that droplets of the first liquid form in the second liquid, and (ii) forming microcarriers from the droplets.

In some embodiments the rate of flow of the first liquid is the same or slower than the rate of flow of the second liquid. In some embodiments the ratio of the flow rate of the first liquid to second liquid is from about 1:1 to about 1:10, optionally one of about 1:2 to about 1:5, or about 1:2 to about 1:3. It may be one of about 1:1, 1:2, 1:3, 1:4, 1:5, 1:6, 1:7, 1:8, 1:9, or 1:10.

In some embodiments, the outlet diameter has a diameter of about 0.05 mm to about 0.3 mm, and the ratio of the flow rate of the first liquid and the flow rate of the second liquid is about 1:1 to about 5:1.

Advantageously, the resulting microcarriers obtained after step (ii) may have a mean diameter of about 50 to 400 microns, and/or a coefficient of variation of the diameter of less than 10%, preferably less than 5%.

In some embodiments the method is a solvent-free method, whereby PCL is heated and extruded by microfluidics into a cold water bath.

In some embodiments, the first liquid additionally comprises any one or more of gelatin, heparin, chitosan, hydroxyapatite, chondroitin sulfate, collagen, a polypeptide, a polysaccharide, or an aqueous solution such as a hydrogel, optionally comprising an artificial molecule, such as polyethylene oxide, polyoxazoline, or polyacrylamide. In some embodiments the polymer has an average molecular weight of about 2000 to about 100,000, or about 60,000 to 100,000 or about 50,000 to about 100,000 as measured by GPC. In some embodiments the first liquid additionally comprises a solvent for the polymer, whereby the first liquid is a solution of the polymer in the solvent. In some embodiments the first liquid has a specific gravity greater than that of the second liquid.

In some embodiments, the solvent is a chlorinated or brominated solvent. In some embodiments the solvent is a chlorinated hydrocarbon, optionally dichloromethane (DCM).

In some embodiments the polymer has a concentration in the first liquid of about 0.1 to about 10% w/v, optionally one of about 0.1 to about 50%, about 0.5 to about 10%, or about 0.5 to about 4% w/v. In some embodiments the first liquid is solvent free, whereby the first liquid comprises a melt of the polymer.

In some embodiments the second liquid is an aqueous liquid. In some embodiments the second liquid comprises a polymeric stabiliser in solution.

In some embodiments neither the first nor the second liquids contains a surfactant other than the polymeric stabiliser. In some embodiments the polymeric stabiliser is polyvinylalcohol or a copolymer thereof. In some embodiments the polyvinylalcohol has a degree of hydrolysis of at least about 75%. In some embodiments the polymeric stabiliser has a concentration in the second liquid of about 0.2 to about 5% w/v, optionally about 0.5 to about 4% w/v.

In some embodiments of the process disclosed herein the polymer comprises ionic monomer units, whereby said polymer is a polyelectrolyte copolymer.

In some embodiments the first liquid additionally comprises a second polymer. In some embodiments the second polymer is a polyelectrolyte.

In some embodiments the first liquid comprises a porogen, optionally a polymeric porogen.

In some embodiments of the process, injecting is such that the first liquid and the second liquid flow in the same direction. In some embodiments, the ratio between the flow rate of the first liquid and the flow rate of the second liquid is about 1:1 to about 5:1, optionally about 2:1 to about 3:1, optionally about 2:1.

In some embodiments of the process, the outlet has an inner diameter of about 0.05 to about 0.3 mm, optionally one of about 0.05 to about 0.2 mm, 0.05 to about 0.1 mm, 0.1 to about 0.3 mm 0.1 to about 0.2 mm 0.1 to about 0.3 mm, or 0.2 to about 0.3 mm. Other injector/needle sizes and tubing diameters may also be used. Different injector/needle diameters, tubing diameters, and flow rates of the continuous and dispersed phases regulates the diameter range of the microspheres.

In some embodiments the injector is in the form of an inner tube and the second liquid flows inside an outer tube which surrounds the inner tube. In some embodiments the first liquid flows at a rate such that droplets of the first liquid form within the second liquid. In some embodiments the flow rate of the second liquid is such that the second liquid is in a laminar flow regime. In some embodiments the flow rate of the first liquid and the flow rate of the second liquid are, independently, between about 1 and about 10,000 microliters per minute. In some embodiments the flow rate of the first liquid is between about 10 and about 500 microliters per minute, optionally between about 10 and 400 microliters per minute. In some embodiments the flow rate of the second liquid is between about 10 and about 500 microliters per minute, optionally between about 10 and 400 microliters per minute.

In some embodiments of the process, the injector comprises a syringe needle and the second liquid flows through a tube, whereby an injecting portion of the syringe needle is substantially concentric with the tube.

In some embodiments of the process, step (i) results in formation of gel microcarriers and step (ii) comprises allowing the gel microcarriers to partially separate from the second liquid, removing the majority of the second liquid from the gel microcarriers and allowing the gel microcarriers to form solid polymer microcarriers.

In preferred embodiments the microcarrier droplets are separated from the liquid phase. In some embodiments the microcarrier droplets are dried.

In some embodiments of the process, the first liquid comprises a bioactive substance such as hydroxyapatite, and the microcarriers comprise the bioactive substance.

In some embodiments, the process additionally comprises the step of treating the microcarriers with an aqueous hydroxide solution. In some embodiments the treatment is for about 1 to about 6 hours, and increases the wettability of the microcarriers. In some embodiments, treatment confers a negative surface charge to the microcarriers. In some embodiments the treatment is for about 1 to about 10 days, and results in porous microcarriers. In some embodiments the hydroxide is sodium hydroxide.

In some embodiments, the process additionally comprises the step of crosslinking the polymer in the microcarriers. In some embodiments crosslinking comprises heating the microcarriers in the presence of a peroxide such as benzoyl peroxide.

In some embodiments, the process additionally comprises coating the microcarriers with a coating comprising one or more adhesion promoting molecules. Adhesion promoting molecules include polypeptides, glycopolypeptides, polysaccharides and cationic polypeptides capable of binding a mammalian surface cell polypeptide, glycopolypeptide, cationic polyelectrolyte or polysaccharide. In some embodiments, the cationic polyelectrolyte is polybrene. In some embodiments the coating comprises poly-L-lysine, an adhesion promoting protein or bovine serum albumin. In some embodiments, the coating comprises one or more matrix components. Matrix components include components of biological matrices. In some embodiments, the matrix component is an extracellular matrix component.

In some embodiments, the process comprises coating the microcarriers with a multilayer coating, wherein a multilayer coating comprises (i) a first layer, comprising an adhesion promoting molecule, and (ii) another layer, comprising an adhesion promoting molecule. In some embodiments the adhesion promoting molecule of the first layer is different from the adhesion promoting molecule of the second layer.

In some embodiments, the microcarriers are sequentially coated with layers of adhesion promoting molecules. In some embodiments, the process comprises first coating the microcarrier with a layer comprising an adhesion promoting molecule, and subsequently coating the microcarriers with another layer comprising an adhesion promoting molecule. In some embodiments the adhesion promoting molecule of the first layer is different from the adhesion promoting molecule of the second layer. In some embodiments, the multilayer coating comprises more than two layers. In some embodiments, the process comprises sequentially coating the microcarrier to yield a microcarrier having a multilayer coating comprising 3, 4, 5, 6, 7, 8, 9 or 10 layers.

In some embodiments the adhesion promoting molecule is a matrix component. In some embodiments, the matrix component is selected from polylysine (or poly-L-lysine), laminin, gelatin, collagen, keratin, fibronectin, vitronectin, elastin, heparan sulphate, dextran, dextran sulphate, chondroitin sulphate or a mixture of laminin, collagen I, heparan sulfate proteoglycans, and entactin 1. In some embodiments, the matrix component is an extracellular matrix component. In some embodiments process additionally comprises treating the microcarriers with a dialdehyde such as glutaraldehyde.

In some embodiments microcarriers are coated with a polypeptide is capable of binding a mammalian cell surface protein. In some embodiments the polypeptide is a polyaminoacid comprising lysine or a lysine derivative. In some embodiments, the polypeptide is an extracellular matrix protein. In some embodiments the extracellular matrix protein is vitronectin, fibronectin, laminin, osteopontin or collagen, or a derivative thereof.

In some embodiments, the process comprises coating the microcarrier with combinations of fibronectin, and/or poly-L-lysine, and/or vitronectin. In some embodiments the process comprises sequentially coating the microcarrier with layers of coating on the same microcarrier. As such, a microcarrier may be coated in a layer of polylysine and/or fibronectin and/or vitronectin, in any order. In some embodiments the process comprises first coating the microcarrier with a layer comprising fibronectin, then a layer comprising poly-L-lysine, then another layer comprising fibronectin. In some embodiments the process comprises first coating the microcarrier with a layer comprising fibronectin then a layer comprising poly-L-lysine, then another layer comprising vitronectin.

In some embodiments, the PCL microcarrier or plurality of PCL microcarriers is further coated with cells, and/or has stem cells attached thereto. In some embodiments the stem cells are mesenchymal stem cells (MSCs) or embryonic stem cells (ESCs). In some embodiments, the MSCs or HSCs are human MSCs or human HSCs, respectively.

Accordingly, in some embodiments, the process additionally comprises exposing the microcarriers to embryonic stem cells or mesenchymal stem cells, to attach the stem cells to the microcarriers. In some embodiments, the process comprises (i) exposing the microcarriers to one or more polypeptides capable of binding a mammalian cell surface protein, and (ii) exposing the microcarriers to embryonic stem cells or mesenchymal stem cells, so as to attach said stem cells to the microcarriers.

Also disclosed herein is a PCL microcarrier sure or plurality thereof obtained by or obtainable by using the process of the present disclosure.

Also disclosed herein is a scaffold comprising, containing or supporting a microcarrier or microcarriers according to the present disclosure.

Also disclosed herein is an implantable device comprising (i) a scaffold comprising a plurality of strands; and (ii) a plurality of PCL microcarriers of the present disclosure disposed in and/or on the scaffold.

In some embodiments, the implantable device comprises a radiopaque marker. In some embodiments, the radiopaque marker is barium sulfate or gold nanoparticles.

In some embodiments the strands of the implantable device have a diameter of about 200 to about 350 microns, optionally from about 230 to about 250 microns. In some embodiments, the strands are overlaid in a lattice. In some embodiments the lattice has a 60° angle change between adjacent layers. In some embodiments, spacing between adjacent strands is about 400 microns.

In some embodiments of the implantable device, the scaffold is made from a biodegradable or bioresorbable material.

The microcarrier or plurality of microcarriers of the present invention may be used (i) in static or stirred spinner or bioreactor culture of cells; (ii) in tissue engineering; (iii) in generation of inflammatory/ectopic effects; (iv) for expansion of human mesenchymal stem cells and/or embryonic stem cells; (v) in differentiation of human mesenchymal stem cells; (vi) in treatment of, or implantation into, a patient wherein differentiated or undifferentiated mesenchymal stem cells cultured on said microcarriers are implanted into said patient; (vii) in treatment of, or implantation into, a patient wherein differentiated or undifferentiated mesenchymal stem cells are cultured on said microcarriers which are then located in and/or on a scaffold prior to said treatment or implantation into said patient; and (viii) as a vehicle for a radiopaque dye.

In one aspect the present invention provides a method of culturing stem cells as defined in the claims, the method comprising (i) attaching cells, optionally stem cells, to a PCL microcarrier, or to a plurality of PCL microcarriers of the invention and (ii) culturing the microcarrier-cell complexes.

Optionally, the surface of the microcarriers may be coated in a matrix or in two or more layers of matrix.

In some embodiments, the culture may be a static or stirred spinner or bioreactor culture. In some embodiments the method may comprise introducing the microcarrier or plurality of microcarriers of the invention into a liquid compatible with said microcarrier or plurality of microcarriers.

In some embodiments, the number of stem cells after step (ii) is expanded. In some embodiments, culturing the microcarrier-cell complexes comprises suspension culture under static conditions and/or suspension culture with agitation. In some embodiments, the microcarriers may support expansion of various mesenchymal stem cells (bone marrow, fetal, adipose, etc.) in static or stirred spinner or bioreactor cultures.

In some embodiments, culturing the microcarrier-stem cell complexes comprises culture in serum free media.

In some embodiments the cells are stem cells and the method may comprise (iii) passaging the cultured cells from (ii); and repeating steps (i)-(iii) through at least 3 passages, wherein the cells preferably retain their pluripotent or multipotent status after passaging. In some embodiments the method may further comprise the step of inducing differentiation of the stem cells obtained.

In some embodiments the method may comprise placing microcarrier-stem cell complexes under conditions which induce the differentiation of the stem cells. Such methods may comprise attaching stem cells obtained after step (iv) to a plurality of second microcarriers to form microcarrier-stem cell complexes, wherein the surface of the second microcarriers is coated in a matrix or is uncoated; and culturing the microcarrier-stem cell complexes in suspension culture under conditions that induce the differentiation of the stem cells.

In some embodiments the method may comprise the step of separating stem cells from the microcarriers and culturing the separated stem cells in non-microcarrier culture under conditions which induce differentiation of the stem cells.

In one aspect the present invention provides a method of differentiating stem cells as defined in the claims, the method comprising (i) attaching stem cells to a PCL microcarrier or a plurality of PCL microcarriers of the invention and (ii) inducing differentiation of the stem cells. In some embodiments, the microcarriers may support *ex vivo* differentiation of various MSCs (bone marrow, fetal, adipose, etc.) into bone or cartilage in static or stirred spinner or bioreactor cultures. In some embodiments, the microcarriers may support *ex vivo* differentiation of various pluripotent cells (for example human embryonic stem cells, induced pluripotent stem cells, etc.) into differentiated cells from the three germ layers (ectoderm, mesoderm and endoderm) in static or stirred spinner or bioreactor cultures.

The present disclosure also provides PCL microcarriers or a plurality of PCL microcarriers of the invention for use in a method of tissue engineering. Also disclosed is the use of PCL microcarriers or a plurality of PCL microcarriers of the invention in the manufacture of a medicament or pharmaceutical composition for use in a method of tissue engineering. In some embodiments, the methods disclosed herein comprise introducing the microcarriers of the invention into a living organism, optionally by injection. In some embodiments, the methods disclosed herein comprise *in vivo* implantation of PCL microcarriers covered with MSC (derived from bone marrow, fetal, adipose etc.), into the body for tissue engineering or generation of anti-inflammatory/ectopic effects. Cells can be used in their non-differentiated form or differentiated into bone or cartilage. In some embodiments, the methods disclosed herein comprise *in vivo* implantation of PCL microcarriers covered with various pluripotent cells (for example human embryonic stem cells, induced pluripotent stem cells, etc.) differentiated to cells from the three germ layers (ectoderm, mesoderm and endoderm) into the body for tissue engineering purposes.

The benefit of not dissociating the cells from the support on which they are cultured is an improved viability and, potentially, a more rapid adaptation to their role in the tissue engineering application.

Advantageously, in some embodiments disclosed herein the microcarriers at least partially resorb or degrade within the organism without production of products which are toxic or otherwise harmful to the organism.

The present disclosure also provides PCL microcarriers or a plurality of PCL microcarriers of the invention for use in methods of medical treatment. Also disclosed is the use of PCL microcarriers or a plurality of PCL microcarriers of the invention in the manufacture of a medicament or pharmaceutical composition for use in a method of medical treatment.

Also disclosed herein is a method comprising implanting PCL microcarriers into the human or animal body, the PCL microcarriers preferably being attached to cells intended to provide a therapeutic effect at or near the site of implantation.

The following numbered paragraphs (paras.) contain statements of broad combinations of the technical features herein disclosed:
1. The fabrication of microspheres with uniform size (CV below 10% and preferably less than 5%), made from bioresorbable, biodegradeable polymer.
2. These microspheres made from polycaprolactone, which is FDA-approved for implantation.
3. The fabrication of PCL microspheres using a solvent-free method, which relies on heating PCL and extruding it, via microfluidics, into a cold water bath.
4. These microcarriers with a subsequent sodium hydroxide treatment (see example) to increase their wettability and confer a negative surface charge.
5. These microcarriers, coated with poly-L-lysine or other cationic polyelectrolyte, such as polybrene.
6. These microcarriers, with or without NaOH and with or without PLL (or similar), coated with adhesion-promoting protein, examples of which include laminin, vitronectin, collagen, keratin, gelatin, etc.
7. These microcarriers, with or without NaOH and with or without PLL (or similar), coated with BSA or other protein or polysaccharide, such as heparin or heparan sulfate.
8. The inclusion of a second biodegradable polymer, blended into PCL.
9. The inclusion of a second biodegradable polymer, as a biphasic emulsion, within the PCL.
10. The use of a more rapidly biodegradable polymer or a polymer that can be dissolved in a solvent different from PCL (e.g. aqueous or ethanol medium) as a porogen, capable of forming a porous structure within the PCL, be it composed of mostly isolated porous or interconnected pores.
11. The inclusion of a polyelectrolyte within the PCL. This maybe blended into the PCL or in the form of a diblock copolymer, with one segment compatible with PCL and a second segment being the polyelectrolyte. One example of this is polystyrene-polyacrylic acid (PS-PAAc) diblock copolymer, which has been successfully used to confer negative charge to polystyrene microspheres, formed from DCM (as described in the example, above).
12. The inclusion of bioactive material, such as hydroxyapatite for bone formation, or other bioactive molecules, within the PCL. This can be achieved by direct blending of PCL with these materials or by encapsulation to incorporate them within the PCL.
13. The inclusion of a compatible polymer with density less than 1 g/cm³, which can be used to adjust the density of microcarriers that will be used within an agitated bioreactor.
14. PCL microcarriers to support expansion of various MSCs (bone marrow, fetal, adipose etc.) in static or stirred spinner or bioreactor cultures.
15. PCL microcarriers to support expansion of various pluripotent cells (hESC, iPSC etc.) in static or stirred spinner or bioreactor cultures.
16. PCL microcarriers to support ex-vivo differentiation of various MSCs (bone marrow, fetal, adipose etc.) into bone or cartilage in static or stirred spinner or bioreactor cultures.
17. PCL microcarriers to support ex-vivo differentiation of various pluripotent cells (hESC, iPSC etc.) into differentiated cells from the three germ layers (ectoderm, mesoderm and endoderm) in static or stirred spinner or bioreactor cultures.
18. In vivo implantation of PCL microcarriers covered with MSC (derived from bone marrow, fetal, adipose etc.), into the body for tissue engineering or generation of antiinflammatory/ectopic effects. Cells can be used in their non-differentiated form or differentiated into bone or cartilage.
19. In vivo implantation of PCL microcarriers covered with various pluripotent cells (hESC, iPSC etc.) differentiated to cells from the three germ layers (ectoderm, mesoderm and endoderm) into the body for tissue engineering purposes.
20. Ex vivo differentiation of scaffold (from variety of biodegradable materials and having different conformation) filled with PCL MCs covered with MSC (derived from bone marrow, fetal, adipose etc.), into bone or cartilage. Differentiation can be carried out in static or stirred spinner or bioreactor cultures.
21. Ex vivo differentiation of scaffold (from variety of biodegradable materials and having different conformation) filled with PCL MCs covered with various pluripotent cells (hESC, iPSC etc.) differentiated to cells from the three germ layers (ectoderm, mesoderm and endoderm). Differentiation can be carried out in static or stirred spinner or bioreactor cultures.
22. In vivo implantation of scaffold (from variety of biodegradable materials and having different conformation) with PCL MCs covered with MSC (derived from bone marrow, fetal, adipose etc.), into the body for tissue engineering or generation of anti-inflammatory/ectopic effects. Cells can be used in their non-differentiated form or differentiated into bone or cartilage.
23. In vivo implantation of scaffold (from variety of biodegradable materials and having different conformation) with PCL MCs covered with pluripotent cells (hESC, iPSC etc.) differentiated into cells from the three germ layers (ectoderm, mesoderm and endoderm).
24. A process for making microspheres comprising:
   a) injecting a first liquid through an outlet in an injector into a second liquid, said outlet being immersed in the second liquid which flows past said outlet during said injecting, wherein the first liquid comprises a polymer and wherein the first and second liquids are sufficiently immiscible that droplets of the first liquid form in the second liquid; and
   b) forming the microspheres from the droplets.
25. The process of Paragraph 24 wherein the polymer is a biodegradable polymer whereby the microspheres are biodegradable and/or bioresorbable.
26. The process of Paragraph 25 wherein the polymer is caprolactone or a copolymer thereof.
27. The process of Paragraph 26 wherein the first liquid additionally comprises any one or more of gelatin, heparin, chitosan, hydroxyapatite, chondroitin sulfate, collagen, a polypeptide, a polysaccharide, or an aqueous solution such as a hydrogel, optionally comprising an artificial molecule, such as polyethylene oxide, polyoxazoline, or polyacrylamide.
28. The process of any one of Paragraphs 24 to 27 wherein the polymer has a weight average molecular weight of about 2000 to about 100,000, or about 60,000 to 100,000 or about 50,000 to about 100,000 as measured by GPC.
29. The process of any one of Paragraphs 24 to 28 wherein the first liquid additionally comprises a solvent for the polymer, whereby the first liquid is a solution of the polymer in the solvent.
30. The process of Paragraph 29 wherein the first liquid has a specific gravity greater than that of the second liquid.
31. The process of Paragraph 29 or Paragraph 30 wherein the solvent is a chlorinated or brominated solvent.
32. The process of Paragraph 31 wherein the solvent is a chlorinated hydrocarbon, optionally dichloromethane.
33. The process of any one of Paragraphs 29 to 32 wherein the polymer has a concentration in the first liquid of about 0.1 to about 10% w/v, optionally 0.5 to 4% w/v.
34. The process of any one of Paragraphs 24 to 28 wherein the first liquid is solvent free, whereby the first liquid comprises a melt of the polymer.
35. The process of any one of Paragraphs 24 to 34 wherein the second liquid is an aqueous liquid.
36. The process of any one of Paragraphs 24 to 35 wherein the second liquid comprises a polymeric stabiliser in solution.
37. The process of Paragraph 36 wherein neither the first nor the second liquids contains a surfactant other than the polymeric stabiliser.
38. The process of Paragraph 36 or Paragraph 37 wherein the polymeric stabiliser is polyvinylalcohol or a copolymer thereof.
39. The process of Paragraph 38 wherein the polyvinylalcohol has a degree of hydrolysis of at least about 75%.
40. The process of any one of Paragraphs 36 to 39 wherein the polymeric stabiliser has a concentration in the second liquid of about 0.2 to about 5% w/v, optionally about 0.5 to about 4% w/v.
41. The process of any one of Paragraphs 24 to 40 wherein the polymer comprises ionic monomer units, whereby said polymer is a polyelectrolyte copolymer.
42. The process of any one of Paragraphs 24 to 41 wherein the first liquid additionally comprises a second polymer.
43. The process of Paragraph 42 wherein the second polymer is a polyelectrolyte.
44. The process of any one of Paragraphs 24 to 43 wherein the first liquid comprises a porogen, optionally a polymeric porogen, whereby the microspheres are porous.
45. The process of any one of Paragraphs 24 to 44 wherein the injecting is such that the first liquid and the second liquid flow in the same direction.
46. The process of Paragraph 45 wherein the ratio between the flow rate of the first liquid and the flow rate of the second liquid is about 1:1 to about 5:1, optionally about 2:1 to about 3:1, optionally about 2:1.
47. The process of any one of Paragraphs 24 to 46 wherein the outlet has an inner diameter of about 0.05 to about 0.3mm, optionally about 0.1 to 0.2mm.
48. The process of any one of Paragraphs 24 to 47 wherein the injector is in the form of an inner tube and the second liquid flows inside an outer tube which surrounds the inner tube.
49. The process of any one of Paragraphs 24 to 48 wherein the first liquid flows at a rate such that droplets of the first liquid form within the second liquid.
50. The process of any one of Paragraphs 24 to 49 wherein the flow rate of the second liquid is such that the second liquid is in a laminar flow regime.
51. The process of any one of Paragraphs 24 to 50 wherein the flow rate of the first liquid and the flow rate of the second liquid are, independently, between about 1 and about 10,000 microliters per minute.
52. The process of Paragraph 51 wherein the flow rate of the first liquid is between about 10 and about 500 microlitres per minute, optionally between about 10 and 400 microlitres per minute.
53. The process of Paragraph 51 or Paragraph 25 wherein the flow rate of the second liquid is between about 10 and about 500 microlitres per minute, optionally between about 10 and 400 microlitres per minute.
54. The process of any one of Paragraphs 24 to 53 wherein the injector comprises a syringe needle and the second liquid flows through a tube, whereby an injecting portion of the syringe needle is substantially concentric with the tube.
55. The process of any one of Paragraphs 24 to 54 wherein step a) results in formation of gel microparticles and step b) comprises allowing the gel microparticles to partially separate from the second liquid, removing the majority of the second liquid from the gel microparticles and allowing the gel microparticles to form solid polymer microparticles.
56. The process of any one of Paragraphs 24 to 55 wherein the first liquid comprises a bioactive substance such as hydroxyapatite, whereby the microparticles comprise the bioactive substance.
57. The process of any one of Paragraphs 24 to 56 additionally comprising the step of treating the microparticles with an aqueous hydroxide solution.
58. The process of Paragraph 57 wherein the treatment is for about 1 to about 6 hours, such that said treatment increases the wettability of the microparticles.
59. The process of Paragraph 57 or 58 wherein the treatment is for about 1 to about 10 days, such that said treatment results in porous microparticles.
60. The process of any one of Paragraphs 24 to 59 additionally comprising the step of crosslinking the polymer in the microparticles.
61. The process of Paragraph 60 wherein the step of crosslinking comprises heating the microparticles in the presence of a peroxide such as benzoyl peroxide.
62. The process of any one of Paragraphs 24 to 61 additionally comprising exposing the microparticles to an aqueous solution of at least one polypeptide or glycopolypeptide or cationic polyelectrolyte or polysaccharide so as to substantially coat the microparticles in said at least one polypeptide or glycopolypeptide or cationic polyelectrolyte or polysaccharide.
63. The process of Paragraph 62 wherein the at least one polypeptide or glycopolypeptide comprises, or is, polylysine or a protein, such as gelatin, vitronectin or laminin.
64. The process of Paragraph 63 wherein the at least one polypeptide is gelatin and the process additionally comprises treating the resulting microparticles with a dialdehyde such as glutaraldehyde.
65. The process of Paragraph 63 wherein the polypeptide is capable of binding a mammalian cell surface protein.
66. The process of Paragraph 65 wherein the polypeptide capable of binding a mammalian cell surface protein is a polyaminoacid comprising lysine or a lysine derivative.
67. The process of Paragraph 65 or Paragraph 66 wherein the polypeptide capable of binding a mammalian cell surface protein is an extracellular matrix protein.
68. The process of Paragraph 67 wherein the extracellular matrix protein is vitronectin, fibronectin, laminin, osteopontin or collagen, or a derivative thereof.
69. The process of any one of Paragraphs 24 to 68 additionally comprising: a) exposing the microparticles to one or more polypeptides capable of binding a mammalian cell surface protein; and b) exposing the microparticles to embryonic stem cells or mesenchymal stem cells, so as to attach said stem cells to the microparticles.
70. The process of any one of Paragraphs 24 to 69 wherein the microparticles have a coefficient of variation of their diameter of less than about 10%, preferably less than about 5%.
71. Polymeric microparticles comprising a biodegradable polymer, said particles having a coefficient of variation of their diameter of less than about 10%, preferably less than about 5%, said microparticles being solid.
72. The polymeric microparticles of Paragraph 71 made by the process of any one of Paragraphs 1 to 47.
73. The polymeric microparticles of Paragraph 71 or Paragraph 72 having a substantially uniform particle diameter.
74. The polymeric microparticles of any one of Paragraphs 71 to 73 wherein the biodegradable polymer is polycaprolactone or a copolymer thereof.
75. The polymeric microparticles of Paragraph 74 additionally comprising any one or more of gelatin, heparin, chitosan, hydroxyapatite and chondroitin sulfate.
76. The polymeric microparticles of any one of Paragraphs 71 to 75 wherein the biodegradable polymer is a copolymer of an ionic monomer, whereby said polymer is a polyelectrolyte copolymer.
77. The polymeric microparticles of any one of Paragraphs 71 to 76 additionally comprising a polyelectrolyte copolymer which is different to the biodegradable polymer.
78. The polymeric microparticles of Paragraph 77 having a morphology selected from the group consisting of a homogeneous polymer blend, an interpenetrating polymer network, a dispersion of a discontinuous polymeric phase in a continuous polymeric phase and a core shell structure.
79. The polymeric microparticles of any one of Paragraphs 71 to 78 wherein said microparticles are substantially spherical or are substantially cylindrical.
80. The polymeric microparticles of any one of Paragraphs 71 to 79 having a mean diameter of about 50 to about 400 microns, optionally about 150 to about 250 microns, preferably about 160 to about 200 microns.
81. The polymeric microparticles of any one of Paragraphs 71 to 80 having a density of 1.03 to 1.1, optionally 1.03 to 1.05 or 1.08 to 1.1.
82. The microparticles of any one of Paragraphs 71 to 81 having a hydrophilic surface.
83. The microparticles of Paragraph 82, said microparticles being wettable in water.
84. The microparticles of any one of Paragraphs 71 to 83, said microparticles having less than 0.01% surfactant by weight.
85. The microparticles of any one of Paragraphs 71 to 84, said microparticles being porous, optionally macroporous.
86. The microparticles of any one of Paragraphs 71 to 85 comprising a bioactive substance such as hydroxyapatite.
87. The microparticles of any one of Paragraphs 71 to 86, said microparticles being substantially completely coated with a coating comprising one or more polypeptides or glycopolypeptides capable of binding a mammalian surface cell protein or cationic polyelectrolytes or polysaccharides.
88. The microparticles of Paragraph 87 wherein the coating comprises poly-L-lysine, an adhesion promoting protein or bovine serum albumin.
89. The microparticles of Paragraph 88 wherein the coating comprises one or more extracellular matrix proteins crosslinked with a dialdehyde, for example glutaraldehyde.
90. The microparticles of any one of Paragraphs 87 to 89, said coating having embryonic stem cells or mesenchymal stem cells attached thereto.
91. Use of microparticles according to any one of Paragraphs 71 to 90 in any one of the following applications:
   i) a static or stirred spinner or bioreactor culture;
   ii) tissue engineering;
   iii) generation of inflammatory/ectopic effects;
   iv) expansion of human mesenchymal stem cells and/or embryonic stem cells;
   v) differentination of human mesenchymal stem cells
   vi) treatment of, or implantation into, a patient wherein differentiated or undifferentiated mesenchymal stem cells cultured on said microparticles are implanted into said patient;
   vii) treatment of, or implantation into, a patient wherein differentiated or undifferentiated mesenchymal stem cells are cultured on said microparticles which are then located in and/or on a scaffold prior to said treatment or implantation into said patient;
   viii) as a vehicle for a radiopaque dye.
92. A method for preparing a static or stirred spinner or bioreactor culture comprising the step of introducing microparticles according to any one of Paragraphs 71 to 90 into a liquid compatible with said microparticles.
93. A method for tissue engineering or generation of inflammatory/ectopic effects comprising introducing microparticles according to any one of Paragraphs 71 to 90 into a living organism.
94. The method of Paragraph 93 wherein the introducing comprises injecting said microparticles into said organism.
95. The method of Paragraph 93 or Paragraph 94 wherein the microparticles at least partially resorb or degrade within the organism without production of products which are toxic or otherwise harmful to the organism.
96. The method of any one of Paragraphs 92 to 95 wherein the organism is a non-human organism.
97. An implantable device comprising:
   i) a scaffold comprising a plurality of strands; and
   ii) a plurality of microparticles according to Paragraph 90 disposed in and/or on said scaffold.
98. The implantable device of Paragraph 97 wherein either the scaffold or the microparticles or both comprise a radiopaque marker.
99. The implantable device of Paragraph 98 wherein the radiopaque marker is barium sulfate or gold nanoparticles.
100. The implantable device of any one of Paragraphs 97 to 99 wherein the strands have a diameter of about 200 to about 350 microns, optionally from about 230 to about 250 microns.
101. The implantable device of any one of Paragraphs 97 to 100 wherein the strands are overlaid in a lattice.
102. The implantable device of Paragraph 101 wherein the lattice has a 60° angle change between adjacent layers.
103. The implantable device of any one of Paragraphs 97 to 102 having a spacing between adjacent strands of about 400 microns.
104. The implantable device of any one of Paragraphs 97 to 103 wherein the scaffold is made from a biodegradable or bioresorbable material.
105. The implantable device of Paragraph 104 wherein the biodegradable or bioresorbable material is polycaprolactone or a copolymer of caprolactone.
106. The use of attachment substrates to coat biodegradable microcarriers, such that mesenchymal stem cells can attach and proliferate on these microcarriers in serum-containing or serum-free media.
   a. Such biodegradable microcarriers include polycaprolactone microcarriers, coated with combinations of fibronectin, and/or poly-L-lysine, and/or vitronectin.
   b. The use of multiple layers of such attachment substrates on the same microcarrier, which in combination may give better adhesion and/or proliferation of mesenchymal stem cells on the microcarrier.
   c. Examples of such combinations include but are not limited to PCL coated with layers of fibronectin, then poly-L-lysine, then fibronectin.
   d. The inclusion of vitronectin as attachment substrate to allow mesenchymal stem cell adhesion and/or growth on PCL microcarriers in serum-free media. One manifestation of this technology is PCL coated with fibronectin, and/or poly-L-lysine, and/or vitronectin.
   e. The use of fibronectin, and/or poly-L-lysine, and/or vitronectin, to allow mesenchymal stem cell proliferation in static or agitated culture, where such attachment substrates allow cell adhesion and proliferation during agitation. Examples include the use of fibronectin-PLL-fibronectin coatings to allow mesenchymal stem cell adhesion and growth in serum-containing media, and the use of fibronectin-PLL-vitronectin to allow mesenchymal stem cell adhesion and growth in serum-free media.

107. Serum-free media in this application includes any media containing the absence of serum, human or bovine, or other animals. This includes commercially available serum-free media such as Biological Industries MSC Nutristem SFM XF, and Gibco StemPro MSC SFM XF. Other examples of serum-free media include the KA3 serum-free media included in this application.

### Description

Microcarriers of the pesent disclosure consist of, or comprise, polycaprolactone (PCL). In some embodiments the microcarriers are pure or near pure polycaprolactone. In other embodiments the polycaprolactone may be blended with one or more other polymers, active substances or selected agents.

Microcarriers may comprise, or be manufactured from material having, at least 30% PCL, or one of at least 40%, 50%, 60%, 70%, 75%, 80%, 85%, 90%, 91%, 92%, 93%, 94%, 95%, 96%, 97%, 98%, 99% or 100% PCL.

Varying grades of PCL, including medical grade PCL, potentially composed of different molecular weight distributions, may similarly be used to fabricate microspheres, as described below.

PCL preferably has the molecular formula (C₆H₁₀O₂)ₙ. PCL may be prepared by ring opening polymerization of ε-caprolactone using a catalyst. PCL is biodegradable by hydrolysis of its ester linkages, including under physiological conditions in the human or animal body and is therefore suitable for use in implants.

Microcarriers of the present disclosure may have a surface area in the range about 300 to about 700 cm²/g (dry weight). This may be one of about 300 to about 600, about 300 to about 500, about 300 to about 400, about 400 to about 700, about 400 to about 600, about 400 to about 500, about 500 to about 700, about 500 to about 600, or one of about 300, 350, 400, 450, 500, 550, 600, 650 or 700 cm²/g (dry weight).

The number of microcarriers per gram (dry weight) may be in the range about 0.25x10⁸ to about 3.2x10⁸. This may be one of about 0.25x10⁸ to 3x10⁸, about 0.25x10⁸ to 2.5x10⁸, about 0.25x10⁸ to 2x10⁸, about 0.25x10⁸ to 1.5x10⁸, about 0.25x10⁸ to 1x10⁸, about 0.25 x10⁸ to 0.5x10³, about 0.3x10⁸ to 3x10⁸, about 0.3x10⁸ to 2.5x10⁸, about 0.3x10⁸ to 2 x10⁸, about 0.3x10⁸ to 1.5x10⁸, about 0.3x10⁸ to 1x10⁸, about 0.3x10⁸ to 0.5x10⁸, about 0.4x10⁸ to 3x10⁸, about 0.4x10⁸ to 2.5x10⁸, about 0.4x10⁸ to 2x10⁸, about 0.4x10⁸ to 1.5 x10⁸, about 0.4x10⁸ to 1x10⁵, about 0.4x10⁸ to 0.5x10⁸, about 0.5x10⁸ to 3x10⁸, about 0.5 x10⁸ to 2.5x10⁸, about 0.5x10⁸ to 2x10⁸, about 0.5x10⁸ to 1.5x10⁸, about 0.5x10⁸ to 1 x10⁸, about 0.75x10⁸ to 3x10⁸, about 0.75x10⁸ to 2.5x10⁸, about 0.75x10⁸ to 2x10⁸, about 0.75x10⁸ to 1.5x10⁸, about 0.75x10⁸ to 1x10⁸, about 1x10⁸ to 3x10⁸, about 1x10⁸ to 2.5 x10⁸, about 1x10⁸ to 2x10⁸, about 1x10⁸to 1.5x10⁸, about 1.5x10⁸ to 3x10⁸, about 1.5x10⁸ to 2.5x10⁸, about 1.5x10⁸ to 2x10⁸, about 2x10⁸ to 3x10⁸, about 2x10⁸ to 2.5x10⁸, about 2.5x10⁸ to 3x10⁸. The number of microcarriers per gram (dry weight) may be about 0.25 x10⁸, 0.5x10⁸, 0.75x10⁸, 1.0x10⁸, 1.25x10⁸, 1.5x10⁸, 1.75x10⁸, 2.0x10⁸, 2.25x10⁸, 2.5 x10⁸, 2.75x10⁸, or 3.0x10⁸.

The microcarrier may have defined size distribution parameters. For example, the mass-median diameter (MMD or dso) may be in the range about 75 µm to about 190 µm, e.g. one of about 75 µm to about 180 µm, about 75 µm to about 170 µm, about 75 µm to about 160 µm, about 75 µm to about 150 µm, about 75 µm to about 140 µm, about 75 pm to about 130 µm, about 80 µm to about 180 µm, about 80 µm to about 170 µm, about 80 µm to about 160 µm, about 80 µm to about 150 µm, about 80 µm to about 140 µm, about 80 µm to about 130 µm, about 90 µm to about 180 µm, about 90 µm to about 170 µm, about 90 µm to about 160 µm, about 90 µm to about 150 µm, about 90 µm to about 140 µm, about 90 µm to about 130 µm, about 100 µm to about 180 µm, about 100 µm to about 170 µm, about 100 µm to about 160 µm, about 100 µm to about 150 µm, about 100 µm to about 140 µm, about 100 µm to about 130 µm, about 110 µm to about 180 µm, about 110 µm to about 170 µm, about 110 µm to about 160 µm, about 110 µm to about 150 µm, about 110 µm to about 140 µm, about 110 µm to about 130 µm, about 120 µm to about 180 µm, about 120 µm to about 170 µm, about 120 µm to about 160 µm, about 120 µm to about 150 µm, about 120 µm to about 140 µm, about 120 µm to about 130 µm.

The microcarrier may have a d₅₀ of one of about 75 to 80 µm, 80 to 85 µm, 85 to 90 µm, 90 to 95 µm, 95 to 100 µm, 100 to 105 µm, 105 to 110 µm, 110 to 115 µm, 115 to 120 µm, 120 to 125 µm, 125 to 130 µm, 130 to 135 µm, 135 to 140 µm, 140 to 145 µm, 145 to 150 µm, 150 to 155 µm, 155 to 160 µm, 160 to 165 µm, 165 to 170 µm, 170 to 175 µm, 175 to 180 µm, 180 to 185 µm, or 185 to 190 µm.

The microcarrier may have a d₅₀ of one of about 75, 80, 85, 90, 95, 100, 105, 110, 115, 120, 125, 130, 135, 140, 145, 150, 155, 160, 165, 170, 175, 180, 185 or 190 µm.

None porous pure PCL has a density of about 1.145 g/cm³ at 25°C stp. Surface pores in the microcarriers reduce the overall density of PCL microcarriers. The microcarrier may have a pore size in the range 0.1 to 1.5 µm.

### Suspension Culture and Passage of Stem Cells

We have previously demonstrated that it is possible to culture, propagate and passage stem cells, including primate and human stem cells and induced pluripotent stem cells on particles having a matrix coating (e.g. see WO2009/116951 and WO2010/107392). In particular, we have shown that stem cells may be grown continuously on matrix-coated microcarriers in suspension culture and passaged.

Methods may comprise growing, propagating, proliferating, culturing or expanding stem cells, preferably *in vitro,* or increasing the number of stem cells. The stem cells may be passaged for one or more passages, as described below.

The methods may comprise the step of providing particles. The particles may be uncoated or may comprise a matrix coated thereon. They may have a positive charge or have a coating that provided a positively charged surface. The particles may have a size to allow aggregation of stem cells attached thereto. Stem cells may be allowed to attach to the particle. The cells growing on different particles may be allowed to contact each other and to form aggregates. The culture may be passaged for at least one passage. The stem cells may be used attached to the carriers or detached or separated from them. They may be used in an undifferentiated or pluripotent/multipotent state or both, or may be differentiated into a desired cell type. They may be used to form embryoid bodies.

In order for the particles to support continuous growth, they should have a size which is compatible with the dimensions of a stem cell, e.g. a primate or human stem cell, such as one of about 10µm, 20µm, 30µm, 40µm, 50µm, 60µm, 70µm, 80µm, 90µm, 100µm, 110µm, 120µm, 130µm, 140µm, 150µm, 160µm, 170µm, 180µm, 190µm, 200µm, 210µm, 220µm, 230µm, 240µm, 250µm, 260µm, 270µm, 280µm, 290µm, 300µm, 310µm, 320µm, 330µm, 340µm, 350µm, 360µm, 370µm, 380µm, 390µm, or 400µm or so. Culture of stem cells on such a particle with this order of size will enable cells growing thereon to aggregate with each other and support continuous growth. Suitable compositions, shapes and sizes of particles are described in further detail below.

The Examples show that stem cell cultures may be inoculated onto microcarrier particles and grown in suspension culture for several generations with one or more passages. The stem cells may be passaged by dislodging from the surface by any means such as mechanical or enzymatic dissociation, or combination of both methods.

The microcarrier particle cultures may be grown from generation to generation on particles. Alternatively, or in addition, the cultures may be grown on conventional 2D cultures for one or more generations in between. Stem cells growing on microcarriers may be transferred back to 2D colony cultures and vice versa.

The methods described here make available methods for efficient propagation of stem cells in undifferentiated form. The microcarrier cultures may be passaged onto microcarriers by mechanical or enzymatic dissociation with a splitting ratio of between 1 to 2 and 1 to 10, which is higher than is typically possible for conventional 2D cultures. This enables more efficient utilisation of biomaterial with more rapid scale up of culture.

Volumetric yields of cells in microcarrier cultures are routinely 2 to 4 times more than 2D colony controls. The volumetric yield of human stem cells propagated by the methods described here may be up to 2 million cells/ml or more.

The methods described here enable the passaging of stem cells from particles to particles for 9 passages or more, as described in further detail below.

The methods described here enable the propagation of stem cells that retain their pluripotent or multipotent character. Thus, propagated stem cells may show expression of pluripotent markers, Oct-4, Tra-1-60 and mAb 84 for 5 or more passages equivalent to stem cells grown as 2D colony cultures, retain a normal karyotype.

Significantly, by anchoring stem cells on microcarriers, it is possible to serially passage the cells in larger scale spinner flasks.

Any stem cells may be propagated using the methods described here. These may comprise primate stem cells, such as monkey, ape or human stem cells. The stem cells may comprise embryonic stem cells or adult stem cells. The stem cells may comprise induced pluripotent stem cells. For example, the stem cells may comprise human embryonic stem cells (hESCs). These and other stem cells suitable for use in the methods and compositions described here are described in further detail below.

The methods and compositions described here have various advantages over known "2D" culture methods. The particles are more efficient in attaching stem cells than 2D colony culture substrates. For this and other reasons, the suspension cultured cells are able to be passaged more effectively. The methods described here enable the stem cells to be frozen and thawed through several cycles. They may be frozen directly on the microcarriers and thawed onto growing medium (whether traditional plate culture, or on particulate microcarriers). The stem cells propagated on microcarriers may be grown in serum free media, which is GMP compliant.

The methods described here essentially enable the culture and maintenance of stem cells such as embryonic stem cells in an undifferentiated state. The propagated stem cells may be differentiated partially or totally, in culture (e.g., on microcarriers) or detached therefrom.

The propagated stem cells may be used to form embryoid bodies for further use. Stem cells growing on microcarriers may simply be transferred to differentiation medium to form embryoid bodies directly, in contrast with prior methods, which require an additional step of removing cells from a 2D growing surface prior to embryoid body formation.

Accordingly, the methods and compositions described here enable directed differentiation of stem cells on the growing surface or substrate without removal therefrom.

The methods and compositions described here enable expansion and scale up of cultured stem cells to larger volumes. The scale up to bioreactor or industrial scale enables more productive culture of stem cells. The ability to grow stem cells on microcarriers in agitated culture means that the cultures can be scaled up into suspension conditions. Controlled bioreactors such as the Wave Bioreactor or stirred cultures may be used. This enables cells to be expanded in larger volumes compared to the current limitations of anchorage dependent 2 dimensional colony cultures. Large scale suspension culture in bioreactors up to 100's of litres is possible.

### Positive Charge

The particle or microcarrier may comprise a positive charge at for example neutral pH or physiologically relevant pH such as pH 7.4 or pH 7.2.

The quantity of positive charge may vary, but in some embodiments is intended to be high enough to enable cells to attach to the particle. For example, where the particles are charged by coupling with amines, such as quaternary or tertiary amines, the charge on the , particle may correspond to a small ion exchange capacity of about 0.5 to 4 milli-equivalents per gram dry material (of the particle), for example between about 1 to 3.5 milli-equivalents per gram dry material (of the particle) or between about 1 to 2 milli-equivalents per gram dry material (of the particle).

The positive charge may be such that that the pKa of the particle is greater than 7 (e.g., greater than 7.4, e.g., 7.5, 8, 8.5, 9, 9.5, 10, 10.5, 11, 11.5 or more).

The particle may be derivatised by coupling for example to protamine sulphate or poly-L-lysine hydrobromide at a concentration of up to 20mg/ml particles.

Without wishing to be bound by theory, we believe that the presence of a positive charge on the particles assists attachment of cells thereto.

The particle may carry a positive charge through any means known in the art. The particle may comprise positively charged groups, or it may be derivatised to carry these.

The particle may be derivatised to carry positive charges. For example, the particle may comprise amine groups attached thereto. The amine groups may be primary amine groups, secondary amine groups, tertiary amine groups or quaternary amine groups. The amine groups may be attached to the particle by coupling the particle with amine containing compounds. Methods of coupling are well known in the art. For example, the amine may be coupled to the particle by the use of cyanogen bromide.

Crosslinkers may also be used. These are divided into homobifunctional crosslinkers, containing two identical reactive groups, or heterobifunctional crosslinkers, with two different reactive groups. Heterobifunctional crosslinkers allow sequential conjugations, minimizing polymerization. Coupling and crosslinking reagents may be obtained from a number of manufacturers, for example, from Calbiochem or Pierce Chemical Company.

The particle may be activated prior to coupling, to increase its reactivity. The compact particle may be activated using chloroacetic acid followed by coupling using EDAC/NHS-OH. Particles may also be activated using hexane di isocyanate to give a primary amino group. Such activated particles may be used in combination with any heterobifunctional cross linker.

The compact particle in certain embodiments is activated using divinyl sulfon. Such activated compact particles comprise moieties which can react with amino or thiol groups, on a peptide, for example.

The particle may also be activated using tresyl chloride, giving moieties which are capable of reacting with amino or thiol groups. The particle may also be activated using cyanogen chloride, giving moieties which can react with amino or thiol groups.

### Matrix Coating

In many embodiments the microcarriers are coated in a matrix. One or several layers of matrix coating may be applied to a microcarrier.

Accordingly, the particle may be coated with a matrix, which in the context of this document refers to a layer (e.g. a thin layer or film) of substance attached to the particle such as on its surface. The matrix may comprise a biologically or compatible or physiologically relevant matrix capable of supporting growth of cells. It may comprise a substrate for cell growth.

The matrix may comprise a component of the extracellular matrix (ECM). Any of the known components of the ECM such as those capable of supporting growth of stem cells may be used. Components of the extracellular matrix are known in the art and are described in for example Alberts et al. (2002), Molecular Biology of the Cell, Chapter IV and references cited therein.

Matrix materials may include poly-L-lysine, laminin, gelatin, collagen, keratin, fibronectin, vitronectin, elastin, heparan sulphate, dextran, dextran sulphate, chondroitin sulphate or a mixture of laminin, collagen I, heparan sulfate proteoglycans, and entactin 1, and derivatives or fragment thereof..

The ECM component may be attached or coupled to or coated on the particle through conventional means. For example, any of the coupling reagents and crosslinkers described above may be used to couple the ECM component to the particle.

The ECM component may comprise a macromolecule such as a polysaccharide, protein, proteoglycan, glycoprotein, glycosaminoglycan (GAG), usually found covalently linked to protein in the form of proteoglycans, a fibrous protein, including elastin, fibronectin, and laminin, collagen (e.g. collagen I, collagen III, collagen IV, collagen VI) etc.

The matrix coating may comprise a glycosaminoglycan (GAG). Glycosaminoglycans comprise unbranched polysaccharide chains composed of repeating disaccharide units. One of the two sugars in the repeating disaccharide is always an amino sugar (N-acetylglucosamine or N-acetylgalactosamine), which in most cases is sulfated. The second sugar is usually a uronic acid (glucuronic or iduronic).

The matrix coating may comprise hyaluronan (also called hyaluronic acid or hyaluronate) or a derivative thereof. The hyaluronic acid may be derived from any number of sources, such as from bovine vitreous humor. A salt or base of hyaluronic acid may be employed, such as hyaluronic acid sodium. This may be from streptococcus.

The matrix coating may comprise laminin and/or fibronectin and/or vitronectin and/or collagen and/or keratin and/or gelatin.

The matrix coating may comprise for example a GAG such as chondroitin sulfate, dermatan sulfate, heparan sulfate and keratan sulfate, for example as linked to a protein as a proteoglycan. The matrix coating may comprise aggrecan, decorin, etc.

The matrix coating may comprise heparan or its derivatives such as bases or salts. The matrix coating may comprise heparan sulphate proteoglycan. The heparan sulphate proteoglycan may be derived from any number of sources, such as from bovine kidney.

The matrix coating may comprise a dextran such as dextran sulphate or dextran sulphate sodium. The matrix coating may comprise fibronectin, laminin, nidogen or Type IV collagen. The matrix coating may comprise chondroitin sulphate.

The matrix may comprise gelatin, polyomithine, or binding motifs of the RGD binding domain of fibronectin.

ECM matrix materials include: laminin, fibronectin, vitronectin, collagen, elastin, aggrecan, decorin, chondroitin sulfate, dermatan sulfate, heparan sulphate, keratan sulphate, and hyaluronan.

The matrix coating may comprise a mixture of any two or more of the components described above in various proportions. The matrix coating may comprise a purified or substantially purified component, e.g. of the ECM. The matrix component may comprise a partially purified component of the ECM. It may comprise an ECM extract such as Matrigel™.

A cell culture may comprise particles having different matrix coatings. For example, a first particle population having a first matrix coating selected from those described above and a second particle population having a second coating selected from those described above.

### Matrigel

The particle may be coated with a matrix coating comprising Matrigel™.

Matrigel is the trade name for a gelatinous protein mixture secreted by mouse tumor cells and marketed by BD Biosciences (Bedford, Massachusetts, USA). This mixture resembles the complex extracellular environment found in many tissues and is used by cell biologists as a substrate for cell culture.

BD Matrigel™ Matrix is a solubilised basement membrane preparation extracted from EHS mouse sarcoma, a tumor rich in ECM proteins. Its major component is laminin (about 56%), followed by collagen IV (about 31%), heparan sulfate proteoglycans, and entactin 1 (about 8%). At room temperature, BD Matrigel™ Matrix polymerizes to produce biologically active matrix material resembling the mammalian cellular basement membrane.

As such, the matrix coating may comprise matrix comprises a mixture of laminin, collagen I, heparan sulfate proteoglycans, and entactin 1.

A common laboratory procedure is to dispense small volumes of chilled (4°C) Matrigel onto a surface such as plastic tissue culture labware. When incubated at 37°C (body temperature) the Matrigel proteins self-assemble producing a thin film that covers the surface.

Matrigel provides a physiologically relevant environment with respect to cell morphology, biochemical function, migration or invasion, and gene expression.

The ability of Matrigel to stimulate complex cell behaviour is a consequence of its heterogeneous composition. The chief components of Matrigel are structural proteins such as laminin and collagen which present cultured cells with the adhesive peptide sequences that they would encounter in their natural environment. Also present are growth factors that promote differentiation and proliferation of many cell types. Matrigel comprises the following growth factors (range of concentrations, average concentration): EGF (0.5-1.3 ng/ml, 0.7 ng/ml), bFGF (< 0.1-0.2 pg/ml, unknown), NGF (< 0.2 ng/ml, unknown), PDGF (5-48 pg/ml, 12 pg/ml), IGF-1 (11-24 ng/ml, 16 ng/ml), TGF-β (1.7-4.7 ng/ml, 2.3 ng/ml). Matrigel contains numerous other proteins in small amounts.

### Alternating matrix coatings

In some embodiments cells may be cultured on a particle having a first matrix coating for one or more passages (e.g. 1, 2, 3, 4, 5, 6, 7, 8, 9, or 10 passages or more), before being transferred to particles having a different (second) matrix coating for one or more passages (e.g. 1, 2, 3, 4, 5, 6, 7, 8, 9, or 10 passages or more). Optionally the cells may then be transferred to particles having a matrix coating different to the second coating, e.g. back to the first matrix coating or to another matrix coating or to uncoated particles.

### Particle Composition

In the methods and compositions described here, stem cells are propagated on particles or microcarriers. The particle may comprise a microcarrier, as described in the IUPAC Compendium of Chemical Terminology (2nd Edition, 1992, Vol. 64, p. 160).

The particle is made of polycaprolactone. The particle may be formulated as a solid material or semi-solid, gel, etc. The particle may be prepared to be porous or non-porous.

The particle is preferably at least reactive to allow attachment of positive charges and/or a matrix coating, or capable of being made reactive by an activator, but may otherwise comprise a generally inert substance.

The particle may be treated prior to allowing cells to grow thereon. Such treatment may seek to achieve greater adherence, availability of charges, biocompatibility, etc, as described elsewhere in this document.

A cell culture may comprise a mixture of more than one type of particle. For example, a first particle population (e.g. of compact shape particles) and a second particle population (e.g. of elongate shape particles). In some embodiments a first cell type, e.g. feeder cells, may be attached to the first particles and a second cell type, e.g. hESCs, may be attached to the second particles. Each particle type may have the same or a different matrix coating. Optionally one or both particle types may not have a matrix coating.

The particle may be a bead or microbead. Suitable microbead sizes include a bead size (mean diameter) of 40-120 µm, 20-50 µm, 20-80 µm, 50-150 µm, 100-300 µm.

The particle may comprise any suitable shape for cell growth, e.g., a compact shape or an elongate shape.

### Compact Shape

Examples of compact shapes are generally spherical shaped particles, ellipsoid shaped particles, or granular shaped particles.

By "compact", we mean a shape which is not generally elongate. In other words, "compact" shapes are those which are generally non-elongate or unextended, or which are not extended in any one dimension. The compact shape may be one which is not generally spread out, or not long or spindly. Therefore, such "compact shapes" generally possess linear dimensions which may be generally similar, or which do not differ by a large amount.

Thus, the ratio of any two dimensions of the compact shape may be 5:1 or less, such as 4:1 or less, such as 3:1, 2.5:1, 2.4:1, 2.3:1, 2.2:1, 2.1:1, 2:1, 1.9:1, 1.8:1, 1.7:1, 1.6:1, 1.5:1, 1.4:1, 1.3:1, 1.2:1, 1.1:1, or less. For example, no two pairs of dimensions may have a ratio of 5:1 or more.

In some embodiments, the longest dimension of the compact shape is less than five times the shortest dimension of the compact shape. In other embodiments, the longest dimension of the compact shape is not significantly greater than the shortest dimension, i.e., the shape is relatively uniform.

The "longest dimension" as the term is used in this document should be taken to mean the length of the major axis, i.e., the axis containing the longest line that can be drawn through the particle. Similarly, the "shortest dimension" is the length of the minor axis, which is the axis containing the shortest line that can be drawn through the particle.

Regular shapes in which the linear dimensions are approximately the same, or are comparable, or in which the ratio of the longest dimension to the shortest dimension is less than 5:1 are included in the compact particles described here. The above ratios may therefore relate to the ratio of the longest dimension to the shortest dimension. In some embodiments, the ratio of two dimensions (such as the longest dimension to the shortest dimension) is less than 1.1:1, such as 1:1 (i.e., a regular or uniform shape).

Therefore, where applicable, the length of the particle may be less than 5x its width or diameter, such as less than 4x its width or diameter, such as less than 3x, such as less than 2x or less.

The compact shape may comprise a regular solid, a sphere, a spheroid, an oblate spheroid, a flattened spheroid, an ellipsoid, a cube, a cone, a cylinder, or a polyhedron. Polyhedra include simple polyhedra or regular polyhedra. Polyhedra include, for example, a hexahedron, holyhedron, cuboid, deltahedron, pentahedron, tetradecahedron, polyhedron, tetraflexagon, trapezohedron, truncated polyhedron, geodesic dome, heptahedron and hexecontahedron. Any of the above shapes may be used such that they are "compact", according to the definition provided above. For example, where the shape comprises an oblate spheroid, this has the appropriate oblateness such that the spheroid is compact, and not elongate.

In some embodiments, the compact shape may comprise a balloon shape, a cigar shape, a sausage shape, a disc shape, a teardrop shape, a ball shape or an elliptical shape, so long as the dimensions are as given above. The compact shape may also comprise a sphere shape, a cube shape, a cuboid shape, a tile shape, an ovoid shape, an ellipsoid shape, a disc shape, a cell shape, a pill shape, a capsule shape, a flat cylinder shape, a bean shape, a drop shape, a globular shape, a pea shape, a pellet shape, etc.

### Elongate Shape

The particle may have a generally elongate shape. Examples of elongate shapes are generally rod shaped particles, cylindrical shaped particles, or stick shaped particles. The elongate particles may comprise hollow fibres.

By "elongate", we mean a shape which is not generally compact. In other words, "elongate" shapes are those which are generally extended in one dimension relative to another. The elongate shape may be one which is spread out, long or spindly. Therefore, such "elongate shapes" generally possess linear dimensions which generally differ from one another to a greater or lesser extent.

Thus, the ratio of any two dimensions of the elongate shape may be 5:1 or more, 4:1 or less, such as 1.1:1 or more, 1.2:1 or more, 1.3:1 or more, 1.4:1 or more, 1.5:1 or more, 1.6:1 or more, 1.7:1 or more, 1.8:1 or more, 1.9:1 or more, 2:1 or more, 2.1:1 or more, 2.2:1 or more, 2.3:1 or more, 2.4:1 or more, 2.5:1 or more, 3:1 or more, 4:1 or more, or 5:1 or more.

For example, any two pairs of dimensions may have a ratio of 5:1 or more. Thus, in some embodiments, the longest dimension of the elongate shape is more than five times the shortest dimension of the elongate shape.

Therefore, where applicable, the length of the particle may be more than 2x its width or diameter, such as more than 3x its width or diameter, such as more than 4x, such as more than 5x or more than 10x.

### Particle Size

In order for the particles to support continuous growth, they may have a size which enables cells to grow on the particles. The size of the particles also enables cells to aggregate with other cells growing on other particles. For example, it may be necessary for the size of the particle to be such that at least one dimension is compatible with the dimensions of a primate or human stem cell.

As an example, the particle may comprise a compact microcarrier having a generally spherical or granular shape. Where this is the case, the compact microcarrier may have a dimension ranging between about 50 µm and about 400 µm. In some preferred embodiments the dimension is between 100 µm to 200 µm.

The upper limit of the range of dimensions (e.g. mean diameter) for the compact microcarrier may be one of about 400 µm, about 390 µm, about 380 µm, about 370 µm, about 360 µm, about 350 µm, about 340 µm, about 333 µm, about 320 µm, about 310 µm, about 290 µm, about 280 µm, about 270 µm, about 260 µm, about 250 µm, about 240 µm, about 230 µm, about 220 µm, about 210 µm, about 200 µm, about 190 µm, about 180 µm, about 170 µm, about 160 µm, about 150 µm, about 140 µm, about 130 µm, about 120 µm, about 110 µm, about 100 µm, about 90 µm, about 80 µm, about 70 µm, about 60 µm, or about 50 µm,.

The lower limit of the range of dimensions (e.g. mean diameter) of the compact microcarrier may be one of about 50 µm, about 60 µm, about 70 µm, about 80 µm, about 90 µm, about 100 µm, about 110 µm about 120 µm, about 130 µm, about 140 µm, about 150 µm, about 160 µm, about 170 µm, about 180 µm, about 190 µm, about 200 µm, about 210 µm, about 220 µm, about 230 µm, about 240 µm, about 250 µm, about 260 µm, about 270 µm, about 280 µm, about 290 µm, about 300 µm, about 310 µm, about 320 µm, about 330 µm, about 340 µm, about 350 µm, about 360 µm, about 370 µm, about 380 µm, about 390 µm, or about 400 µm.

The compact microcarriers may have a dimension (e.g. mean diameter) between 50 µm to 400 µm, 50 µm to 350 µm, 50 µm to 300 µm, 50 µm to 350 µm, 50 µm to 200 µm, 50 µm to 150 µm, 50 µm to 100 µm, 50 µm to 90 µm, 50 µm to 80 µm, 50 µm to 70 µm, or 50 µm to 60 µm.

The compact microcarrier may have a dimension (e.g. mean diameter) of one of about 50 µm, about 60 µm, about 70 µm, about 80 µm, about 90 µm, about 100 µm, about 110 µm, about 120 µm, about 130 µm, about 140 µm, about 150 µm, about 160 µm, about 170 µm, about 180 µm, about 190 µm, about 200 µm, about 270 µm, about 280 µm, about 290 µm, about 300 µm, about 310 µm, about 320 µm, about 330 µm, about 340 µm, about 350 µm, about 360 µm, about 370 µm, about 380 µm, about 390 µm, or about 400 µm.

The dimension may preferably be the mean average diameter of the microcarriers, e.g. of the microcarriers in a composition, preparation, cell culture, package, container, or vial. This may be the preferred dimension for spherical microcarriers.

For elongate microcarriers the dimension may be the longest dimension of the microcarrier (e.g. the length of the rod or cylinder) or its shortest dimension (e.g. the diameter of the rod or cylinder).

The particle may be porous or non-porous. Porous particles enable medium to circulate within and through the growing area and this may assist cell growth.

### Particle Density

Unless stated otherwise, reference to density, e.g. of microcarriers or polymer materials, is the relative density measured at 25°C stp.

### Culture of Stem Cells

Any suitable method of culturing stem cells, for example as set out in the Examples, may be used in the methods and compositions described here.

Any suitable container may be used to propagate stem cells according to the methods and compositions described here. Suitable containers include those described in US Patent Publication US2007/0264713 (Terstegge).

Containers may include bioreactors and spinners, for example. A "bioreactor", as the term is used in this document, is a container suitable for the cultivation of eukaryotic cells, for example animal cells or mammalian cells, such as in a large scale. A typical cultivation volume of a regulated bioreactor is between 20 ml and 500 ml.

The bioreactor may comprise a regulated bioreactor, in which one or more conditions may be controlled or monitored, for example, oxygen partial pressure. Devices for measuring and regulating these conditions are known in the art. For example, oxygen electrodes may be used for oxygen partial pressure. The oxygen partial pressure can be regulated via the amount and the composition of the selected gas mixture (e.g., air or a mixture of air and/or oxygen and/or nitrogen and/or carbon dioxide). Suitable devices for measuring and regulating the oxygen partial pressure are described by Bailey, J E. (Bailey, J E., Biochemical Engineering Fundamentals, second edition, McGraw-Hill, Inc. ISBN 0-07-003212-2 Higher Education, (1986)) or Jackson A T. Jackson A T., Verfahrenstechnik in der Biotechnologie, Springer, ISBN 3540561900 (1993)).

Other suitable containers include spinners. Spinners are regulated or unregulated bioreactors, which can be agitated using various agitator mechanisms, such as glass ball agitators, impeller agitators, and other suitable agitators. The cultivation volume of a spinner is typically between 20 ml and 500 ml. Roller bottles are round cell culture flasks made of plastic or glass having a culture area of between 400 and 2000 cm². The cells are cultivated along the entire inner surface of these flasks; the cells are coated with culture medium accomplished by a "rolling" motion, i.e. rotating the bottles about their own individual axis.

Alternatively, culture may be static, i.e. where active agitation of the culture/culture media is not employed. By reducing agitation of the culture aggregates of cells/microcarriers may be allowed to form. Whilst some agitation may be employed to encourage distribution and flow of the culture media over the cultured cells this may be applied so as not to substantially disrupt aggregate formation. For example, a low rpm agitation, e.g. less than 30 rpm or less than 20 rpm, may be employed.

### Propagation with Passage

The methods and compositions described here may comprise passaging, or splitting during culture. The methods may involve continuous or continual passage.

By "continual" or "continuous", we mean that our methods enable growth of stem cells on microcarriers in a fashion that enables them to be passaged, e.g., taken off the microcarriers on which they are growing and transferred to other microcarriers or particles, and that this process may be repeated at least once, for example twice, three times, four times, five times, etc. (as set out below). In some cases, this may be repeated any number of times, for example indefinitely or infinitely. Most preferably the process is repeated 5 or more times, e.g. 6 or more time, 7 or more times, 8 or more times, 9 or more times, 10 or more times, 11 or more times, 12 or more times, 13 or more times, 14 or more times, 15 or more times, 16 or more times, 17 or more times, 18 or more times, 19 or more times, 20 or more times, 21 or more times, 22 or more times, 23 or more times, 24 or more times, 25 or more times. The terms "continual" or "continuous" may also be used to mean a substantially uninterrupted extension of an event, such as cell growth. For example, our methods enable the expansion of stem cells to any number of desired generations, without needing to terminate the growth or culture.

Cells in culture may be dissociated from the substrate or flask, and "split", subcultured or passaged, by dilution into tissue culture medium and replating.

Cells growing on particles may be passaged back onto particle culture. Alternatively, they may be passaged back onto conventional (2D) cultures. Tissue culture cells growing on plates may be passaged onto particle culture. Each of these methods are described in more detail below.

The term "passage" may generally refer to the process of taking an aliquot of a cell culture, dissociating the cells completely or partially, diluting and inoculating into medium. The passaging may be repeated one or more times. The aliquot may comprise the whole or a portion of the cell culture. The cells of the aliquot may be completely, partially or not confluent. The passaging may comprise at least some of the following sequence of steps: aspiration, rinsing, trypsinization, incubation, dislodging, quenching, re-seeding and aliquoting. The protocol published by the Hedrick Lab, UC San Diego may be used (http://hedricklab.ucsd.edu/Protocol/COSCell.html).

The cells may be dissociated by any suitable means, such as mechanical or enzymatic means known in the art. The cells may be broken up by mechanical dissociation, for example using a cell scraper or pipette. The cells may be dissociated by sieving through a suitable sieve size, such as through 100 micron or 500 micron sieves. The cells may be split by enzymatic dissociation, for example by treatment with collagenase or trypLE harvested. The dissociation may be complete or partial.

The dilution may be of any suitable dilution. The cells in the cell culture may be split at any suitable ratio. For example, the cells may be split at a ratio of 1:2 or more, 1:3 or more, 1:4 or more or 1:5 or more. The cells may be split at a ratio of 1:6 or more, 1:7 or more, 1:8 or more, 1:9 or more or 1:10 or more. The split ratio may be 1:10 or more. It may be 1:11, 1:12, 1:13, 1:14, 1:15, 1:16, 1:17, 1:18, 1:19 or 1:20 or more. The split ratio may be 1:21, 1:22, 1:23, 1:24, 1:25 or 1:26 or more.

Thus, stem cells may be passaged for 1 passage or more. For example, stem cells may be passaged for 2, 3, 4, 5, 6, 7, 8, 9, 10, 11, 12, 13, 14, 15, 16, 17, 18, 19, 20, 21, 22, 23, 24, 25 passages or more. The stem cells may be passaged for 25, 30, 35, 40, 45, 50, 55, 60, 65, 70, 75, 80, 85, 90, 95 or more passages. The stem cells may be propagated indefinitely in culture.

Passages may be expressed as generations of cell growth. Our methods and compositions allow stem cells to propagate for 1, 2, 3, 4, 5, 6, 7, 8, 9, 10, 11, 12, 13, 14, 15, 16, 17, 18, 19, 20, 21, 22, 23, 24, 25 generations or more. The stem cells may be grown for 25, 30, 35, 40, 45, 50, 55, 60, 65, 70, 75, 80, 85, 90, 95 or more generations.

Passages may also be expressed as the number of cell doublings. Our methods and compositions allow stem cells to propagate for 1, 2, 3, 4, 5, 6, 7, 8, 9, 10, 11, 12, 13, 14, 15, 16, 17, 18, 19, 20, 21, 22, 23, 24, 25 cell doublings or more. The stem cells may be grown for 25, 30, 35, 40, 45, 50, 55, 60, 65, 70, 75, 80, 85, 90, 95 or more cell doublings.

The stem cells may be cultured for more than 5, more than 10, more than 15, more than 20, more than 25, more than 30, more than 40, more than 45, more than 50, more than 100, more than 200, more than 500 or more than 800 passages, generations or cell doublings. The stem cells may be maintained for 100, 200, 500 or more passages, generations or cell doublings.

### Maintenance of Stem Cell Characteristics

The propagated stem cells may retain at least one characteristic of a mammalian, primate or human stem cell. The stem cells may retain the characteristic after one or more passages. They may do so after a plurality of passages. They may do so after the stated number of passages as described above.

The characteristic may comprise a morphological characteristic, immunohistochemical characteristic, a molecular biological characteristic, etc. The characteristic may comprise a biological activity.

### Stem Cell Characteristics

The stem cells propagated by our methods may display any of the following stem cell characteristics.

Stem cells may display increased expression of Oct4 and/or SSEA-1 and/or TRA-1-60 and/or Mab84. Stem cells which are self-renewing may display a shortened cell cycle compared to stem cells which are not self-renewing.

Stem cells may display defined morphology. For example, in the two dimensions of a standard microscopic image, human embryonic stem cells display high nuclear/cytoplasmic ratios in the plane of the image, prominent nucleoli, and compact colony formation with poorly discernable cell junctions.

Stem cells may also be characterized by expressed cell markers as described in further detail below.

### Expression of Pluripotency Markers

The biological activity that is retained may comprise expression of one or more pluripotency markers.

Stage-specific embryonic antigens (SSEA) are characteristic of certain embryonic cell types. Antibodies for SSEA markers are available from the Developmental Studies Hybridoma Bank (Bethesda Md.). Other useful markers are detectable using antibodies designated Tra-1-60 and Tra-1-81 (Andrews et al., Cell Lines from Human Germ Cell Tumors, in E. J. Robertson, 1987, supra). Human embryonic stem cells are typically SSEA-1 negative and SSEA-4 positive. hEG cells are typically SSEA-1 positive. Differentiation of primate pluripotent stem cells (pPS) cells *in vitro* results in the loss of SSEA-4, Tra-1-60, and Tra-1-81 expression and increased expression of SSEA-1. pPS cells can also be characterized by the presence of alkaline phosphatase activity, which can be detected by fixing the cells with 4% paraformaldehyde, and then developing with Vector Red as a substrate, as described by the manufacturer (Vector Laboratories, Burlingame Calif.).

Embryonic stem cells are also typically telomerase positive and OCT-4 positive. Telomerase activity can be determined using TRAP activity assay (Kim et al., Science 266:2011, 1997), using a commercially available kit (TRAPeze.RTM. XK Telomerase Detection Kit, Cat. s7707; Intergen Co., Purchase N.Y.; or TeloTAGGG.TM. Telomerase PCR ELISA plus, Cat. 2,013,89; Roche Diagnostics, Indianapolis). hTERT expression can also be evaluated at the mRNA level by RT-PCR. The LightCycler TeloTAGGG.TM. hTERT quantification kit (Cat. 3,012,344; Roche Diagnostics) is available commercially for research purposes.

Any one or more of these pluripotency markers, including FOXD3, PODXL, alkaline phosphatase, OCT-4, SSEA-4, TRA-1-60 and Mab84, etc., may be retained by the propagated stem cells.

Detection of markers may be achieved through any means known in the art, for example immunologically. Histochemical staining, flow cytometry (FACS), Western Blot, enzyme-linked immunoassay (ELISA), etc. may be used.

Flow immunocytochemistry may be used to detect cell-surface markers. Immunohistochemistry (for example, of fixed cells or tissue sections) may be used for intracellular or cell-surface markers. Western blot analysis may be conducted on cellular extracts. Enzyme-linked immunoassay may be used for cellular extracts or products secreted into the medium.

For this purpose, antibodies to the pluripotency markers as available from commercial sources may be used.

Antibodies for the identification of stem cell markers including the Stage-Specific Embryonic Antigens 1 and 4 (SSEA-1 and SSEA-4) and Tumor Rejection Antigen 1-60 and 1-81 (TRA-1-60, TRA-1-81) may be obtained commercially, for example from Chemicon International, Inc (Temecula, CA, USA). The immunological detection of these antigens using monoclonal antibodies has been widely used to characterize pluripotent stem cells (Shamblott M.J. et al. (1998) PNAS 95: 13726-13731; Schuldiner M. et. al. (2000). PNAS 97: 11307 - 11312; Thomson J.A. et al. (1998). Science 282: 1145-1147; Reubinoff B.E. et al. (2000). Nature Biotechnology 18: 399-404; Henderson J.K. et al. (2002). Stem Cells 20: 329-337; Pera M. et al. (2000). J. Cell Science 113: 5-10.).

The expression of tissue-specific gene products can also be detected at the mRNA level by Northern blot analysis, dot-blot hybridization analysis, or by reverse transcriptase initiated polymerase chain reaction (RT-PCR) using sequence-specific primers in standard amplification methods. Sequence data for the particular markers listed in this disclosure can be obtained from public databases such as GenBank (URL www.ncbi.nlm.nih.gov:80/entrez). See U.S. Pat. No. 5,843,780 for further details.

Substantially all of the propagated cells, or a substantial portion of them, may express the marker(s). For example, the percentage of cells that express the marker or markers may be 50% or more, 60% or more, 70% or more, 80% or more, 90% or more, 93% or more, 95% or more, 97% or more, 98% or more, 99% or more, or substantially 100%.

### Cell Viability

The biological activity may comprise cell viability after the stated number of passages. Cell viability may be assayed in various ways, for example by Trypan Blue exclusion.

A protocol for vital staining follows. Place a suitable volume of a cell suspension (20-200 µL) in an appropriate tube, add an equal volume of 0.4% Trypan blue and gently mix, let stand for 5 minutes at room temperature. Place 10 µl of stained cells in a hemocytometer and count the number of viable (unstained) and dead (stained) cells. Calculate the average number of unstained cells in each quadrant, and multiply by 2 x 10⁴ to find cells/ml. The percentage of viable cells is the number of viable cells divided by the number of dead and viable cells.

The viability of cells may be 50% or more, 60% or more, 70% or more, 80% or more, 90% or more, 93% or more, 95% or more, 97% or more, 98% or more, 99% or more, or substantially 100%.

### Karyotype

The propagated stem cells may retain a normal karyotype during or after propagation. A "normal" karyotype is a karyotype that is identical, similar or substantially similar to a karyotype of a parent stem cell from which the stem cell is derived, or one which varies from it but not in any substantial manner. For example, there should not be any gross anomalies such as translocations, loss of chromosomes, deletions, etc.

Karyotype may be assessed by a number of methods, for example visually under optical microscopy. Karyotypes may be prepared and analyzed as described in McWhir et al. (2006), Hewitt et al. (2007), and Gallimore and Richardson (1973). Cells may also be karyotyped using a standard G-banding technique (available at many clinical diagnostics labs that provide routine karyotyping services, such as the Cytogenetics Lab at Oakland Calif.) and compared to published stem cell karyotypes.

All or a substantial portion of propagated cells may retain a normal karyotype. This proportion may be 50% or more, 60% or more, 70% or more, 80% or more, 90% or more, 93% or more, 95% or more, 97% or more, 98% or more, 99% or more, or substantially 100%.

### Pluripotency

The propagated stem cells may retain the capacity to differentiate into all three cellular lineages, i.e., endoderm, ectoderm and mesoderm. Methods of induction of stem cells to differentiate into each of these lineages are known in the art and may be used to assay the capability of the propagated stem cells.

All or a substantial portion of propagated cells may retain this ability. This may be 50% or more, 60% or more, 70% or more, 80% or more, 90% or more, 93% or more, 95% or more, 97% or more, 98% or more, 99% or more, or substantially 100% of the propagated stem cells.

### Co-Culture and Feeders

Methods may comprise culturing stem cells in the presence or absence of co-culture. The term "co-culture" refers to a mixture of two or more different kinds of cells that are grown together, for example, stromal feeder cells. The two or more different kinds of cells may be grown on the same surfaces, such as particles or cell container surfaces, or on different surfaces. The different kinds of cells may be grown on different particles.

Feeder cells, as the term is used in this document, may mean cells which are used for or required for cultivation of cells of a different type. In the context of stem cell culture, feeder cells have the function of securing the survival, proliferation, and maintenance of ES-cell pluripotency. ES-cell pluripotency may be achieved by directly co-cultivating the feeder cells. Alternatively, or in addition, the feeder cells may be cultured in a medium to condition it. The conditioned medium may be used to culture the stem cells.

The inner surface of the container such as a culture dish may be coated with a feeder layer of mouse embryonic skin cells that have been treated so they will not divide. The feeder cells release nutrients into the culture medium which are required for ES cell growth. The stem cells growing on particles may therefore be grown in such coated containers.

The feeder cells may themselves be grown on particles. They may be seeded on particles in a similar way as described for stem cells. The stem cells to be propagated may be grown together with or separate from such feeder particles. The stem cells may therefore be grown on a layer on such feeder cell coated particles. On the other hand, the stem cells may be grown on separate particles. Any combinations of any of these arrangements are also possible, for example, a culture which comprises feeder cells grown on particles, particles with feeder cells and stem cells, and particles with stem cells growing. These combinations may be grown in containers with a feeder layer or without.

The particles on which the feeder cells are grown may be either coated or not coated in a matrix coating.

Arrangements in which feeder cells are absent or not required are also possible. For example, the cells may be grown in medium conditioned by feeder cells or stem cells (Conditioned Media).

### Media and Feeder Cells

Media for isolating and propagating pluripotent stem cells can have any of several different formulas, as long as the cells obtained have the desired characteristics, and can be propagated further.

Suitable sources are as follows: Dulbecco's modified Eagles medium (DMEM), Gibco#11965-092; Knockout Dulbecco's modified Eagles medium (KO DMEM), Gibco#10829-018; 200 mM L-glutamine, Gibco#15039-027; non-essential amino acid solution, Gibco 11140-050; beta-mercaptoethanol, Sigma#M7522; human recombinant basic fibroblast growth factor (bFGF), Gibco#13256-029. Exemplary serum-containing embryonic stem (ES) medium is made with 80% DMEM (typically KO DMEM), 20% defined fetal bovine serum (FBS) not heat inactivated, 0.1 mM non-essential amino acids, 1 mM L-glutamine, and 0.1 mM beta-mercaptoethanol. The medium is filtered and stored at 4 degrees C for no longer than 2 weeks. Serum-free embryonic stem (ES) medium is made with 80% KO DMEM, 20% serum replacement, 0.1 mM non-essential amino acids, 1 mM L-glutamine, and 0.1 mM beta-mercaptoethanol. An effective serum replacement is Gibco#10828-028. The medium is filtered and stored at 4 degrees C for no longer than 2 weeks. Just before use, human bFGF is added to a final concentration of 4 ng/mL (Bodnar et al., Geron Corp, International Patent Publication WO 99/20741).

The media may comprise Knockout DMEM media (Invitrogen-Gibco, Grand Island, New York), supplemented with 10% serum replacement media (Invitrogen- Gibco, Grand Island, New York), 5ng/ml FGF2 (Invitrogen-Gibco, Grand Island, New York) and 5ng/ml PDGF AB (Peprotech, Rocky Hill, New Jersey).

Feeder cells (where used) may be propagated in mEF medium, containing 90% DMEM (Gibco#11965-092), 10% FBS (Hyclone#30071-03), and 2 mM glutamine. mEFs are propagated in T150 flasks (Coming#430825), splitting the cells 1:2 every other day with trypsin, keeping the cells subconfluent. To prepare the feeder cell layer, cells are irradiated at a dose to inhibit proliferation but permit synthesis of important factors that support human embryonic stem cells (about 4000 rads gamma irradiation). Six-well culture plates (such as Falcon#304) are coated by incubation at 37 degrees C. with 1 mL 0.5% gelatin per well overnight, and plated with 375,000 irradiated mEFs per well. Feeder cell layers are typically used 5 h to 4 days after plating. The medium is replaced with fresh human embryonic stem (hES) medium just before seeding pPS cells.

Conditions for culturing other stem cells are known, and can be optimized appropriately according to the cell type. Media and culture techniques for particular cell types referred to in the previous section are provided in the references cited.

### Serum Free Media

The methods and compositions described here may include culture of stem cells in a serum-free medium.

The term "serum-free media" may comprise cell culture media which is free of serum proteins, e.g., fetal calf serum. Serum-free media are known in the art, and are described for example in US Patents 5,631,159 and 5,661,034, US5908782, US7109032, US2005/0265980, WO2012/031263 and in Chase et al. (Development and Characterization of a Clinically Compliant Xeno-Free Culture Medium in Good Manufacturing Practice for Human Multipotent Mesenchymal Stem Cells (2012) Stem Cells Trans Med 2012; 1:750-758), Santos et al. (Toward a Clinical-Grade Expansion of Mesenchymal Stem Cells from Human Sources: A Microcarrier-Based Culture System Under Xeno-Free Conditions (2011) Santos et al., Tissue Eng Part C Methods. 17(12):1201-10). Serum-free media are commercially available from, for example, Gibco-BRL (Invitrogen), StemPro® MSC SFM (Life Technologies), MesenCult™ SF (StemCell Technologies).

The serum-free media may be protein free, in that it may lack proteins, hydrolysates, and components of unknown composition. The serum-free media may comprise chemically-defined media in which all components have a known chemical structure. Chemically-defined serum-free media is advantageous as it provides a completely defined system which eliminates variability, allows for improved reproducibility and more consistent performance, and decreases possibility of contamination by adventitious agents. It may also be free of animal derived components.

The serum-free media may comprise Knockout DMEM media (Invitrogen-Gibco, Grand Island, New York).

The serum-free media may be supplemented with one or more components, such as serum replacement media, at a concentration of for example, 5%, 10%, 15%, etc. The serum-free media may be supplemented with 10% serum replacement media from Invitrogen-Gibco (Grand Island, New York).

The serum-free medium in which the dissociated or disaggregated embryonic stem cells are cultured may comprise one or more growth factors. A number of growth factors are known in the art, including FGF2, IGF-2, Noggin, Activin A, TGF beta 1, HRG1 beta, LIF, S1P, PDGF, BAFF, April, SCF, Flt-3 ligand, Wnt3A and others. The growth factor(s) may be used at any suitable concentration such as between 1pg/ml to 500ng/ml.

### Media Supplements

Culture media may be supplemented with one or more additives. For example, these may be selected from one or more of: a lipid mixture, Bovine Serum Albumin (e.g. 0.1% BSA), hydrolysate of soybean protein.

### Stem Cells

As used in this document, the term "stem cell" refers to a cell that on division faces two developmental options: the daughter cells can be identical to the original cell (self-renewal) or they may be the progenitors of more specialised cell types (differentiation). The stem cell is therefore capable of adopting one or other pathway (a further pathway exists in which one of each cell type can be formed). Stem cells are therefore cells which are not terminally differentiated and are able to produce cells of other types.

In general, reference herein to cells (plural) may include the singular (stem cell), and *vice versa.* In particular, methods of culturing and differentiating stem cells may include single cell and aggregate culturing techniques. In the present disclosure stem cell cultures may be of aggregates or single cells.

Stem cells can be described in terms of the range of cell types into which they are able to differentiate, as discussed below.

"Totipotent" stem cells refers to a cell which has the potential to become any cell type in the adult body, or any cell of the extraembryonic membranes (e.g., placenta). Thus, normally, the only totipotent cells are the fertilized egg and the first 4 or so cells produced by its cleavage.

"Pluripotent" stem cells are true stem cells, with the potential to make any differentiated cell in the body. However, they cannot contribute to make the extraembryonic membranes which are derived from the trophoblast. Embryonic Stem (ES) cells are examples of pluripotent stem cells, and may be isolated from the inner cell mass (ICM) of the blastocyst, which is the stage of embryonic development when implantation occurs.

"Multipotent" stem cells are true stem cells which can only differentiate into a limited number of cell types. For example, the bone marrow contains multipotent stem cells that give rise to all the cells of the blood but not to other types of cells. Multipotent stem cells are found in adult animals, and are sometimes called adult stem cells. It is thought that every organ in the body (brain, liver) contains them where they can replace dead or damaged cells.

Methods of characterising stem cells are known in the art, and include the use of standard assay methods such as clonal assay, flow cytometry, long-term culture and molecular biological techniques e.g. PCR, RT-PCR and Southern blotting.

In addition to morphological differences, human and murine pluripotent stem cells differ in their expression of a number of cell surface antigens (stem cell markers). Markers for stem cells and methods of their detection are described elsewhere in this document (under "Maintenance of Stem Cell Characteristics").

### Sources of Stem Cells

U.S. Pat. No. 5,851,832 reports multipotent neural stem cells obtained from brain tissue.
U.S. Pat. No. 5,766,948 reports producing neuroblasts from newborn cerebral hemispheres.
U.S. Pat. Nos. 5,654,183 and 5,849,553 report the use of mammalian neural crest stem cells.

U.S. Pat. No. 6,040,180 reports *in vitro* generation of differentiated neurons from cultures of mammalian multipotential CNS stem cells. WO 98/50526 and WO 99/01159 report generation and isolation of neuroepithelial stem cells, oligodendrocyte-astrocyte precursors, and lineage-restricted neuronal precursors.

U.S. Pat. No. 5,968,829 reports neural stem cells obtained from embryonic forebrain and cultured with a medium comprising glucose, transferrin, insulin, selenium, progesterone, and several other growth factors.

Primary liver cell cultures can be obtained from human biopsy or surgically excised tissue by perfusion with an appropriate combination of collagenase and hyaluronidase. Alternatively, EP 0 953 633 A1 reports isolating liver cells by preparing minced human liver tissue, resuspending concentrated tissue cells in a growth medium and expanding the cells in culture. The growth medium comprises glucose, insulin, transferrin, T3, FCS, and various tissue extracts that allow the hepatocytes to grow without malignant transformation.

The cells in the liver are thought to contain specialized cells including liver parenchymal cells, Kupffer cells, sinusoidal endothelium, and bile duct epithelium, and also precursor cells (referred to as "hepatoblasts" or "oval cells") that have the capacity to differentiate into both mature hepatocytes or biliary epithelial cells (L. E. Rogler, Am. J. Pathol. 150:591, 1997; M. Alison, Current Opin. Cell Biol. 10:710, 1998; Lazaro et al., Cancer Res. 58:514, 1998).

U.S. Pat. No. 5,192,553 reports methods for isolating human neonatal or fetal hematopoietic stem or progenitor cells. U.S. Pat. No. 5,716,827 reports human hematopoietic cells that are Thy-1 positive progenitors, and appropriate growth media to regenerate them in vitro. U.S. Pat. No. 5,635,387 reports a method and device for culturing human hematopoietic cells and their precursors. U.S. Pat. No. 6,015,554 describes a method of reconstituting human lymphoid and dendritic cells.

U.S. Pat. No. 5,486,359 reports homogeneous populations of human mesenchymal stem cells that can differentiate into cells of more than one connective tissue type, such as bone, cartilage, tendon, ligament, and dermis. They are obtained from bone marrow or periosteum. Also reported are culture conditions used to expand mesenchymal stem cells. WO 99/01145 reports human mesenchymal stem cells isolated from peripheral blood of individuals treated with growth factors such as G-CSF or GM-CSF. WO 00/53795 reports adipose-derived stem cells and lattices, substantially free of adipocytes and red cells. These cells reportedly can be expanded and cultured to produce hormones and conditioned culture media.

Stem cells of any vertebrate species can be used. Included are stem cells from humans; as well as non-human primates, domestic animals, livestock, and other non-human mammals such as rodents, mice, rats, etc. Stem cells may be non-human, e.g. rabbit, guinea pig, rat, mouse or other rodent (including cells from any animal in the order Rodentia), cat, dog, pig, sheep, goat, cattle, horse, non-human primate or other non-human vertebrate organism; and/or non-human mammalian cells; and/or human cells.

Amongst the stem cells suitable for use in the methods and compositions described here are primate or human pluripotent stem cells derived from tissue formed after gestation, such as a blastocyst, or fetal or embryonic tissue taken any time during gestation. Non-limiting examples are primary cultures or established lines of embryonic stem cells.

### Mesenchymal stem cells

In some preferred embodiments the stem cells are mesenchymal stem cells (MSCs), e.g. capable of differentiation into connective tissue and/or bone cells such as chondrocytes, osteoblasts, myocytes and adipocytes.

Mesenchymal stem cells are multipotent progenitor cells having the ability to generate cartilage, bone, muscle, tendon, ligament, fat and other connective tissues. They are capable of differentiation into a wide variety of cell types, including bone cells (osteoblasts), cartilage cells (chondrocytes), muscle cells (myocytes) and fat cells (adipocytes) (e.g. see Rastegar et al. World Journal of Stem cells 2010 August 26; 2(4): 67-80).

These primitive progenitors exist postnatally and exhibit stem cell characteristics, namely low incidence and extensive renewal potential. These properties in combination with their developmental plasticity have generated tremendous interest in the potential use of mesenchymal stem cells to replace damaged tissues.
Mesenchymal stem cells can be isolated from a range of tissue types, including bone marrow, muscle, fat, dental pulp, adult tissue, fetal tissue, neonatal tissue, and umbilical cord. Mesenchymal stem cells may be obtained from non-human mammals, or from humans.

Mesenchymal stem cells are easily obtainable from bone marrow by minimally invasive techniques and can be expanded in culture and permitted to differentiate into the desired lineage. Differentiation can be induced by the application of specific growth factors. The transforming growth factor beta (TGF-beta) superfamily member proteins such as the bone morphogenetic proteins (BMPs) are important factors of chondrogenic and osteogenic differentiation of mesenchymal stem cells.

Human bone marrow mesenchymal stem cells can be isolated and detected using selective markers, such as STRO-I, from a CD34+ fraction indicating their potential for marrow repopulation. These cell surface markers are only found on the cell surface of mesenchymal stem cells and are an indication of the cells multipotency.

Culture mesenchymal stem cells normally express CD73, CD90, CD105 on their surface as well as CD146, while lacking expression of CD11b, CD14, CD19, CD34, CD45, CD79a.

Suitable mesenchymal stem cells may be obtained or derived from bone marrow mononuclear cells (BMMNCs) collected from aspirates of bone marrow (e.g. Wexler et al. Adult bone marrow is a rich source of human mesenchymal 'stem' cells but umbilical cord and mobilized adult blood are not. HAEMOPOIESIS AND LEUCOCYTES British Journal of Haematology 121(2):368-374, April 2003.) or Wharton's Jelly of the umbilical cord (e.g. Ta et al. Long-term Expansion and Pluripotent Marker Array Analysis of Wharton's Jelly-Derived Mesenchymal Stem Cells. Stem Cells Dev. 2009 July 20 (Epub)).

Mesenchymal stem cells may be obtained by differentiation of pluripotent stem cells, such as human embryonic stem cells or induced pluripotent stem cells, by application of suitable differentiating factors, as is well known in the art.

Mesenchymal stem cells are multipotent progenitor cells with the ability to generate components of cartilage, bone, muscle, tendon, ligament, fat and blood. These primitive progenitors exist postnatally and exhibit stem cell characteristics, namely low incidence and extensive renewal potential. These properties in combination with their developmental plasticity have generated tremendous interest in their potential use to replace damaged tissues. In essence these stem cells could be cultured to expand their numbers then transplanted to the injured site or after seeding in/on scaffolds to generate appropriate tissue constructs.

Thus, an alternative approach for skeletal, muscular, tendon, ligament and blood repair/regeneration is the selection, expansion and modulation of the appropriate progenitor cells such as mesenchymal stem cells (or precursors of chondrocytes, osteoblasts, myoblasts derived therefrom).

In aspects of the present disclosure mesenchymal stem cells attached to microcarriers, including as aggregates of cells or microcarriers, or cells differentiated from mesenchymal stem cells attached to microcarriers, including as aggregates of cells or microcarriers, may be implanted into a subject in order to repair or regenerate tissue in the subject

### Embryonic Stem Cells

Embryonic stem cells may be isolated from blastocysts of members of primate species (Thomson et al., Proc. Natl. Acad. Sci. USA 92:7844, 1995). Human embryonic stem (hES) cells can be prepared from human blastocyst cells using the techniques described by Thomson et al. (U.S. Pat. No. 5,843,780; Science 282:1145, 1998; Curr. Top. Dev. Biol. 38:133 ff., 1998) and Reubinoff et al, Nature Biotech. 18:399,2000.

Briefly, human blastocysts may be obtained from human *in vivo* preimplantation embryos. Alternatively, *in vitro* fertilized (IVF) embryos can be used, or one cell human embryos can be expanded to the blastocyst stage (Bongso et al., Hum Reprod 4: 706, 1989). Human embryos are cultured to the blastocyst stage in G1.2 and G2.2 medium (Gardner et al., Fertil. Steril. 69:84, 1998). Blastocysts that develop are selected for embryonic stem cell isolation. The zona pellucida is removed from blastocysts by brief exposure to pronase (Sigma). The inner cell masses are isolated by immunosurgery, in which blastocysts are exposed to a 1:50 dilution of rabbit anti-human spleen cell antiserum for 30 minutes, then washed for 5 minutes three times in DMEM, and exposed to a 1:5 dilution of Guinea pig complement (Gibco) for 3 minutes (see Solter et al., Proc. Natl. Acad. Sci. USA 72:5099, 1975). After two further washes in DMEM, lysed trophectoderm cells are removed from the intact inner cell mass (ICM) by gentle pipetting, and the ICM plated on mEF feeder layers.

After 9 to 15 days, inner cell mass-derived outgrowths are dissociated into clumps either by exposure to calcium and magnesium-free phosphate-buffered saline (PBS) with 1 mM EDTA, by exposure to dispase or trypsin, or by mechanical dissociation with a micropipette; and then replated on mEF in fresh medium. Dissociated cells are replated on mEF feeder layers in fresh embryonic stem (ES) medium, and observed for colony formation. Colonies demonstrating undifferentiated morphology are individually selected by micropipette, mechanically dissociated into clumps, and replated. Embryonic stem cell-like morphology is characterized as compact colonies with apparently high nucleus to cytoplasm ratio and prominent nucleoli. Resulting embryonic stem cells are then routinely split every 1-2 weeks by brief trypsinization, exposure to Dulbecco's PBS (without calcium or magnesium and with 2 mM EDTA), exposure to type IV collagenase (about.200 U/mL; Gibco) or by selection of individual colonies by micropipette. Clump sizes of about 50 to 100 cells are optimal.

### Embryonic Germ Cells

Human Embryonic Germ (hEG) cells may be prepared from primordial germ cells present in human fetal material taken about 8-11 weeks after the last menstrual period. Suitable preparation methods are described in Shamblott et al., Proc. Natl. Acad. Sci. USA 95:13726, 1998 and U.S. Pat. No. 6,090,622.

### Induced Pluripotent Stem Cells

The methods and compositions described here may be used for the propagation of induced pluripotent stem cells.

Induced pluripotent stem cells, commonly abbreviated as iPS cells or iPSCs, are a type of pluripotent stem cell artificially derived from a non-pluripotent cell, typically an adult somatic cell, for example fibroblasts, lung or B cells, by inserting certain genes.

iPS cells are reviewed and discussed in Takahashi, K. & Yamanaka (Induction of pluripotent stem cells from mouse embryonic and adult fibroblast cultures by defined factors. Cell 2006;126:663-676), Yamanaka S, et al. (Yamanaka S, et al. Induction of Pluripotent Stem Cells from Adult Human Fibroblasts by Defined Factors. doi:10.1016/j.cell.2007.11.019, and Yamanaka S, et al. Generation of germline-competent induced pluripotent stem cells. Nature 2007;448:313-7), Wernig M, et al. (In vitro reprogramming of fibroblasts into a pluripotent ES-cell-like state. Nature 2007;448:318-24), Maherali N, et al. (Directly reprogrammed fibroblasts show global epigenetic remodeling and widespread tissue contribution. Cell Stem Cell 2007;1:55-70) and Thomson JA, Yu J, et al. (Induced Pluripotent Stem Cell Lines Derived from Human Somatic Cells. Science DOI: 10.1126/science.1151526) and Takahashi et al., (Induction of pluripotent stem cells from adult human fibroblasts by defined factors. Cell. (2007) 131(5):861-72.)

iPS cells are typically derived by transfection of certain stem cell-associated genes into non-pluripotent cells, such as adult fibroblasts. Transfection is typically achieved through viral vectors, such as retroviruses. Transfected genes include the master transcriptional regulators Oct-3/4 (Pouf51) and Sox2, although it is suggested that other genes enhance the efficiency of induction. After 3-4 weeks, small numbers of transfected cells begin to become morphologically and biochemically similar to pluripotent stem cells, and are typically isolated through morphological selection, doubling time, or through a reporter gene and antibiotic infection.

### Sources of Pluripotent Cells

Several methods have now been provided for the isolation of pluripotent stem cells that do not lead to the destruction of an embryo, e.g. by transforming (inducing) adult somatic cells or germ cells. These methods include:
1. Reprogramming by nuclear transfer. This technique involves the transfer of a nucleus from a somatic cell into an oocyte or zygote
2. Reprogramming by fusion with embryonic stem cells. This technique involves the fusion of a somatic cell with an embryonic stem cell.
3. Spontaneous re-programming by culture. This technique involves the generation of pluripotent cells from non-pluripotent cells after long term culture. For example, pluripotent embryonic germ (EG) cells have been generated by long-term culture of primordial germ cells (PGC) (Matsui et al., Derivation of pluripotential embryonic stem cells from murine primordial germ cells in culture. Cell 70, 841-847, 1992).
4. Reprogramming by defined factors. For example the generation of iPS cells by the retrovirus-mediated introduction of transcription factors (such as Oct-3/4, Sox2, c-Myc, and KLF4) into mouse embryonic or adult fibroblasts, e.g. as described above. Kaji et al. (Virus-free induction of pluripotency and subsequent excision of reprogramming factors. Nature. 2009; 458(7239): 771-775) also describe the non-viral transfection of a single multiprotein expression vector, which comprises the coding sequences of c-Myc, Klf4, Oct4 and Sox2 linked with 2A peptides, that can reprogram both mouse and human fibroblasts. iPS cells produced with this non-viral vector show robust expression of pluripotency markers, indicating a reprogrammed state confirmed functionally by *in vitro* differentiation assays and formation of adult chimaeric mice. They succeeded in establishing reprogrammed human cell lines from embryonic fibroblasts with robust expression of pluripotency markers.
   Methods 1-4 are described and discussed by Shinya Yamanaka in Strategies and New Developments in the Generation of Patient-Specific Pluripotent Stem Cells (Cell Stem Cell 1, July 2007 a2007 Elsevier Inc.).
5. Derivation of hESC lines from single blastomeres or biopsied blastomeres. See Klimanskaya I, Chung Y, Becker S, Lu SJ, Lanza R. Human embryonic stem cell lines derived from single blastomeres. Nature 2006; 444:512, Lei et al. Xeno-free derivation and culture of human embryonic stem cells: current status, problems and challenges. Cell Research (2007) 17:682-688, Chung Y, Klimanskaya I, Becker S, et al. Embryonic and extraembryonic stem cell lines derived from single mouse blastomeres. Nature. 2006;439:216-219. Klimanskaya I, Chung Y, Becker S, et al. Human embryonic stem cell lines derived from single blastomeres. Nature. 2006;444:481-485. Chung Y, Klimanskaya I, Becker S, et al. Human embryonic stem cell lines generated without embryo destruction. Cell Stem Cell. 2008;2:113-117 and Dusko IIic et al. (Derivation of human embryonic stem cell lines from biopsied blastomeres on human feeders with a minimal exposure to xenomaterials. Stem Cells And Development. 2009; 18(9): 1343-1350).
6. hESC lines obtained from arrested embryos which stopped cleavage and failed to develop to morula and blastocysts in vitro. See Zhang X, Stojkovic P, Przyborski S, et al. Derivation of human embryonic stem cells from developing and arrested embryos. Stem Cells 2006; 24:2669-2676 and Lei et al. Xeno-free derivation and culture of human embryonic stem cells: current status, problems and challenges. Cell Research (2007) 17:682-68.
7. Parthogenesis (or Parthenogenesis). This technique involves chemical or electrical stimulation of an unfertilised egg so as to cause it to develop into a blastomere from which embryonic stem cells may be derived. For example, see Lin et al. Multilineage potential of homozygous stem cells derived from metaphase II oocytes. Stem Cells. 2003;21(2):152-61 who employed the chemical activation of nonfertilized metaphase II oocytes to produce stem cells.
8. Stem cells of fetal origin. These cells lie between embryonic and adult stem cells in terms of potentiality and may be used to derive pluripotent or multipotent cells. Human umbilical-cord-derived fetal mesenchymal stem cells (UC fMSCs) expressing markers of pluripotency (including Nanog, Oct-4, Sox-2, Rex-1, SSEA-3, SSEA-4, Tra-1-60, and Tra-1-81,minimal evidence of senescence as shown by β-galactosidase staining, and the consistent expression of telomerase activity) have been successfully derived by Chris H. Jo et al. (Fetal mesenchymal stem cells derived from human umbilical cord sustain primitive characteristics during extensive expansion. Cell Tissue Res (2008) 334:423-433). Winston Costa Pereira et al (Reproducible methodology for the isolation of mesenchymal stem cells from human umbilical cord and its potential for cardiomyocyte generation J Tissue Eng Regen Med 2008; 2: 394-399) isolated a pure population of mesenchymal stem cells from Wharton's jelly of the human umbilical cord. Mesenchymal stem cells derived from Wharton's jelly are also reviewed in Troyer & Weiss (Concise Review: Wharton's Jelly-Derived Cells Are a primitive Stromal Cell Population. Stem Cells 2008:26:591-599). Kim et al. (Ex vivo characteristics of human amniotic membrane-derived stem cells. Cloning Stem Cells 2007 Winter;9(4):581-94) succeeded in isolating human amniotic membrane-derived mesenchymal cells from human amniotic membranes. Umbilical cord is a tissue that is normally discarded and stem cells derived from this tissue have tended not to attract moral or ethical objection.
9. Chung et al. [(2008) Human Embryonic Stem Cell Lines Generated without Embryo Destruction. Cell Stem Cell. 2(2) 113-117. Epub 2008 Jan 10] describes the generation of human embryonic setm cell lines with the destruction of an embryo.

Induced pluripotent stem cells have the advantage that they can be obtained by a method that does not cause the destruction of an embryo, more particularly by a method that does not cause the destruction of a human or mammalian embryo.

The method described by Chung et al. (item 9 above) also permits obtaining of human embryonic stem cells by a method that does not cause the destruction of a human embryo.

As such, aspects of the present disclosure may be performed or put into practice by using cells that have not been prepared exclusively by a method which necessarily involves the destruction of human or animal embryos from which those cells may be derived. This optional limitation is specifically intended to take account of Decision G0002/06 of 25 November 2008 of the Enlarged Board of Appeal of the European Patent Office.

### Differentiation / Embryoid Bodies

Cultured stem cells, including pluripotent cells, may be differentiated into any suitable cell type by using differentiation techniques known to those of skill in the art.

We describe a process for producing differentiated cells, the method comprising propagating a stem cell by a method as described herein, and then differentiating the stem cell in accordance with known techniques. For example, we provide for methods of differentiating to ectoderm, mesoderm and endoderm, as well as to cardiomyocytes, adipocytes, chondrocytes and osteocytes, etc. We further provide embryoid bodies and differentiated cells obtainable by such methods. Cell lines made from such stem cells and differentiated cells are also provided.

Methods of differentiating stem cells are known in the art and are described in for example Itskovitz-Eldor (J Itskovitz-Eldor, M Schuldiner, D Karsenti, A Eden, O Yanuka, M Amit, H Soreq, N Benvenisty. Differentiation of human embryonic stem cells into embryoid bodies compromising the three embryonic germ layers. Mol Med. 2000 Feb ;6 (2):88-95) and Graichen et al. (Ralph Graichen, Xiuqin Xu, Stefan R Braam, Thavamalar Balakrishnan, Siti Norfiza, Shirly Sieh, Set Yen Soo, Su Chin Tham, Christine Mummery, Alan Colman, Robert Zweigerdt, Bruce P Davidson. Enhanced cardiomyogenesis of human embryonic stem cells by a small molecular inhibitor of p38 MAPK. Differentiation 2007 (Nov 15)), Kroon et al. (Kroon et al. (2008) Pancreatic endoderm derived from human embryonic stem cells generates glucose-responsive insulin-secreting cells in vivo. Nat Biotechnol Apr;26(4):443-52.) and Hay et al. (Hay et al. (2008). Highly efficient differentiation of hESCs to functional hepatic endoderm requires ActivinA and Wnt3a signalling. PNAS Vol. 105. No.34 12310-12306), WO 2007/030870, WO 2007/070964, Niebrugge et al (Niebrugge et al (2009). Generation of Human Embryonic Stem Cell-Derived Mesoderm and Cardiac Cells Using Size-Specified Aggregates in an Oxygen-Controlled Bioreactor. Biotechnology and Bioengineering. Vol.102, no.2, February 1, 2009), R Passier et al. (R Passier et al. 2005. Serum free media in cocultures (FBS inhibits cardiomyocytes differentiation). Curr Opin Biotechnol. 2005 Oct;16(5):498-502. Review. Stem Cells. 2005 Jun-Jul;23(6):772-80), P W Burridge et al. (P W Burridge et al. 2006. Defined Medium with polyvinyl alcohol (PVA), Activin A and bFGF. Stem Cells. 2007 Apr;25(4):929-38. Epub 2006 Dec 21), M A Laflamme et al. (M A Laflamme et al. 2007. Culture sequentially supplemented with Activin A for 24 h, and BMP 4 for 4 days. Nat. Biotechnol. 2007 Sep;25(9):1015-24. Epub 2007 Aug 26), L Yang et al. (L Yang et al. 2008. Defined medium supplemented with BMP4 (1 day), BMP4, Activin A and bFGF (days 1-4), Activin A and bFGF (days 4-8), and DKK1 and VEGF. Nature. 2008 May 22;453(7194):524-8. Epub 2008 Apr 23), and X Q Xu et al. (X Q Xu et al. 2008. SB203580 (p38 MAP kinase inhibitor) PGI2 (prostaglandin member accumulated in END2-CM). Differentiation. 2008 Nov;76(9):958-70. Epub 2008 Jun 13).

Stem cells can be induced to differentiate to the neural lineage by culture in media containing appropriate differentiation factors. Such factors may include one or more of activin A, retinoic acid, basic fibroblast growth factor (bFGF), and antagonists of bone morphogenetic protein (BMP), such as noggin (Niknejad et al. European Cells and Materials Vol.19 2010 pages 22-29). Cells differentiating towards the neural lineage may be identified by expression of neural markers, such as Pax6, Nestin, Map2, β-tubulin III and GFAP. Cells of the neural lineage may cluster to form neurospheres (which may be nestin-positive cell aggregates), and these may be expanded by application of selected growth factors such as EGF and/or FGF1 and/or FGF2.

Differentiation may be into a specific cell type selected from one of a gland cell, a hormone secreting cell, a neural cell, a metabolic cell, a blood cell, a germ cell, an immune cell, a contractile cell, or a secretion cell.

The cultured stem cells may also be used for the formation of embryoid bodies. Embryoid bodies, and methods for making them, are known in the art. The term "embryoid body" refers to spheroid colonies seen in culture produced by the growth of embryonic stem cells in suspension. Embryoid bodies are of mixed cell types, and the distribution and timing of the appearance of specific cell types corresponds to that observed within the embryo. Embryoid bodies may be generated by plating out embryonic stem cells onto media such as semi-solid media. Methylcellulose media may be used as described in Lim et al., Blood. 1997;90:1291-1299.

Embryonic stem cells may be induced to form embryoid bodies, for example using the methods described in Itskovitz-Eldor (2000, *supra*). The embryoid bodies contain cells of all three embryonic germ layers.

The embryoid bodies may be further induced to differentiate into different lineages for example by exposure to the appropriate induction factor or an environmental change. Graichen et al. (2007, *supra*) describes the formation of cardiomyocytes from human embryonic stem cells by manipulation of the p38MAP kinase pathway. Graichen demonstrates induction of cardiomyocyte formation from stem cells by exposure to a specific inhibitor of p38 MAP kinase such as SB203580 at less than 10 micromolar.

Differentiated cells may be employed for any suitable purpose, such as regenerative therapy, as known in the art.

Stem cells obtained through culture methods and techniques according to this invention may be used to differentiate into another cell type for use in a method of medical treatment. Thus, the differentiated cell type may be derived from a stem cell obtained by the culture methods and techniques described herein which has subsequently been permitted to differentiate. The differentiated cell type may be considered as a product of a stem cell obtained by the culture methods and techniques described herein which has subsequently been permitted to differentiate. Pharmaceutical compositions may be provided comprising such differentiated cells, optionally together with a pharmaceutically acceptable carrier, adjuvant or diluent. Such pharmaceutical composition may be useful in a method of medical treatment.

### Differentiation on Microcarriers

Stem cells, particularly embryonic stem cells and iPS, may be induced to differentiate during suspension culture on microcarriers.

Embryonic stem cells may be induced to differentiate into the three primary germ layers: ectoderm, endoderm and mesoderm and their derivatives. Embryonic stem cells may be induced to form embryoid bodies. A range of cell types or tissues may therefore be obtained, for example cardiomyocytes, cardiac mesoderm, hepatocytes, hepatic endoderm, pancreatic islet cells, pancreatic endoderm, insulin producing cells, neural tissue, neuroectoderm, epidermal tissue, surface ectoderm, bone, cartilage, muscle, ligament, tendon or other connective tissue.

Methods for the differentiation of stem cells and the formation of embryoid bodies are described above, and are applicable to the differentiation of stem cells in microcarrier culture.

Methods of differentiation of stem cells during microcarrier culture may require the microcarrier to be uncoated or coated in a matrix coating as described above. For example, suitable coatings may include one or more of: Matrigel, Laminin, Fibronectin, Vitronectin, Hyaluronic Acid.

Methods of differentiation of stem cells during microcarrier culture may include the addition of supplements to the culture media. For example, supplements may include Bovine Serum Albumin, Lipids or Hy-Soy (Sigma-Aldrich - this is an enzymatic hydrolysate of soybean protein).

Methods of differentiation of stem cells during microcarrier culture may involve an initial culture and propagation of the stem cells in either 2D culture or in 3D suspension microcarrier culture followed by induction of differentiation during microcarrier culture.

### Biomaterials. Scaffolds and implants

Pharmaceutical compositions and medicaments of the present disclosure may take the form of a biomaterial that is coated and/or impregnated with PCL microcarriers. An implant or prosthesis may be formed from the biomaterial. Such implants or prostheses may be surgically implanted to assist in tissue (e.g. bone or connective tissue) growth, repair, regeneration, restructuring and/or re-modelling.

PCL microcarriers may be applied to implants, prostheses to accelerate new tissue formation at a desired location.

The biomaterial may be coated or impregnated with PCL microcarriers. Impregnation may comprise forming the biomaterial by mixing PCL microcarriers with the constitutive components of the biomaterial, e.g. during polymerisation, or absorbing PCL microcarriers into the biomaterial. Coating may comprise adsorbing the PCL microcarriers onto the surface of the biomaterial.

The biomaterial should allow the coated or impregnated PCL microcarriers to be released from the biomaterial when administered to or implanted in the subject. Biomaterial release kinetics may be altered by altering the structure, e.g. porosity, of the biomaterial.

In addition to coating or impregnating a biomaterial with PCL microcarriers, one or more biologically active molecules may be impregnated or coated on the biomaterial. For example, at least one chosen from the group consisting of: BMP-2, BMP-4, OP-1, FGF-1, FGF-2, TGF-β1, TGF-β2, TGF-β3; VEGF; collagen; laminin; fibronectin; vitronectin. In addition or alternatively to the above bioactive molecules, one or more bisphosphonates may be impregnated or coated onto the biomaterial along with PCL microcarriers. Examples of useful bisphosphonates may include at least one chosen from the group consisting of: etidronate; clodronate; alendronate; pamidronate; risedronate; zoledronate.

Biomaterials coated or impregnated with PCL microcarriers may be useful in both medical and veterinary purposes. It will be appreciated that the products and methods disclosed herein may improve the quality of life of a patient or potentially extend the life of an animal, for example a valuable race horse for use in breeding.

The biomaterial provides a scaffold or matrix support. The biomaterial may be suitable for implantation in tissue, or may be suitable for administration (e.g. as microcapsules in solution).

The implant or prosthesis should be biocompatible, i.e. non-toxic and of low immunogenicity (most preferably non-immunogenic). The biomaterial may be biodegradable such that the biomaterial degrades as wound healing occurs, ultimately leaving only the regenerated bone *in situ* in the subject. Alternatively a non-biodegradable biomaterial may be used, e.g. to guide bone regeneration over a large discontinuity, with surgical removal of the biomaterial being an optional requirement after successful wound healing.

Biomaterials may be soft and/or flexible, e.g. hydrogels, fibrin web or mesh, or collagen sponges. A "hydrogel" is a substance formed when an organic polymer, which can be natural or synthetic, is set or solidified to create a three-dimensional open-lattice structure that entraps molecules of water or other solutions to form a gel. Solidification can occur by aggregation, coagulation, hydrophobic interactions or cross-linking.

Alternatively biomaterials may be relatively rigid structures, e.g. formed from solid materials such as plastics or biologically inert metals such as titanium.

The biomaterial may have a porous matrix structure which may be provided by a crosslinked polymer. The matrix is preferably permeable to nutrients and growth factors required for bone growth.

Matrix structures may be formed by crosslinking fibres, e.g. fibrin or collagen, or of liquid films of sodium alginate, chitosan, or other polysaccharides with suitable crosslinkers, e.g. calcium salts, polyacrylic acid, heparin. Alternatively scaffolds may be formed as a gel, fabricated by collagen or alginates, crosslinked using well established methods known to those skilled in the art.

Suitable polymer materials for matrix formation include biodegradable/bioresorbable polymers which may be chosen from the group of: collagen, fibrin, chitosan, polycaprolactone, poly(DL-lactide-co-caprolactone), poly(L-lactide-co-caprolactone-co-glycolide), polyglycolide, polylactide, polyhydroxyalcanoates, co-polymers thereof, or non-biodegradable polymers which may be chosen from the group of: cellulose acetate; cellulose butyrate, alginate, agarose, polysulfone, polyurethane, polyacrylonitrile, sulfonated polysulfone, polyamide, polyacrylonitrile, polymethylmethacrylate, co-polymers thereof.

Collagen is a promising material for matrix construction owing to its biocompatibility and favourable property of supporting cell attachment and function (U.S. Pat. No. 5,019,087; Tanaka, S.; Takigawa, T.; Ichihara, S. & Nakamura, T. Mechanical properties of the bioabsorbable polyglycolic acid-collagen nerve guide tube Polymer Engineering & Science 2006, 46, 1461-1467). Collagen sponges are well known in the art (e.g. from Integra Life Sciences)

Fibrin scaffolds (described herein) provide an alternative matrix material.

A further example of a biomaterial is a polymer that incorporates hydroxyapatite or hyaluronic acid.

The biomaterial can be supplemented with additional cells. For example, one can "seed" the biomaterial (or co-synthesise it) with undifferentiated bone precursor cells, e.g. stem cells such as mesenchymal stem cells, more preferably human mesenchymal stem cells.

Methods according to the present disclosure may be performed in vitro or in vivo. The term "in vitro" is intended to encompass experiments with materials, biological substances, cells and/or tissues in laboratory conditions or in culture whereas the term "in vivo" is intended to encompass experiments and procedures with intact multi-cellular organisms.

The disclosure includes the combination of the aspects and preferred features described except where such a combination is clearly impermissible or expressly avoided.

The section headings used herein are for organizational purposes only and are not to be construed as limiting the subject matter described.

Aspects and embodiments of the present invention will now be illustrated, by way of example, with reference to the accompanying figures. Further aspects and embodiments will be apparent to those skilled in the art.

### Brief Description of the Figures

**Figure 1****.** Graphs showing (A) mean diameter (µm), and (B) and the coefficient of variation (CV), as a function of the continuous and dispersed phase flow rates, for PCL microspheres as a function of flow rates of the dispersed and continuous phases.
**Figure 2****.** Micrograph showing PCL microcarriers after (upper panel) 30 hours and (lower panel) 7 days of NaOH treatment.
**Figure 3****.** Graphs showing (A) poly-L-Lysine (PLL), and (B) laminin (LN) surface density on PCL microspheres, as a function of coating solution concentration, on untreated and NaOH treated microcarriers.
**Figure 4****.** Graph showing coating of collagen and gelatin on PCL microcarriers.
**Figure 5****.** Table 1, summarising microcarriers and coatings used for hESC.
**Figure 6****.** Bar chart showing cell densities achieved of hESC cultured on different ECM-coated PS or PCL microcarriers after 1 week.
**Figure 7****.** Photographs showing growth of hESC on PCL microcarriers with different coatings, NaOH, PLL, LN and VN. High densities of cells form large clusters of cell-microcarrier aggregates for several coating conditions.
**Figure 8****.** Table 2, summarising microcarriers and coatings used for MSC.
**Figure 9****.** Bar chart showing cell density and expansion fold at Day 4. Numbers above bars indicate cell expansion fold in duplicates. Dotted line indicates initial seeding density at 5 x 10⁴ cells/well.
**Figure 10****.** Micrographs showing growth of MSCs on PCL microcarriers with different coatings (polylysine, PLL, NaOH, gelatin, GL or collagen CL) after 4 days compared to commercial Cytodex 3.
**Figure 11****.** Bar chart showing growth of hfMSC on PCL microcarriers coated with FN-PLL-FN cultured in a 6-well ULA plate. Data are expressed as the mean ± standard deviation (SD) of triplicate results. Student's t-test analysis of variance was applied as comparison between groups, with *indicating significant differences (p ≤0.05) compared to the 6d static and 1d static 4d rocking cultures.
**Figure 12****.** Micrographs showing PCL microcarriers coated with FN-PLL-FN and seeded with stem cells after 6 days in culture. Representative images are shown.
**Figure 13****.** Bar chart showing expression of stem cell surface markers CD90, CD105 and CD146 on hfMSC after 6 days in culture on 6-well ULA plates. After 6 days in culture under static or mixed static/rocking conditions, cells were harvested from microcarriers, stained with antibodies for MSC stem cell surface markers CD90, CD105 and CD146 and analyzed by flow cytometry. From left to right, bars are CD90, CD105 and CD146.
**Figure 14****.** Bar chart showing growth of hfMSC on PCL microcarriers coated with FN-PLL-FN cultured in a T25 ULA flasks. Data are expressed as the mean ± standard deviation (SD) of triplicate results. Student's t-test analysis of variance was applied as comparison between groups, with 2d static and 5d rocking cultures yielding higher cell numbers compared to the 7d static cultures (*indicating p ≤0.05) and 1d static and 6d rocking cultures (**indicating p ≤0.01).
**Figure 15****.** Micrographs showing PCL microcarriers coated with FN-PLL-FN and seeded with stem cells after 7 days in culture in ultra low adhesion T25 flasks.
**Figure 16****.** Bar chart showing expression of stem cell surface markers CD90, CD105 and CD146 on hfMSC after 7 days in culture in T25 ULA flasks. After 7 days in culture under static or mixed static/rocking conditions, cells were harvested from microcarriers, stained with antibodies for MSC stem cell surface markers CD90, CD105 and CD146 and analyzed by flow cytometry. From left to right, bars are CD90, CD105 and CD146.

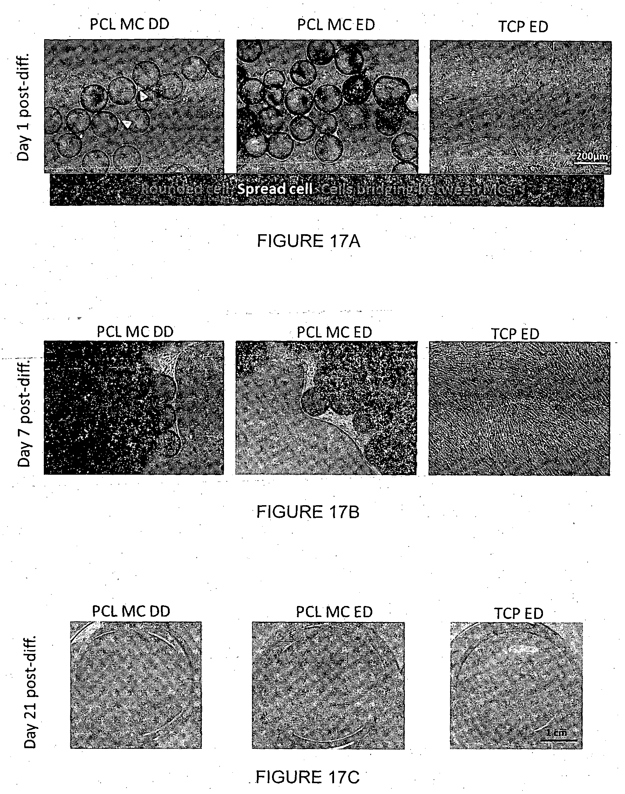

**Figure 17****.** (A) & (B) Micrographs and (C) Photographs showing adhesion and proliferation of hfMSCs on FN-PLL-FN coated PCL microcarriers *in vitro* under static conditions. Adhesion and growth of hfMSCs was assessed for 3 different conditions: (1) PCL microcarrier Direct Differentiation (DD), (2) PCL microcarrier Expansion/Differentiation (ED), and (3) TCP microcarrier Expansion/ Differentiation (ED). Micrographs of hfMSCs after (A) 1 day and (B) 7 days of differentiation by culture in osteogenic induction media. (C) Pictures indicate the size of microcarrier aggregates after 21 days of osteogenic differentiation.
**Figure 18****.** Graph showing total calcium deposited by hfMSCs cultured on either tissue culture plastic or PCL microcarriers, measured at 1, 7, 14, 21 and 28 days post culture in osteogenic induction medium. hfMSC mineralization was tested on 3 different conditions: (1) PCL microcarrier Direct Differentiation, (2) PCL microcarrier Expansion/ Differentiation, and (3) TCP Expansion/ Differentiation, with (3) serving a positive control. Each condition was analyzed in triplicate, bars indicate standard error, *** p<0.001, ****p<0.0001 in Graphpad by one-way ANOVA with post-hoc Tukey.
**Figure 19****.** Upper Panels - Phase microscopy of 21 days of MC-hfMSC-unharvested culture demonstrates confluent cell growth on the microcarriers entrapped in the scaffold and dispersed growth of single cell hfMSC culture. Lower Panels - Graphs showing kinetics of total calcium deposition (mg per scaffold) demonstrates faster deposition kinetics and higher levels of deposition (more than two fold calcium deposition) in the MC-hfMSC-unharvested scaffold culture over the single cell MSC.

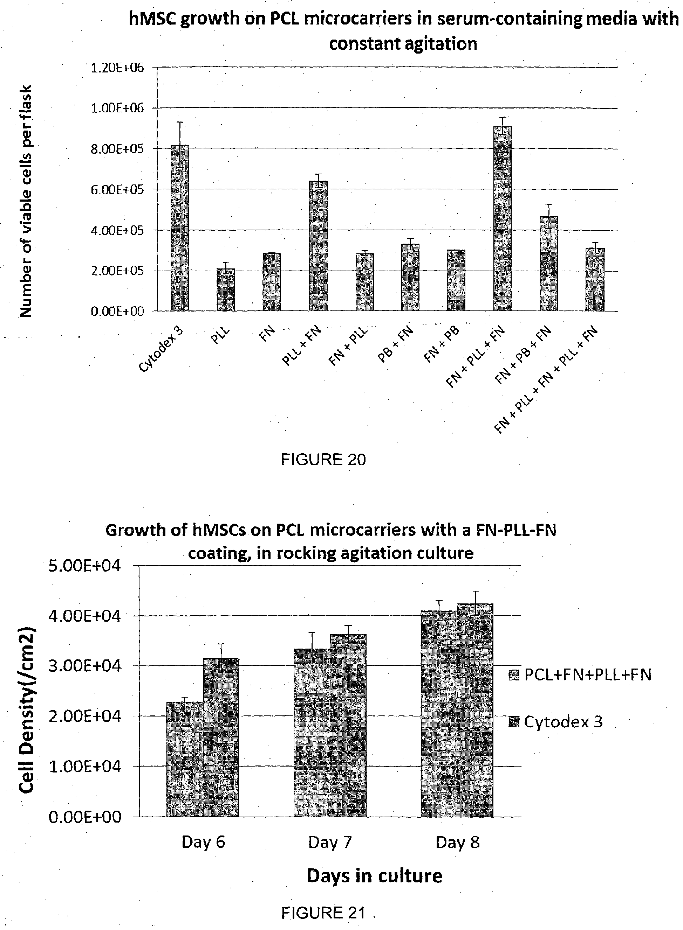

**Figure 20****.** Bar chart showing cell density and expansion of hMSCs on PCL microcarriers in serum-containing media in constant agitation culture. hMSCs were seeded onto PCL microcarriers at 6.5 x 10⁴ cells per 40 mg of PCL microcarriers (or 4 mg of Cytodex 3 microcarriers as a control), and grown in T25 ultra-low adherence flasks with constant agitation on a rocking platform (28 oscillations/min). Dotted line indicates initial seeding density at 6.5 x 10⁴ cells/well. PLL: poly-L-lysine; FN: fibronectin; PB: polybrene. For multiple layers of coatings: FN+PLL+FN refers to a triple layer of fibronectin, poly-L-lysine, and fibronectin.
**Figure 21****.** Bar chart showing growth of hMSCs on PCL microcarriers in serum-containing media in constant agitation culture. hMSCs were seeded onto PCL microcarriers at 6.5 x 10⁴ cells per 40 mg of PCL microcarriers (or 4 mg of Cytodex 3 microcarriers as a control), and grown in T25 ultra-low adherence flasks with constant agitation on a rocking platform (28 oscillations/min). FN+PLL+FN refers to a triple layer of fibronectin, poly-L-lysine, and fibronectin. Left bars = PCL+FN+PLL+FN, right bars = Cytodex 3.
**Figure 22****.** (A) Photograph of MSCs on PCL-FN-PLL-FN microcarriers after 4 days of constant agitation culture in serum-containing media (B) Histograms of flow cytometry data, showing that the harvested cells express cell surface markers characteristic of MSCs. hMSCs were grown on PCL microcarriers coated with a triple layer of fibronectin-poly-L-lysine-fibronectin (PCL-FN-PLL-FN) in constant agitation on a rocking platform (28 oscillations/min).
**Figure 23****.** Bar chart showing hMSC growth on PCL microcarriers in serum-containing media in static culture. hMSCs were seeded onto PCL microcarriers at 6.5 x 104 cells per 40 mg of PCL microcarriers (or 4mg of Cytodex 3 microcarriers as a control), and grown in T25 ultra-low adherence flasks in static culture.
**Figure 24****.** Bar chart showing growth of hMSCs on PCL microcarriers in serum-containing media in constant agitation culture. hMSCs were seeded onto PCL microcarriers at 6.5 x 10⁴ cells per 40 mg of PCL microcarriers (or 4 mg of Cytodex 3 microcarriers as a control), and grown in T25 ultra-low adherence flasks with constant agitation on a rocking platform (28 oscillations/min), in alphaMEM media supplemented with 10% FBS.

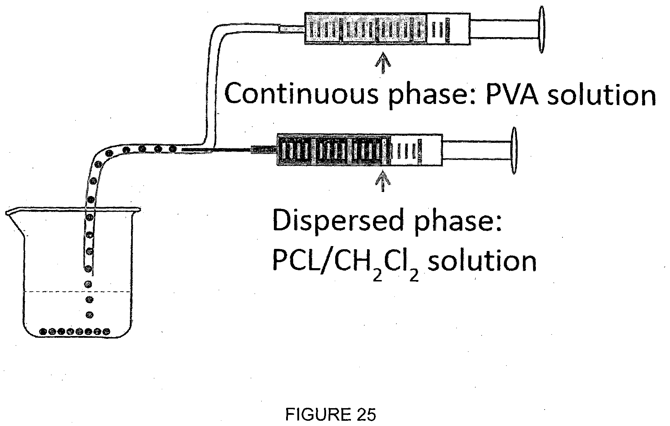

**Figure 25****.** Schematic of the production method of the PCL microcarriers.

### Examples

### Example 1 - Fabrication of polymer microspheres

This example describes the fabrication of spherical microspheres with uniform size from polycaprolactone (PCL).

PCL was sourced from Sigma-Aldrich (Ref. 440744), with MW in the approximate range 70,000-90,000, as measured by gas permeation chromatography (GPC).

### Fabrication of polymer microspheres using a microfluidic device

7.5 g of polyvinyl alcohol (PVA) and 250 ml of di-water were added in a 250 ml glass bottle, then dissolved and shaken at 80°C, yielding 250ml of 3 wt% aqueous polyvinyl alcohol (PVA) solution (which will be referred to as the continuous phase).
100 ml of 1% (w/v) PCL solution in dichloromethane (DCM; CH₂CL₂) was prepared (which will be referred to as the dispersed phase).

Two 20 ml syringes were mounted on respective syringe pumps, with individually adjustable flow rates. One syringe fed the continuous phase into PTFE tubing (I.D.=0.5 mm), and the other syringe fed the dispersed phase into a needle (27G, I.D.=0.191 mm). Because the needle diameter is smaller than PTFE tubing, and the needle is positioned concentric in the axis of the tubing, the dispersed phase flowing through the needle is injected into the continuous phase flowing through the tubing.

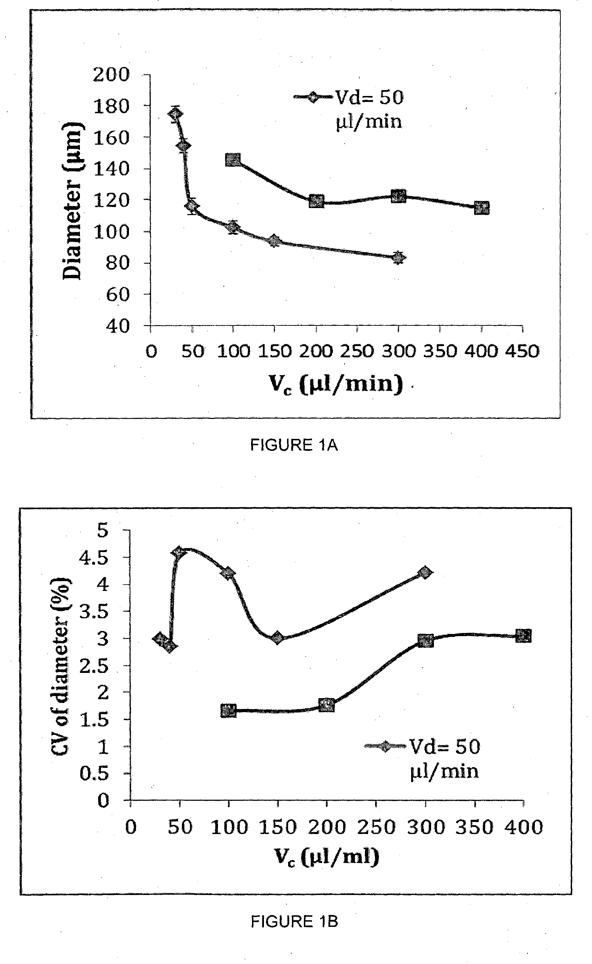

The syringe pump flow rates of the continuous and dispersed phases were adjusted to values suitable for preparing the polymer microspheres. The flow rates used in this example are shown in Figure 1.

Syringe pumps were run to form the PCL/DCM microdroplets, which were collected in a 250 ml beaker, with 50 ml of 3% aqueous PVA solution at room temperature. Approximately 40 ml of PCL/DCM microdroplets can be collected into one beaker.

Polymer/DCM microdroplets were then dried at room temperature by evaporating DCM, thus yielding the polymer microspheres. The PVA solution above the microdroplets was removed, to leave a depth of a few millimetres. DCM diffuses through the aqueous phase and was evaporated under a fume hood over a period of 3-7 days. When the microdroplets appeared to have solidified into microspheres, as evidenced by a lack of fluidity and some adhesion to the surface of the container, the container was placed in a vacuum oven at room temperature overnight to complete the drying process.

Following their dispersal in pure water and five subsequent rinses to remove any PVA residues, the sphere diameters were measured with optical microscopy. Figure 1 shows the mean diameter and coefficient of variation (CV, calculated as the standard deviation divided by the mean diameter) as a function of the continuous and dispersed phase flow rates. The PCL microcarriers fabricated had a diameter of 184 +/- 11 µm, with a density of 1.085 g/cm³.

Following this general method, microcarriers having varying pore sizes and overall density were generated by varying the amount (in grams) of PVA and/or the % (w/v) PCL in DCM solution, i.e. by varying the concentration of the PVA and PCL solutions. After drying, all pores were found to be air-filled, as determined by scanning electron microscopy.

### Example 2 - NaOH treatment of PCL microcarriers

After rinsing five times in pure water, the PCL microspheres were incubated in 5M aqueous sodium hydroxide for 3 hours at room temperature to increase the PCL wettability and generate a negative surface charge. Microspheres were subsequently rinsed five times in pure water and then exposed to 70% aqueous ethanol for 1 hour, for sanitization. Following a further five times rinsing in sterile pure water, the microspheres were rinsed twice in 1x sterile PBS and then left in sterile 1x PBS suspension, for protein coating.

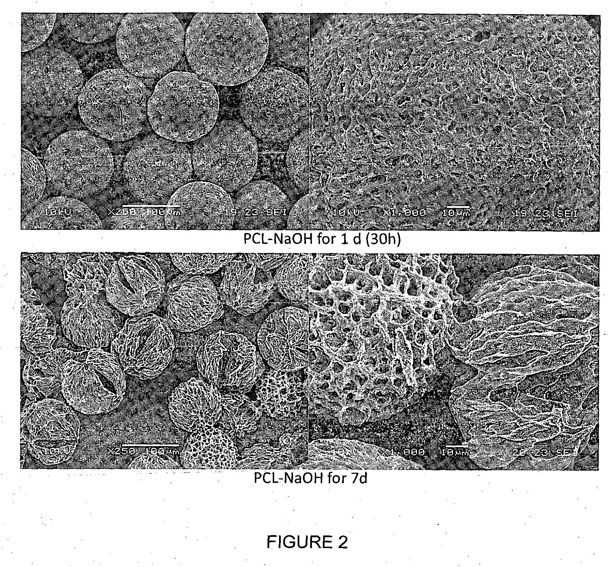

The microspheres can be made porous from prolonged NaOH treatment, lasting typically 1-7 days. Pores may also be generated by a porogen, such as a biodegradeable or water soluble hydrogel phase, which would be blended into the PCL prior to microcarrier formation, potentially with the use of a surface-active agent, such as a surfactant or hydrophilic/hydrophobic diblock copolymer. Figure 2 shows porous microcarriers created by extended NaOH streatment.

### Example 3 - Coating PCL microspheres with poly-l-lysine (PLL) and protein

Poly-L-lysine (PLL), with molecular weight of 70 kDa-150 kDa, was purchased from Sigma-Aldrich Inc. Human plasma vitronectin (VN) >90% purity was purchased from Millipore and natural mouse laminin (LN) >95% purity from the Engelbreth-Holm-Swam sarcoma was obtained from Invitrogen.

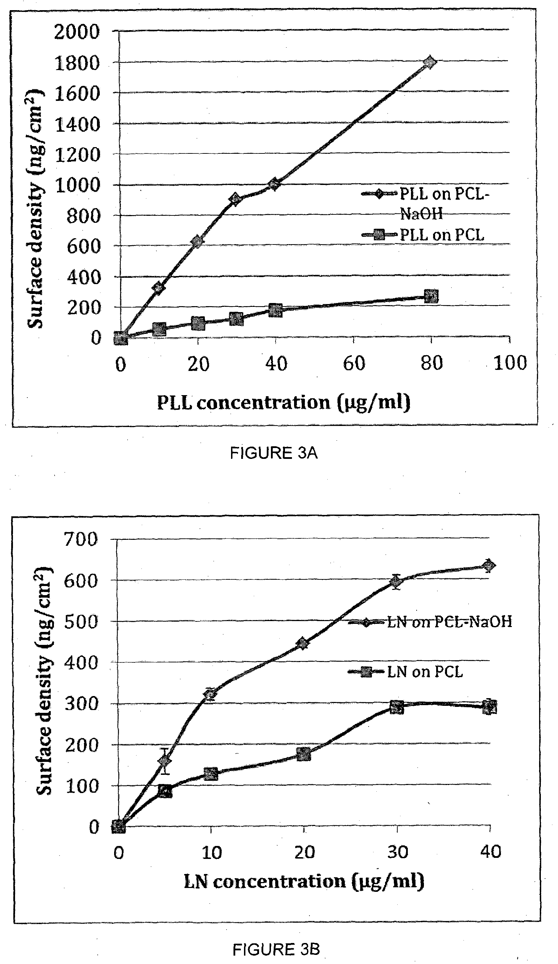

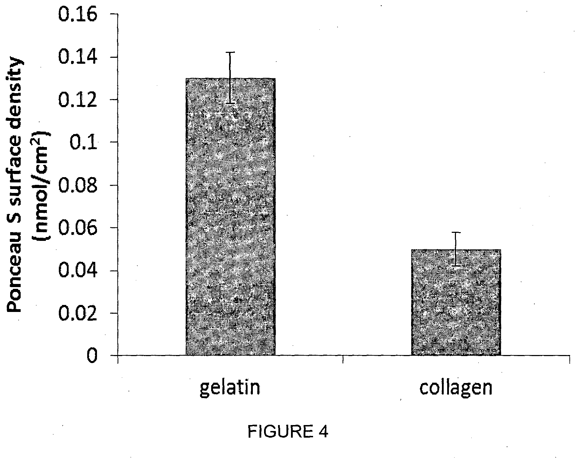

The microspheres were coated with poly-L-Lysine (PLL) or protein, including laminin (LN) and collagen (CLG) by incubating 25 mg of PCL microspheres in 600 µl of aqueous PLL or protein suspension in a well of a 24-well tissue culture polystyrene plate (Becton Dickinson Ref. 353047) for 15 hours at 4° C. The initial PLL and protein solution concentrations used are shown in Figure 3. Concentrations were measured before and after incubation using a modified Bradford Assay, allowing quantification of the PLL or protein adsorbed from solution. This measurement was complemented by measuring staining of adsorbed PLL protein on the container and on the microspheres. While Ponceau S does not provide an absolute measure of adsorbed protein, it allows the ratio of protein adsorbed on the container to that adsorbed on the microspheres to be determined. Having calculated the surface area of 15 cm² for 25 mg of 175 µm mean diameter PCL microspheres, the adsorbed PLL or protein was quantified for each solution concentration and expressed as a surface density. Surface density data for PLL, laminin, gelatin and collagen adsorbed to PCL microspheres are shown in Figures 3 and 4.

### Coating PCT microspheres with gelatin

The microspheres were coated with gelatin (GL) by incubating 40 mg of PCL microspheres in 1 ml of 1 mg/ml gelatin solution in a well of a 24-well tissue culture polystyrene plate for 15 hours at 4° C. After being coated with gelatin, the PCL microspheres in the gelatin solution were transferred into a 1.5 ml Eppendorf tube and the gelatin supernatant was removed. One milliliter of 0.07% glutaraldehyde solution in PBS was added into the tube to cross-link the gelatin coated onto the PCL microspheres. After overnight incubation in the glutaraldehyde solution at room temperature, the gelatin coated PCL were rinsed ten times in pure water to remove any residue.

### Example 4 - Coating PCL microspheres with fibronectin/poly-L-lysine/fibronectin multilayer

PCL microcarriers were coated with a multilayer of fibronectin-poly-L-lysine-fibronectin (FN-PLL-FN) with three overnight (15-18 hours) incubation steps carried out at room temperature on a rocking platform. First, microcarriers in a 1.5 ml Eppendorf tube were incubated in a recombinant fibronectin (FN) solution in PBS, and every one centimeter square of PCL MC was coated with 1 µg of FN. Second, microcarriers were incubated in poly-l-lysine (PLL) in PBS, and every one centimeter square of PCL MC was coated with 2 µg of PLL. Third, microcarriers were incubated in fibronectin (FN) solution in PBS again, at the same concentration as the first FN solution, every one centimeter square of PCL MC was coated with 1 µg of FN. After each overnight incubation step, PCL microcarriers were rinsed twice with PBS.

### Example 5 - Growth of hESCs on PCL microcarriers

Human embryonic stem cell line, HES-3 (ES Cell International) were routinely maintained on Matrigel-coated tissue culture plates with mTeSR1 medium (StemCell Technologies; catalog number 05850). Polycaprolactone (PCL; catalog number 440744) was obtained from Sigma-Aldrich. Polystyrene beads (PS; catalog number 7602B) with an average diameter of 97±10 µm were purchased from Thermo-Fisher Scientific as positive control. Poly-L-lysine (PLL; catalog number P6268), with molecular weight of 70kDa-150kDa, was bought from Sigma-Aldrich Inc. Human plasma vitronectin (VN; catalog number CC080) >90% purity was purchased from Millipore and natural mouse laminin (LN; catalog number 23017-015) >95% purity from the Engelbreth-Holm-Swam sarcoma was obtained from Invitrogen.

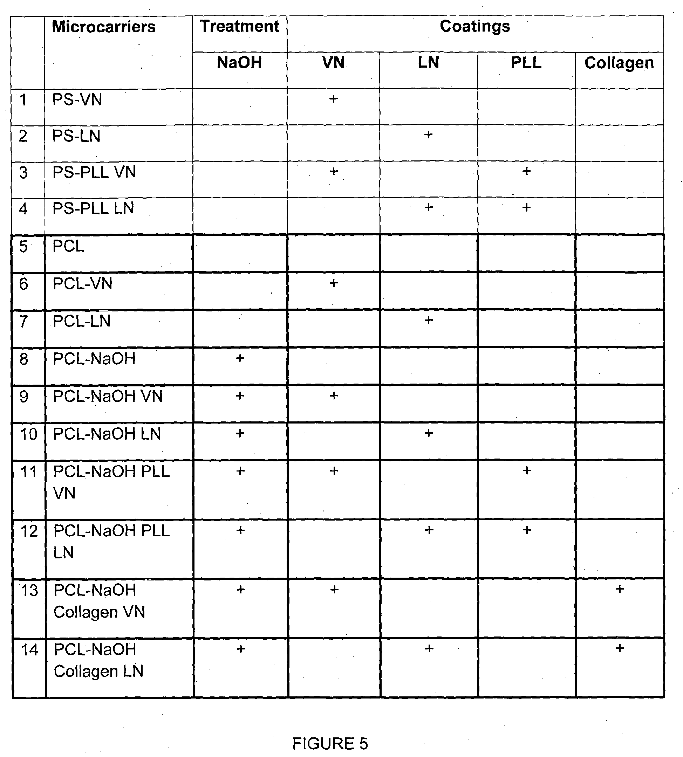

PCL microcarriers were coated with different reagents as shown in Table 1 (Figure 5). Following enzymatic dissociation into single cells with TrypLE™ Express (Invitrogen; catalog number 12604-021), the cells were seeded at 1x10⁶ cells per well (in 5mL) onto 20mg of ECMs-coated PS or onto 30mg of ECMs-coated PCL (with or without NaOH treatment), settled on the base of ultra-low adherent 6-well plate (Corning; catalog number 3471).

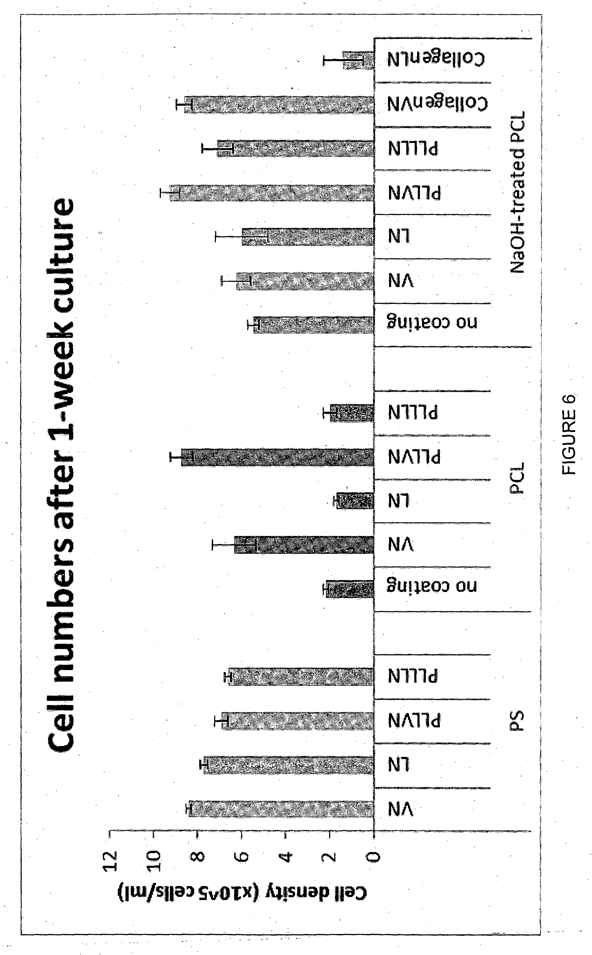

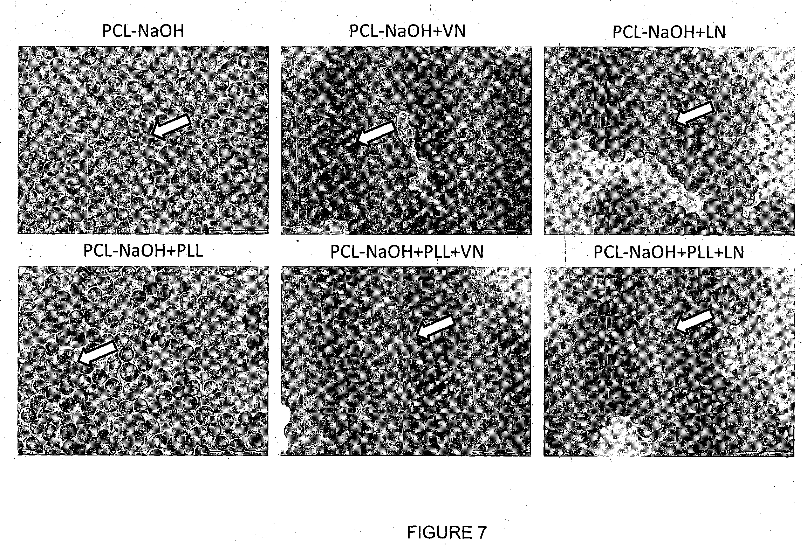

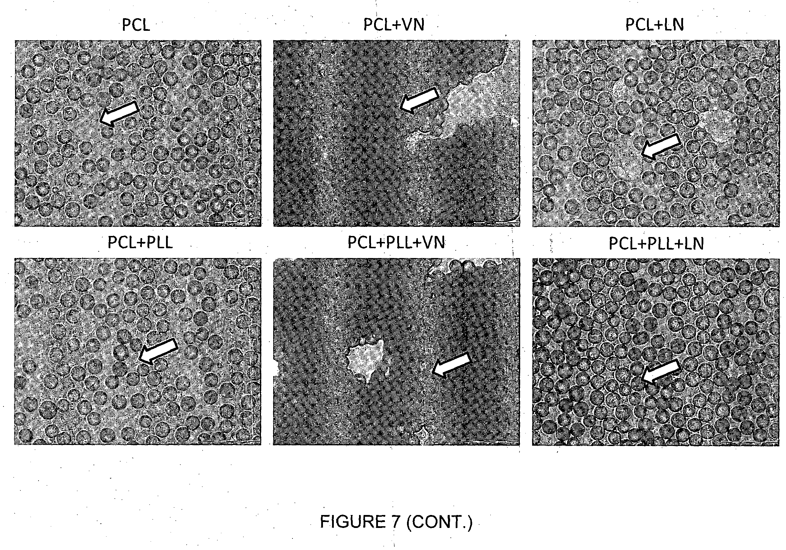

Immediately after seeding, the culture plates were temporarily placed on an orbital shaker at 110rpm in a 37°C/5% CO₂ incubator for 2 hours to promote contact between hESCs and microcarriers. They were then cultivated under static conditions for 7 days and medium was refreshed daily (80%). Cells numbers were counted on day 7 using the NucleoCounter NC-3000 (Chemometec, Inc.). Cultures were passaged after 7 days for 4 weeks. Figure 6 shows cell densities achieved of hESC cultured on different ECM-coated PS or PCL microcarriers after 1 week. Figure 7 shows growth of hESC on PCL microcarriers with different coatings, NaOH, PLL, LN and VN. High densities of cells form large clusters of cell-microcarrier aggregates for several coating conditions.

### Example 6 - Growth of MSCs on PCL microcarriers

### Reagents and Chemicals

High glucose Dulbecco's modified Eagle's medium (DMEM; Cat. No. 11960), fetal bovine serum (FBS; Cat. No. 10270), 0.25% trypsin-EDTA (Cat. No. 25200) and Dulbecco's phosphate buffered saline (PBS; Cat. No. 14190) were purchased from Gibco, USA. Cytodex type 3 microcarriers (Cat. No. C3275) and Polycaprolactone (PCL; Cat. No. 440744) was obtained from Sigma-Aldrich. All consumables other than ultra-low attachment tissue culture plates (Sigma-Aldrich, CLS3516) and T-175 flasks (Nunc) were purchased from Becton-Dickinson (Franklin Lakes, NJ).

### MSC culture

Human fetal mesenchymal stem cells at 13 week gestation were obtained from clinically-indicated termination of pregnancy with written consent from pregnant women. The fetal tissue collection was approved by the Domain Specific Review Board of National University Hospital, Singapore (DSRB-D-06-154) in compliance with international guidelines regarding the use of fetal tissue for research (Polkinghorne, 1992). Single-cell suspensions of fetal bone marrow were prepared by flushing the marrow cells out of humeri and femurs using a 22-gauge needle into DMEM supplemented with 10%v/v FBS, 50 U/ml penicillin, and 50 mg/ml streptomycin (referred herein as D10). Cells were then cultured at an inoculation density of 2900 cells/cm² in T-175 flasks in D10 and sub-cultured every 4 days. When near-confluency was reached (70%), the cells were washed with PBS and enzymatically harvested using 3.0 ml of trypsin-EDTA) for each T-175 flask and inoculated into new T-flasks containing fresh D10. All cultures were maintained at 37°C, 5% CO₂ humidified incubator.

### Preparation of Cytodex 3 microcarriers

4 g of dry Cytodex 3 microcarriers (Sigma, CAS Number: 88895-19-6) microcarriers were hydrated for 3 hours in a Pyrex flask, using 400 ml of Ca2+, Mg2+ free PBS for 3 hours at room temperature. The supernatant was decanted and the microcarriers were washed for a few minutes in fresh Ca2+, Mg2+ free PBS (30-50 ml/g Cytodex). The PBS was discarded and replaced with fresh Ca2+, Mg2+ free PBS (30-50 ml/g Cytodex) and the microcarriers were sterilized by autoclaving (115 °C, 15 min, 15 psi). The hydrated microcarriers were autoclaved and stored at 4°C for later use.

### Preparation of Polycaprolactone (PCL) microcarriers

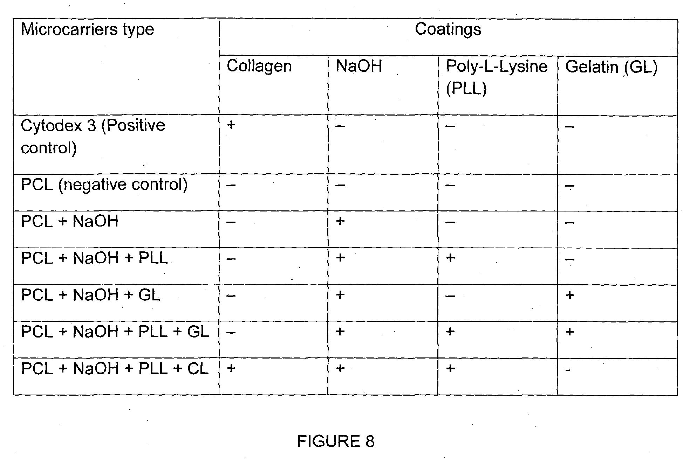

PCL microcarriers were coated with 3 different reagents as shown in Table 2 (Figure 8).

### Microcarrier culture of MSCs

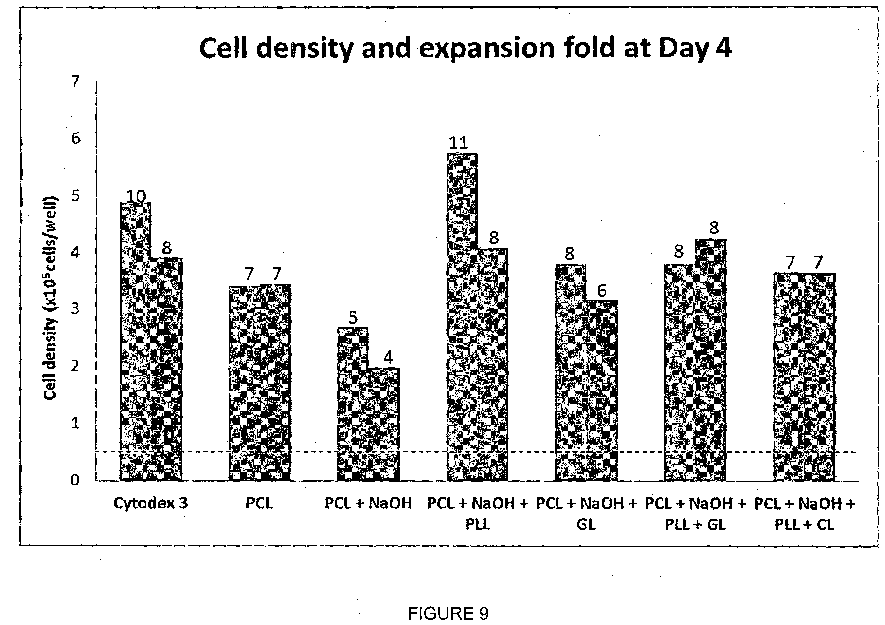

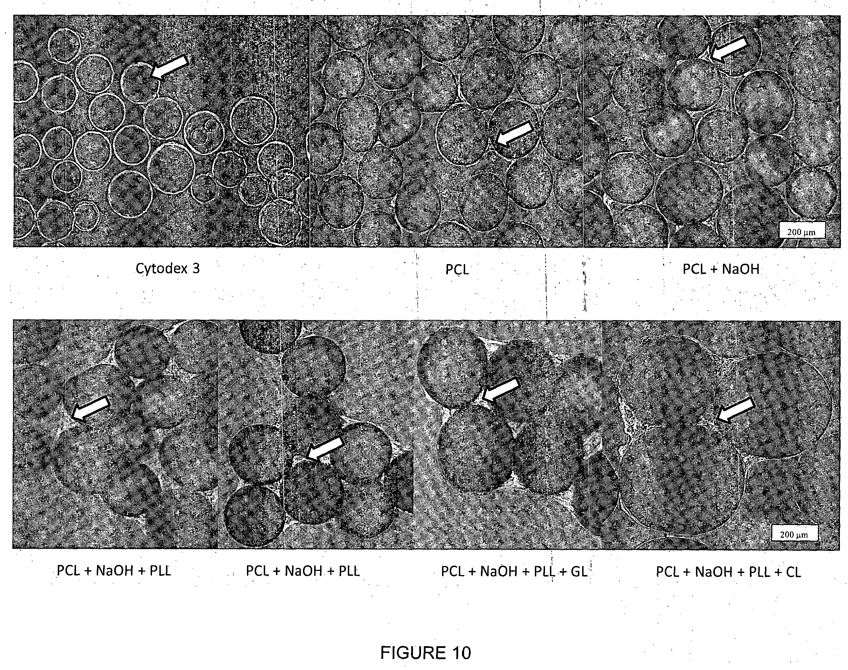

For the Cytodex 3 microcarrier cultures, 4 mg of cytodex 3 microcarriers were added to 5 ml of D10 in a 6 well plate in duplicates. For the PCL microcarrier cultures, 40 mg of microcarriers were used. Passage 5 MSCs were then seeded into the wells at a density of 1 x 10⁴ cells/ml. The cells were expanded in D10 for at least 2 passages before they were seeded. The plates were then gently agitated for 5 minutes and incubated at 37°C with 5% humidified CO₂ for 4 days of growth. On day 4, cells were trypsinized with 2 ml of trypsin-EDTA and counted using a NucleoCounter NC-3000 (Chemometec, Inc.). The results of MSC growth on different PCL microcarriers at Day 4 are show in Figure 9. Figure 10 shows growth of MSCs on PCL microcarriers with different coatings (polylysine, PLL, NaOH, gelatin, GL or collagen CL) after 4 days compared to commercial Cytodex 3.

### Example 7 - Culture and counting of stem cells on PCL microspheres coated with fibronectin/poly-L-lysine/fibronectin

### 6-well ultra-low attachment plates

Following enzymatic dissociation of hfMSC into single cells with Trypsin (Life Technologies), cells were seeded at 5 x 10⁴ cells per well (in 5 ml) onto 40 mg of PCL microcarriers coated sequentially with FN, PLL, and FN (FN-PLL-FN). These were then allowed to settle onto the base of 6-well ultra-low attachment (ULA) plates (Corning). Cells were cultured for 6 days under different rocking regimes, at 25 rpm with a 35 degree angle: (1) static conditions for 6 days (6d static), (2) static for 1 day followed by 5 days with rocking conditions (1d static + 5d rocking), and (3) 2 days under static conditions followed by 4 days under rocking conditions (2d static + 4d rocking), in a 37°C, 5% humidified CO₂ incubator.

### FN-PLL-FN coated PCL microcarriers support stem cell growth under static and rocking conditions in ultra low adhesion 6-well plates

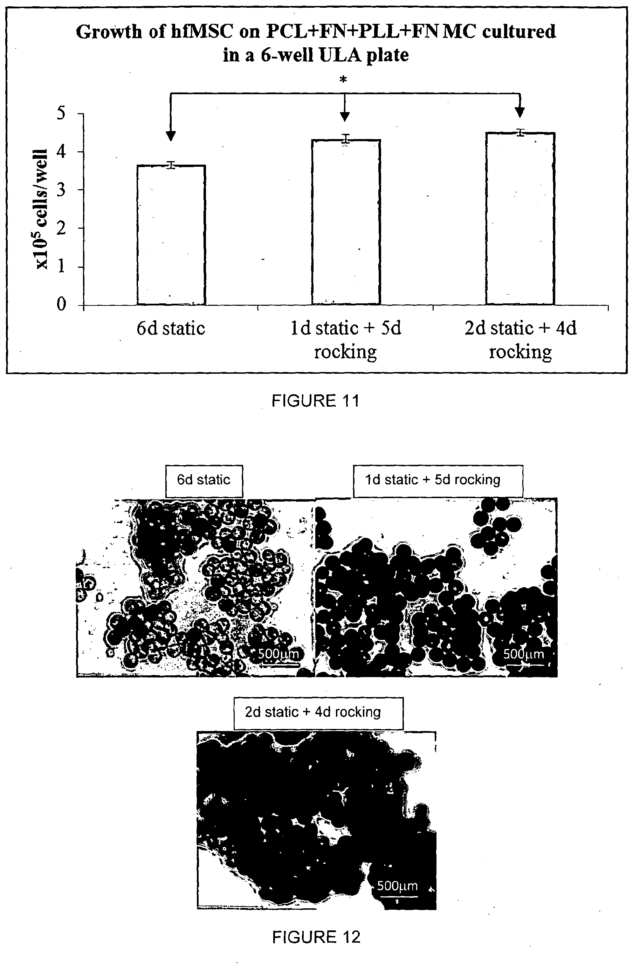

Cells were counted on day 6 using the NucleoCounter NC-3000 (Chemometec, Inc.). Figure 11 shows that PCL microcarriers coated with FN-PLL-FN supported stem cell growth, with highest cell growth observed for 2d static + 4d rocking condition. Counts revealed 3.5 x 10⁵ cells/well for the 6d static condition, representing a 7.4-fold cell expansion. An introduction of continuous rocking after a 1 or 2 day static pause appears to support increased cell growth compared with no rocking at all, achieving 3.9 x 10⁵ (7.8-fold expansion) and 4.5 x 10⁵ cells/well (9-fold expansion) for the 1d static + 5d rocking and 2d static + 4d rocking groups respectively (p≤0.05).

### FN-PLL-FN coated PCL microcarriers seeded with stem cells in ultra low adhesion 6-well plates form small aggregates or large sheet-like aggregates

Microscopic images were captured to determine the shape and organization of cells on microcarriers. Figure 12 shows that PCL microcarriers coated with FN-PLL-FN and seeded with stem cells form small aggregates or large sheet-like aggregates of cells and microcarriers after 6 days in culture (see Figure 12). Under 1d static + 5d rocking conditions, cell-laden microcarriers formed aggregates after 6 days in culture. The 2d static + 4d rocking regime promotes the formation of cells/MC aggregates fused together to form a larger cell-sheets.

### Stem cells grown on FN-PLL-FN coated PCL microcarriers continue to express stem cell markers in ultra low adhesion 6-well plates

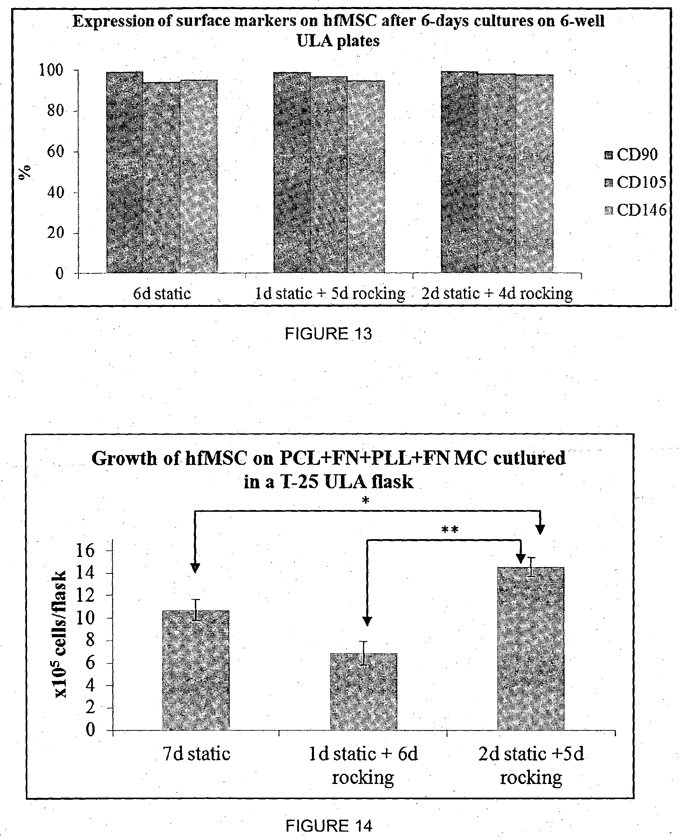

After 6 days in culture under static or mixed static/rocking conditions, cells were harvested from microcarriers, stained with antibodies for MSC stem cell surface markers CD90, CD105 and CD146 and analyzed by flow cytometry. Figure 13 shows that expression remained high (over 90%) for hfMSCs grown in 6-well plates on PCL microcarriers coated with FN-PLL-FN for all the culture conditions tested, indicating that the mesenchymal stem cell phenotype was unchanged.

### Ultra-low attachment T25 flasks

Following enzymatic dissociation of hfMSC into single cells with Trypsin (Life Technologies), cells were seeded at 1.25 x 10⁵ cells per flask onto 100 mg of PCL microcarriers coated with FN-PLL-FN. These were cultured in 10 ml of media total, and allowed to settle onto the base of a T-25 ultra-low attachment plate (Corning). Cells were cultured for 7 days under different rocking regimes, at 25 rpm with a 35 degree angle: (1) statis conditions for 7 days (7d static), (2) static for 1 day followed by 6 days with rocking conditions (1d static + 6d rocking), and (3) 2 days under static conditions followed by 5 days under rocking conditions (2d static + 5d rocking), in a 37°C, 5% humidified CO₂ incubator.

### FN-PLL-FN coated PCL microcarriers support increased stem cell growth under static and rocking conditions in ultra low adhesion T25 flasks

Cells were counted on day 7 using the NucleoCounter NC-3000 to determine stem cell growth. Figure 14 shows that 2d static and 5d rocking cultures yielded higher cell numbers compared to the 7d static cultures (*indicating p ≤0.05) and 1d static and 6d rocking cultures (**indicating p ≤0.01). For the 7d static condition, cell numbers were 10.7 x 10⁵ cells/well (8.6-fold expansion) after 7 days. When rocking regimes were also introduced, cell numbers were lower for the 1d static + 6d rocking compared to 7d static (6.8 x 10⁵ cells/well, 5.4-fold expansion) but higher for the 2d static + 5d rocking condition (14.5 x 10⁵ cells/well, 12-fold expansion, p≤0.05).

### Stem cells grown on FN-PLL-FN Coated PCL microcarriers form monolayers on microcarriers or small microcarrier aggregates under static and rocking conditions in ultra low adhesion T25 flasks

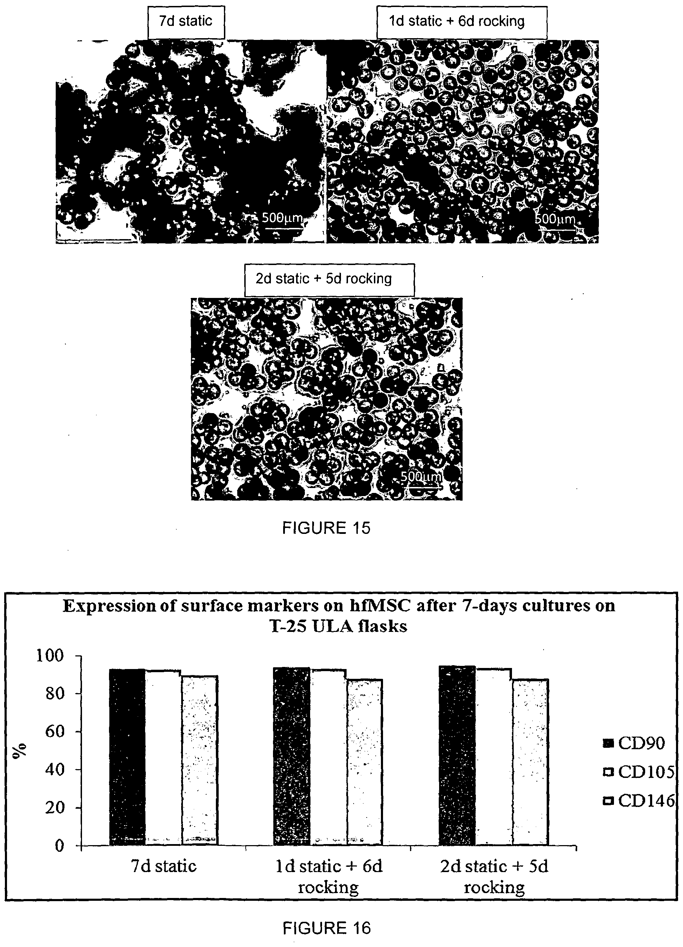

Microscopic images were captured to determine the shape and organization of cells on microcarriers. Figure 15 shows that stem cells grown on FN-PLL-FN coated PCL microcarriers in ultra low adhesion T25 flasks show that cell-laden microcarriers formed large aggregates in the 6d static condition but grew as monolayers under the 1d static + 5d rocking condition and formed small aggregates under the 2d static + 4d rocking conditions.

### Stem cells grown on FN-PLL-FN Coated PCL microcarriers continue to express stem cell markers in ultra low adhesion T25 flasks

After 7 days in culture on plates on PCL microcarriers coated with FN-PLL-FN in ultra low adhesion T25 flasks, cells were harvested from microcarriers, stained with antibodies for MSC stem cell surface markers CD90, CD105 and CD146 and analyzed by flow cytometry. Figure 16 shows that hfMSCs continued to express CD90, CD105 and CD146 in a majority of the cell population (over 90% of cells) for all three culture conditions tested, suggesting the stem cell phenotype was unchanged.

### Example 8 - Culture and osteogenic differentiation of stem cells grown on PCL microspheres with fibronectin/poly-L-lysine/ fibronectin multilayer

2 x 10⁴ hfMSCs were seeded either on tissue culture-treated plastic (TCP) 6-well plates (Nunc) or onto 15 mg of PCL microcarriers in ultra-low adhesion 6-well plates (Corning). After seeding, the cells were either cultured in growth media for 4 days and then cultured in osteogenic induction media for 28 days (expansion/differentiation), or immediately cultured in osteogenic induction media for 28 days (direct differentiation). Both cell seeding and culture were performed under static conditions. The three conditions tested were: (1) TCP expansion/differentiation, (2) PCL microcarriers expansion/differentiation, and (3) PCL microcarriers direct differentiation. Culture medium was replenished every 2-3 days. The growth medium with alpha-MEM supplemented with 10% fetal bovine serum and 1% penicillin/streptomycin. The osteogenic induction medium with DMEM supplemented with 10% fetal bovine serum and 1% penicillin/streptomycin and 10 mM beta glycerophosphate, 10 nM dexamethasone and 0.2 mM ascorbic acid.

### FN-PLL-FN coated PCL microcarriers support hfMSC (stem cell) adhesion and growth under static conditions

Figure 17 shows Micrographs of hfMSCs cultured on PCL microcarriers coated with FN-PLL-FN after 1 Day (Figure 17A) and 7 Days (Figure 17B) of differentiation by culture in osteogenic induction media *in vitro* under static conditions. High levels of initial cells adhesion to the PCL microcarrier surfaces was observed (Figure 17A), and cells proliferated rapidly and aggregated over the next 6 days (Figure 17B). Figure 17C shows that large cell aggregates were formed after 21 days in ultra-low adhesion plates under static conditions. Tissue culture plastic expanded and differentiated (TCP ED) cells were included as a control.

### Example 9 - Measuring calcium deposited by stem cells grown on PCL microspheres with fibronectin/poly-L-lysine/fibronectin multilayer

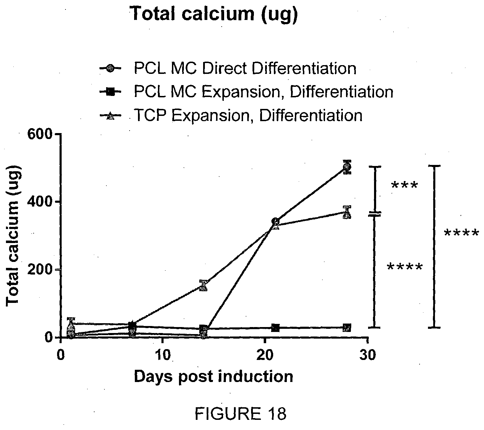

hfMSC mineralization was tested on 3 different conditions: (1) PCL microcarrier Direct Differentiation, (2) PCL microcarrier Expansion/ Differentiation, and (3) TCP Expansion/ Differentiation, with (3) serving a positive control. At 1, 4, 7, 21 and 28 days post-differentiation, the hfMSCs on TCP or on microcarriers were rinsed twice with PBS without calcium and magnesium, transferred into 0.5 M acetic acid and incubated at room temperature for 1 hour. The samples were then vortexed and centrifuged and the acetic acid supernatant was analyzed for calcium content using a Quantichrom Calcium Assay Kit (BioAssay Systems) according to the manufacturer's instructions. Results are shown in Figure 18.

### FN-PLL-FN coated PCL microcarriers support hfMSC (stem cell) osteogenic differentiation and mineralization (conversion to bone phenotype and deposition of calcified matrix)

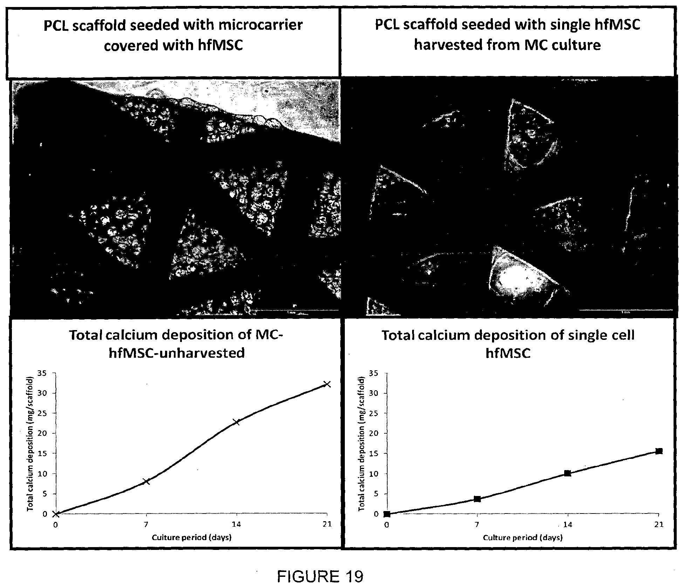

Figure 19 shows that FN-PLL-FN coated PCL microcarriers (MC) support robust *in vitro* osteogenic differentiation of hfMSCs cultured in static conditions. For all groups, calcium deposition levels were at low background levels (less than 50 µgs) at day 1 but increased over 28 days of culture in osteogenic induction medium. By Day 28 post osteogenic induction, calcium levels were higher for hfMSCs cultured on PCL microcarriers under the direct differentiation (DD) when compared to cells cultured on PCL microcarriers or TCP under the expansion/ differentiation (ED) protocol. At Day 28, hfMSCs in the PCL microcarrier DD group contained 503.1 +/- 17.6 µg calcium, indicating a 36% increase in calcium compared to TCP ED (370.3 +/- 14.9 µg calcium), the positive control group, and a 123% increase in calcium compared to the PCL microcarrier ED group (29.7 +/- 0.7 µg calcium).

Taken together, these results indicate that PCL microcarriers support hfMSC adhesion, proliferation and osteogenic differentiation *in vitro.*

### Example 10 - Coating PCL microspheres with poly-L-lysine, polybrene and ECM proteins Fibronectin and Vitronectin

Polybrene (PB) was purchased from Sigma-Aldrich Inc. (Cat # 107689), vitronectin (VN) was purchased from Primogen Biosciences Inc. (Cat #07180), and fibronectin (FN) was obtained from Sigma (Cat #F0895). The microspheres were coated with one layer or several layers of PLL, PB, VN or FN. Each coating layer was applied by incubating 400 mg of PCL microspheres in 1 ml PBS solution of PLL, PB, VN or FN in a 1.5 ml Eppendorf tube for 15 hours at room temperature. The PLL, PB, FN and VN solution concentrations used for deposition were 480, 480, 120 and 120 µg/ml, respectively. After being coated with each layer of PLL, PB, FN or VN, respectively, the coated PCL MC were rinsed twice in PBS. By repeating this process with each coating solution, the following multilayer coatings were deposited on the PCL microcarriers (fabricated as described above):
PCL+PLL,
PCL+FN,
PCL+VN,
PCL+FN+PLL,
PCL+FN+PB,
PCL+PLL+FN,
PCL+PLL+VN,
PCL+PB+FN,
PCL+PLL+FN,
PCL+PLL+FN,
PCL+FN+PB+FN,
PCL+FN+PLL+FN+PLL+FN,
PCL+VN+PLL+FN,
PCL+FN+PLL+VN,
PCL+VN+PLL+VN.

### Example 11 - Culture of mesenchymal stem cells on attachment substrate-coated PCL microcarriers, in constant agitation culture

PCL microcarriers coated with different layers of attachment substrates (Example 9) were tested for their ability to support MSC growth in 10% fetal bovine serum-containing media during agitation culture on a rocking platform. hMSCs were seeded onto PCL microcarriers at 6.5x10⁴ cells per 40mg of PCL microcarriers (or 4 mg of Cytodex 3 microcarriers as a control), and grown in T25 ultra-low adherence flasks either in static culture, or with constant agitation on a rocking platform (28 oscillations/min).

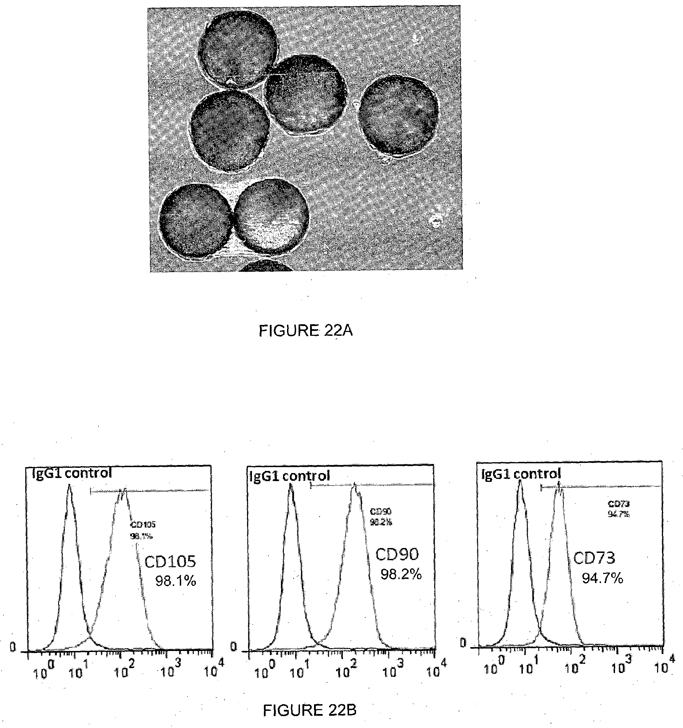

PCL microcarriers coated with a triple layer of fibronectin, poly-L-lysine, and fibronectin (FN-PLL-FN) supported growth of MSCs under such conditions, giving a 14-fold increase in cell numbers over input (Figure 20). These cells expressed cell surface markers characteristic of MSCs (Figure 22B).

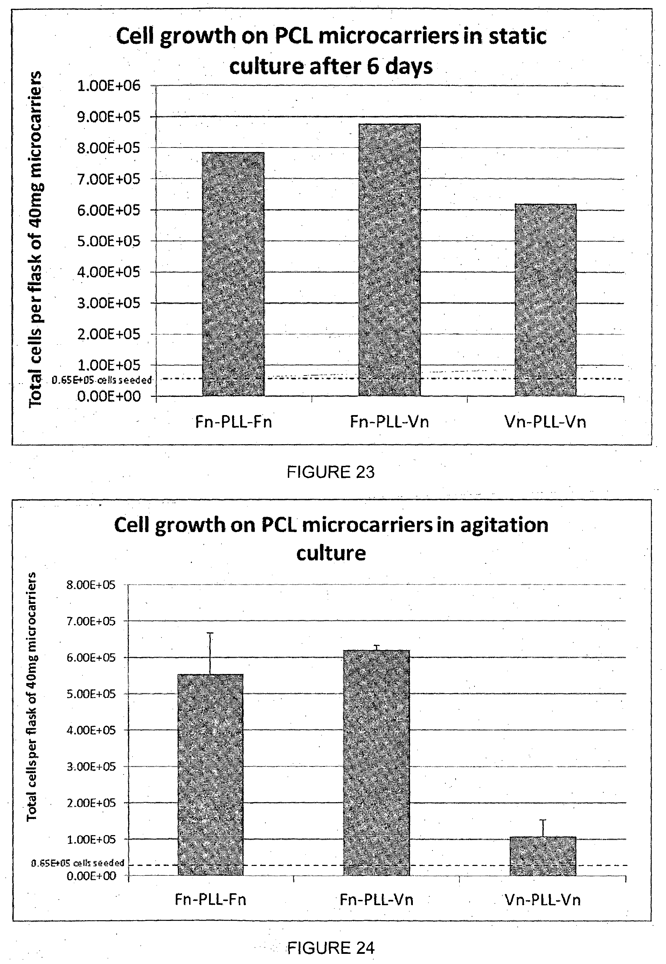

PCL microcarriers with different surface coatings were tested for their ability to support MSC growth in serum-containing media in static culture. Coatings consisting of triple layers of fibronectin and/or vitronectin, with a middle layer of poly-L-lysine all supported MSC growth (Figure 23).

Figure 24 shows MSC growth in serum-containing media with constant agitation on a rocking platform.

### Example 12 - Size distribution parameters

Size distribution parameters (dₓ in µm) for PCL microcarriers, as well as area and number for one gram of dry PCL microcarrier, made with 1%PCL/DCM solution at different flow rates of continuous phase (V_{c}) and dispersed phase (V_{d}) are given in the tables below.

| **V_{c} (µl/ml) as V_{d}= 50 µl/min** | **30** | **40** | **50** | **100** | **150** | **300** |
|---|---|---|---|---|---|---|
| d5 | 168.9 | 146.3 | 102.8 | 95.9 | 89.1 | 80.1 |
| d10 | 169 | 147.2 | 108.3 | 98 | 89.8 | 80.7 |
| d50 | 174.9 | 155.2 | 116.9 | 102 | 93.4 | 83.5 |
| d90 | 182.6 | 160.2 | 120.6 | 108.3 | 97.5 | 86.7 |
| d95 | 185.9 | 162.5 | 122 | 111.1 | 98.2 | 87 |
| Approx area of MC (dry weight) (cm²/g) | 316 | 356 | 475 | 537 | 589 | 662 |
| Number of MC per gram (dry weight) (×10⁶) | 0.33 | 0.475 | 1.123 | 1.627 | 2.144 | 3.053 |

| **V_{c} (µl/ml) as V_{d}= 100 µl/min** | **100** | **200** | **300** | **400** |
|---|---|---|---|---|
| d5 | 140 | 115.6 | 114 | 114.6 |
| d10 | 141.8 | 115.7 | 117 | 117.6 |
| d50 | 145.3 | 119.3 | 122 | 122.4 |
| d90 | 147.8 | 122 | 125 | 125.6 |
| d95 | 150.4 | 122.7 | 128 | 128 |
| Approx area of MC (dry weight) (cm²/g) | 379 | 463 | 451 | 479 |
| Number of MC per gram (dry weight) (×10⁶) | 0.571 | 1.04 | 0.965 | 1.152 |

PCL microcarriers did not swell appreciably in aqueous solution. Scanning electron microscopy of PCL microcarriers confirmed the presence of surface pores contributing to a - reduction in overall density of the microcarriers.

## Claims

1. A PCL microcarrier, or plurality thereof, having a density of 1.03 to 1.09 g/cm³.

2. The PCL microcarrier, or plurality thereof, according to claim 1, having a density of 1.04 to 1.07 g/cm³.

3. The PCL microcarrier, or plurality thereof, according to claim 1, having a density of 1.03 to 1.07 g/cm³.

4. The PCL microcarrier, or plurality thereof, according to any one of claims 1 to 3, having a mean diameter of about 50 to about 400 µm, and a coefficient of variation (CV) of the diameter of less than 10%.

5. The PCL microcarrier, or plurality thereof, according to any one of claims 1 to 3, having a mean diameter of about 150 to 200 µm, and a CV of the diameter of less than 5%.

6. The PCL microcarrier, or plurality thereof, according to any one of claims 1 to 5, wherein the PCL microcarrier is porous.

7. The PCL microcarrier, or plurality thereof, according to any one of claims 1 to 6, wherein the PCL microcarrier is spherical in shape.

8. The PCL microcarrier, or plurality thereof, according to any one of claims 1 to 3, wherein the PCL microcarrier or plurality thereof is provided in dried form.

9. The PCL microcarrier, or plurality thereof, according to any one of claims 1 to 7, further comprising a coating comprising an adhesion promoting polypeptide, glycopolypeptide, cationic polyelectrolyte or polysaccharide.

10. The PCL microcarrier, or plurality thereof, according to any one of claims 1 to 7, further comprising a multilayer coating comprising
(i) a first layer, comprising a matrix component, and
(ii) another layer, comprising a matrix component,
wherein a matrix component is poly-L-lysine, laminin, gelatin, collagen, keratin, fibronectin, vitronectin, hyaluronic acid, elastin, heparan sulphate, dextran, dextran sulphate, chondroitin sulphate or a mixture of laminin, collagen I, heparan sulfate proteoglycans, and entactin 1.

11. The PCL microcarrier, or plurality thereof, according to any one of claims 1 to 10, further comprising a coating of cells.

12. The PCL microcarrier, or plurality thereof, according to claim 11, wherein the cells are stem cells.

13. A method of culturing cells, optionally stem cells, the method comprising:
(i) attaching cells to a PCL microcarrier, or to a plurality of PCL microcarriers, according to any one of claims 1 to 10, to form microcarrier-cell complexes; and
(ii) culturing the microcarrier-cell complexes.

14. The method of claim 13, wherein the number of cells after step (ii) is expanded.

15. The method of claim 13 or claim 14, wherein culturing the microcarrier-cell complexes comprises suspension culture under static conditions and/or suspension culture with agitation, and/or wherein culturing the microcarrier-cell complexes comprises culture in serum free media.

16. A method of differentiating stem cells, the method comprising:
(i) attaching stem cells to a PCL microcarrier, or to a plurality of PCL microcarriers, according to any one of claims 1 to 10, to form microcarrier-stem cell complexes; and
(ii) inducing differentiation of the stem cells.

## Patentansprüche

1. PCL-Mikroträger oder eine Vielzahl davon mit einer Dichte von 1,03 bis 1,09 g/cm³.

2. PCL-Mikroträger oder eine Vielzahl davon nach Anspruch 1 mit einer Dichte von 1,04 bis 1,07 g/cm³.

3. PCL-Mikroträger oder eine Vielzahl davon nach Anspruch 1 mit einer Dichte von 1,03 bis 1,07 g/cm³.

4. PCL-Mikroträger oder eine Vielzahl davon nach einem der Ansprüche 1 bis 3 mit einem mittleren Durchmesser von etwa 50 bis etwa 400 µm und einem Schwankungskoeffizienten (CV) des Durchmessers von weniger als 10 %.

5. PCL-Mikroträger oder eine Vielzahl davon nach einem der Ansprüche 1 bis 3 mit einem mittleren Durchmesser von etwa 150 bis 200 µm und einem CV des Durchmessers von weniger als 5 %.

6. PCL-Mikroträger oder eine Vielzahl davon nach einem der Ansprüche 1 bis 5, wobei der PCL-Mikroträger porös ist.

7. PCL-Mikroträger oder eine Vielzahl davon nach einem der Ansprüche 1 bis 6, wobei der PCL-Mikroträger kugelförmig ist.

8. PCL-Mikroträger oder eine Vielzahl davon nach einem der Ansprüche 1 bis 3, wobei der PCL-Mikroträger oder eine Vielzahl davon in getrockneter Form bereitgestellt ist.

9. PCL-Mikroträger oder eine Vielzahl davon nach einem der Ansprüche 1 bis 7, weiters umfassend eine Beschichtung, die ein haftvermittelndes Polypeptid, Glykopolypeptid, einen kationischen Polyelektrolyt oder ein Polysaccharid umfasst.

10. PCL-Mikroträger oder eine Vielzahl davon nach einem der Ansprüche 1 bis 7, weiters umfassend eine Mehrfachbeschichtung, die
(i) eine erste Schicht, umfassend eine Matrixkomponente, und
(ii) eine weitere Schicht, umfassend eine Matrixkomponente, umfasst,
wobei es sich bei der Matrixkomponente um Poly-L-lysin, Laminin, Gelatine, Collagen, Keratin, Fibronectin, Vitronectin, Hyaluronsäure, Elastin, Heparansulfat, Dextran, Dextransulfat, Chondroitinsulfat oder ein Gemisch aus Laminin, Collagen I, Heparansulfatproteoglycanen und Entacin 1 handelt.

11. PCL-Mikroträger oder eine Vielzahl davon nach einem der Ansprüche 1 bis 10, weiters umfassend eine Beschichtung von Zellen.

12. PCL-Mikroträger oder eine Vielzahl davon nach Anspruch 11, wobei es sich bei den Zellen um Stammzellen handelt.

13. Verfahren zur Kultivierung von Zellen, gegebenenfalls Stammzellen, wobei das Verfahren Folgendes umfasst:
(i) das Binden von Zellen an einen PCL-Mikroträger oder an eine Vielzahl an PCL-Mikroträgern nach einem der Ansprüche 1 bis 10, um Mikroträgerzellenkomplexe zu bilden; und
(ii) das Kultivieren der Mikroträgerzellenkomplexe.

14. Verfahren nach Anspruch 13, wobei die Anzahl der Zellen nach Schritt (ii) erweitert wird.

15. Verfahren nach Anspruch 13 oder Anspruch 14, wobei das Kultivieren der Mikroträgerzellenkomplexe eine Suspensionskultur unter statischen Bedingungen und/oder eine Suspensionskultur mit Bewegung umfasst und/oder wobei das Kultivieren der Mikroträgerzellenkomplexe eine Kultur in einem serumfreien Medium umfasst.

16. Verfahren zur Differenzierung von Stammzellen, wobei das Verfahren Folgendes umfasst:
(i) das Binden von Stammzellen an einen PCL-Mikroträger oder an eine Vielzahl an PCL-Mikroträgern nach einem der Ansprüche 1-10, um Mikroträgerstammzellenkomplexe zu bilden; und
(ii) das Induzieren der Differenzierung von Stammzellen.

## Revendications

1. Microsupport de PCL, ou pluralité de ceux-ci, ayant une masse volumique de 1,03 à 1,09 g/cm³.

2. Microsupport de PCL, ou pluralité de ceux-ci, selon la revendication 1, ayant une masse volumique de 1,04 à 1,07 g/cm³.

3. Microsupport de PCL, ou pluralité de ceux-ci, selon la revendication 1, ayant une masse volumique de 1,03 à 1,07 g/cm³.

4. Microsupport de PCL, ou pluralité de ceux-ci, selon l'une quelconque des revendications 1 à 3, ayant un diamètre moyen d'environ 50 à environ 400 µm, et un coefficient de variation (CV) du diamètre inférieur à 10 %.

5. Microsupport de PCL, ou pluralité de ceux-ci, selon l'une quelconque des revendications 1 à 3, ayant un diamètre moyen d'environ 150 à 200 µm, et un CV du diamètre inférieur à 5 %.

6. Microsupport de PCL, ou pluralité de ceux-ci, selon l'une quelconque des revendications 1 à 5, dans lequel le microsupport de PCL est poreux.

7. Microsupport de PCL, ou pluralité de ceux-ci, selon l'une quelconque des revendications 1 à 6, dans lequel le microsupport de PCL est de forme sphérique.

8. Microsupport de PCL, ou pluralité de ceux-ci, selon l'une quelconque des revendications 1 à 3, dans lequel le microsupport de PCL ou la pluralité de ceux-ci se présente sous forme séchée.

9. Microsupport de PCL, ou pluralité de ceux-ci, selon l'une quelconque des revendications 1 à 7, comprenant en outre un revêtement comprenant un polypeptide favorisant l'adhérence, un glycopolypeptide, un polyélectrolyte cationique ou un polysaccharide.

10. Microsupport de PCL, ou pluralité de ceux-ci, selon l'une quelconque des revendications 1 à 7, comprenant en outre un revêtement multicouche comprenant
(i) une première couche, comprenant un composant de matrice, et
(ii) une autre couche, comprenant un composant de matrice,
dans lequel un composant de matrice est de la poly-L-lysine, de la laminine, de la gélatine, du collagène, de la kératine, de la fibronectine, de la vitronectine, de l'acide hyaluronique, de l'élastine, du sulfate d'héparane, du dextrane, du sulfate de dextrane, du sulfate de chondroïtine ou un mélange de laminine, de collagène I, de sulfate d'héparane-protéoglycanes et d'entactine 1.

11. Microsupport de PCL, ou pluralité de ceux-ci, selon l'une quelconque des revendications 1 à 10, comprenant en outre un revêtement de cellules.

12. Microsupport de PCL, ou pluralité de ceux-ci, selon la revendication 11, dans lequel les cellules sont des cellules souches.

13. Procédé de culture de cellules, facultativement de cellules souches, le procédé comprenant :
(i) la fixation de cellules à un microsupport de PCL, ou à une pluralité de microsupports de PCL, selon l'une quelconque des revendications 1 à 10, pour former des complexes microsupport-cellules ; et
(ii) la culture des complexes microsupport-cellules.

14. Procédé selon la revendication 13, dans lequel le nombre de cellules après l'étape (ii) est augmenté.

15. Procédé selon la revendication 13 ou la revendication 14, dans lequel la culture des complexes microsupport-cellules comprend la culture en suspension dans des conditions statiques et/ou la culture en suspension avec agitation, et/ou dans lequel la culture des complexes microsupport-cellules comprend la culture dans des milieux dépourvus de sérum.

16. Procédé de différenciation de cellules souches, le procédé comprenant :
(i) la fixation de cellules souches à un microsupport de PCL, ou à une pluralité de microsupports de PCL, selon l'une quelconque des revendications 1 à 10, pour former des complexes microsupport-cellules souches ; et
(ii) l'induction de la différenciation des cellules souches.
